# EUROPEAN PATENT APPLICATION

(11) **EP 4 509 145 A1**
(43) Date of publication of application: **19.02.2025**
(21) Application number: 23191752.7
(22) Date of filing: 16.08.2023
(51) Int. Cl.: A61K 51/04, A61P 35/00, A61K 101/02, A61K 103/00

(54) **PSMA THERANOSTIC COMPOUNDS ASSEMBLED WITH TETRAZINE-TRANS-CYCLOOCTENE LIGATION**

(71) Applicant: University of Copenhagen, 1165 Copenhagen K (DK); Rigshospitalet, 2100 Copenhagen Ø (DK); Tetrakit Technologies ApS, 2200 Copenhagen N (DK)
(72) Inventor: HERTH, Matthias Manfred, 21121 Malmö (SE); KJAER, Andreas, 2000 Frederiksberg (DK); BATTISTI, Umberto Maria, 1165 Copenhagen K (DK); SHALGUNOV, Vladimir, 2820 Gentofte (DK); POULIE, Christian Bernard Matthijs, 21837 Bunkeflostrand (SE); MÜLLER, Marius, 1307 Copenhagen K (DK); JENSEN, Andreas, 2830 Virum (DK)
(74) Representative: Budde Schou A/S

(57) **Abstract**

The present disclosure regards PSMA targeting urea-based ligands comprising a PSMA targeting moiety, a linker, and a pyridazine moiety. The PSMA targeting urea-based ligands are suitable for use as radiopharmaceuticals, either as imaging agents, such as diagnostic agents, or for the treatment of prostate cancer or as theranostic agents.

## Description

### Field of the Invention

The present invention relates to urea-based ligands assembled through tetrazine ligation, targeting a prostate-specific membrane antigen and their use in radiotherapy and imaging.

### Background

Prostate cancer (PC) is one of the most commonly diagnosed cancer in men.¹ Further complications arising from PC, such as bone metastases can be developed by a significant number of patients, resulting in a 1-year survival rate of only 40 %. To further complicate this situation, some of these patients are non-responders to conventional hormonal therapy, developing what is known as castration-resistant prostate cancer (CRPC).^{2, 3} Limited therapies are currently available for CRPC patients. Potent and selective radiopharmaceuticals might represent important diagnostic and therapeutic options for this disease. Recently, prostate-specific membrane antigen (PSMA) appeared as a promising target due to its overexpression (8-to-12-fold higher) in PC cells compared to healthy tissue.⁴ Nevertheless, some of the developed and marketed PSMA-targeting radiopharmaceuticals showed a limited response in patients and unpleasant side effects, like renal toxicity and salivary gland build-up.⁴

Current state-of-the-art in PSMA targeted radiotherapy is based on ¹⁷⁷Lu-PSMA-617 (Pluvicto), which has shown good molecular response in clinical evaluations, clearing a noticeable number of metastases and significantly reducing PSA concentrations to normal levels (below 4.0 ng/mL).^{5, 6} Nevertheless, a notable 30% of patients do not respond to β⁻-emitter based therapy such as ¹⁷⁷Lu-PSMA-617, thus alternative strategies are needed.⁶

Alpha emitting radionuclides have recently emerged as a more efficacious alternative to β⁻-emitter due to their ability to deliver higher amount of energy to cancer cells.⁷ This approach is currently being explored for PSMA based therapy. For example, Actinium-225 (²²⁵Ac) has been extensively studied but it is not an ideal radionuclide for therapy as it decays through a chain of four active α-daughter radionuclides, with a total half-life of 10 days.⁸ This means that extensive damage can be caused to healthy tissue thus increasing toxicity effects to the patients and limiting the use of these radiopharmaceuticals.

Astatine-211 (²¹¹At) represent a valid alternative to ²²⁵Ac based on its short half-life of 7.2 hours and its emission that does produce any long-lived alpha-emitting daughters. Both of these properties can significantly reduce cytotoxicity to the patients compared to ²²⁵Ac-based PSMA derivatives.⁹

Radiohalogenated PSMA-targeting pharmaceuticals have been already developed and have shown good, specific tumour uptake, but these compounds were marred by renal and salivary gland build-up, when no blocking agents were administered.¹⁰ Moreover, the synthesis and radiolabeling procedures are based on standard methodologies that are not always easy to implement in radiopharmacies, hospitals and clinics.

Click chemistry has shown great potential in the synthesis of radiopharmaceuticals for imaging and therapy. In particular, the inverse electron demand Diels- Alder cycloaddition reaction (IEDDA) between tetrazines and dienophiles has shown the fastest reaction kinetics in click chemistry, and it has been utilized in radiosynthesis of various labeled radiopharmaceuticals.^{11, 12} The main limitation of this reaction was the formation of multiple isomers after the click. This issue has been recently solved by the synthesis of a new type of trans-cyclooctenes (IsoF-TCOs), that combined with an oxidation step result in the formation of a single isomeric product (PCT/EP2023/055930).

It is shown herein, that by synthesizing a compound comprising a urea-based moiety targeting PSMA, a linker, an IsoF-TCO and a radiolabeled tetrazine, PSMA targeting compounds possessing selected pharmacokinetic properties can be obtained. In addition, the modularity of this approach enables quick and efficient modifications of the radiopharmaceuticals allowing the fast optimization of some properties such as renal excretion and liver accumulation.

As also shown herein, it was surprisingly found that by adding an incremental number of polar amino acids (D-Glutamate and D-Arginine) in the linker between the PSMA binding moiety and the pyridazine moiety the excretion of the PSMA targeting urea-based ligand can be controlled by directing excretion from the liver to the kidneys and bladder. In particular this effect is more pronounced from 2 to 6 amino acids.

Furthermore, the use of the different radiolabeled tetrazines provided herein allows the synthesis of theranostic PSMA compounds starting from the same PSMA targeting urea-based ligand precursor.

Finally, the radiolabeled compounds provided herein showed a surprisingly higher tumor accumulation compared to PSMA-617 retaining the same affinity for the target.

### Summary of the invention:

The present invention provides novel PSMA targeting urea-based ligands having the following general formula (I): 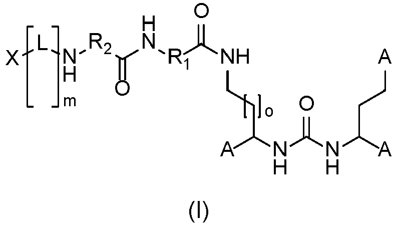 wherein:
A is independently carboxylic acid, sulphonic acid, phosphonic acid, tetrazole or isoxazole;
o is an integer selected from 1-4;
m is an integer selected from 0-10;
R₁ is -CH-CH₂-Z or -CH-CH₂-Y;
wherein Z is selected from the group consisting of: 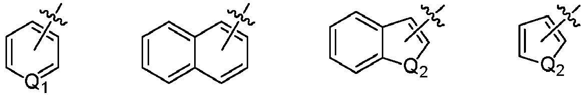
and Y is selected from the group consisting of: 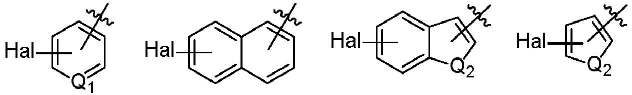 wherein
   Q₁ is -C-R³ or N, wherein R³ is H or C₁-C₅ alkyl;
   Q₂ is O, S or NH;
   Hal (halogen) is a radioisotope selected from the group consisting of radioisotopes of fluorine, radioisotopes of iodine, radioisotopes of bromine and radioisotopes of astatine;
   with the proviso that when R₂ = -CH-CH₂-Y, then R₁ must be = -CH-CH₂-Z
R₂ is -CH-CH₂-Y or -CH₂-T-;
wherein T is an aromatic monocyclic or polycyclic ring system having 6 to 14 carbon atoms, cyclopropyl, cyclobutyl, cyclopentyl, cyclohexyl, cycloheptyl or cyclooctyl; and Y is selected from the group consisting of: 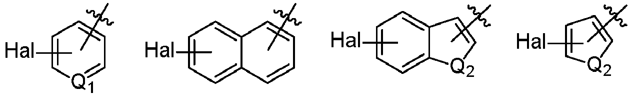 wherein
   Q₁ is -C-R³ or N, wherein R³ is H or C₁-C₅ alkyl;
   Q₂ is O, S or NH;
   Hal (halogen) is a radioisotope selected from the group consisting of radioisotopes of fluorine, radioisotopes of iodine, radioisotopes of bromine and radioisotopes of astatine;
   with the proviso that when R₁ = -CH-CH₂-Y, then R₂ must be = -CH₂-T
L is a moiety comprising groups selected from:
   -CO(CH₂)ₙNH-, -CO(CH₂CH₂O)ₙNH-, -M-, -CO(CH₂)ₙNHMₗ-, -CO(CH₂CH₂O)ₙNHMₗ-, - MnCO(CH₂)ₙNH-, -MnCO(CH₂CH₂O)ₙNH-, -LysG-, -LysJ-, -LysGCO(CH₂CH₂O)ₙNH-, -LysJCO(CH₂)ₙNH-, -LysGCO(CH₂)ₙNHMₗ-,-LysJCO(CH₂)ₙNHMₗ-, -LysGMₙ-, - LysJMₙ-, -LysGCO(CH₂)ₙNHMₗ-, -LysJCO(CH₂)ₙNHMₗ-, -LysGCO(CH₂CH₂O)ₙNHMₗ-, -LysJCO(CH₂CH₂O)ₙNHMₗ-,-LysGMₙCO(CH₂)ₙNH-, -LysJMₙCO(CH₂)ₙNH-, - LysGMₙCO(CH₂CH₂O)ₗNH-, -LysJMₙCO(CH₂CH₂O)ₗNH-, -CO(CH₂CH₂O)ₙNHLysG-, - CO(CH₂)ₙNHLysJ-, -CO(CH₂)ₙNHMₗLysG-, -CO(CH₂)ₙNHMₗLysJ-, -MₙLysG-, - MₙLysJ-, -CO(CH₂)ₙNHMₗLysG-, -CO(CH₂)ₙNHMₗLysJ-, -CO(CH₂CH₂O)ₙNHMₗLysG-, -CO(CH₂CH₂O)ₙNHMₗLysJ-,-MₙCO(CH₂)ₗNHLysG-, -MₙCO(CH₂)ₗNHLysJ-, - MₙCO(CH₂CH₂O)ₗNHLysG-, -MₙCO(CH₂CH₂O)ₗNHLysJ-,
wherein n and I are integers independently selected from 0-10;
where M is selected from one or more natural amino acids, one or more sugars, and a combination of one or more natural amino acid and one or more sugars;
Lys is a D- or L- Lysine amino acid residue modified on the side chain with G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄ wherein p is an integer selected from 0 - 5 and
R₄ is a pyridazine selected from the group consisting of: 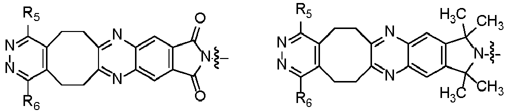 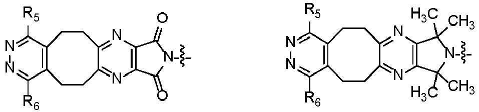 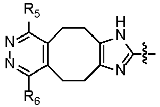
wherein R₅ and R₆ are identical or differs only in the isotope number of the labelling agent and are independently selected from 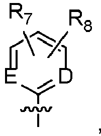 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, - WCONH(CH₂)_{b}, -WNHCO(CH₂)_{b}, where W is -(CH₂)ₕ or -(CH₂CH₂O)ₕ, where b and h are independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, - WNH(CH₂)ᵣ, -WCONH(CH₂)ᵣ, -WNHCO(CH₂)ᵣ, where W is -(CH₂)_{q} or -(CH₂CH₂O)_{q}, and r and q are independently selected from 1-25,
wherein, when R₇ is either (i) or (ii) the isotope labeling agent is selected from the group consisting of:
   ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O,¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
   and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ₋-OCH₂-COOH, wherein u is an integer selected from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, wherein u is an integer selected from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein
X is G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄ wherein p is an integer selected from 0 - 5, and R₄ is a pyridazine selected from the group consisting of: 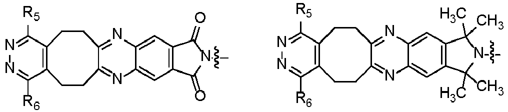 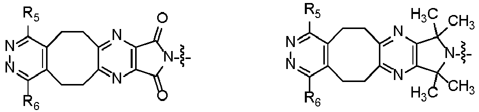 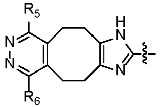 wherein R₅ and R₆ is independently selected from 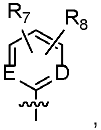 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or - (CH₂CH₂O)ₕ, where b and h are integers independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, -WCONH(CH₂)ᵣ, -WNHCO(CH₂)ᵣ, where W is -(CH₂)_{q} or -(CH₂CH₂O)_{q}, wherein and r and q are integers independently selected from 1-25,
wherein, when R₇ is either (i) or (ii) the isotope labeling agent is selected from the group consisting of:
   ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O,¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷ Pb,²⁰⁸Pb,²¹²Pb, ²⁰⁹Bi, ²¹²Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
   and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ₋-OCH₂-COOH, wherein u is an integer selected from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, and u is an integer selected from 1-5; or H, Methyl, Ethyl, Propyl, optionally substituted heteroaryl, and optionally substituted arylalkyl;
wherein optionally substituted means one or more substituents selected from a halogen, a hydroxy group, a sulfonamide, a carboxyl group, a sulfonyl group, and amine,
and wherein R₅ and R₆ are identical or differs only in the isotope number of the labelling agent;
and wherein formula (I) comprises at least one radioisotope selected from the group consisting of radioisotopes of fluorine, radioisotopes of iodine, radioisotopes of bromine and radioisotopes of astatine; and wherein at least one of L and X comprises J,
and pharmaceutically acceptable salts thereof.

The present disclosure also provides (Me)₃Sn, (Me)₃Si, B(OH)₂ and B(OR₉)₂ precursors that can be used to provide the PSMA targeting ligand according to Formula (I).

Compounds of formula (I) can be used in therapy and for imaging depending on the radionuclides comprised by the specific compound. Accordingly, the present disclosure relates to pharmaceutic formulation comprising a compound of formula (I), a compound of formula (I) for use as a medicament, a compound of formula (I) for use in the treatment of prostate cancer, a compound of formula (I) for use as a radiopharmaceutical, a compound of formula (I) for use as an imaging agent and compound of formula (I) for use as a theranostic agent.

### Brief description of the drawings

Figure 1: Scheme showing the synthesis of compound **15** and **16**.
Figure 2: Scheme showing the synthesis of compound **20** and **21**.
Figure 3: Scheme showing the synthesis of compound **24**, **25** and **26**.
Figure 4: Scheme showing the synthesis of compound **29**, **30** and **31**.
Figure 5: Scheme showing the synthesis of compound **34**, **35** and **36**.
Figure 6: Scheme showing the synthesis of compound **39**, **40** and **41**.
Figure 7: Scheme showing the synthesis of compound **44** and **45.**
Figure 8: Scheme showing the synthesis of compound **48** and **49**.
Figure 9: Scheme showing the synthesis of compound **52** and **53**.
Figure 10: Total synthesis of [¹⁸F]-PSMA derivative [¹⁸F]**25** assembled with tetrazine-trans-cyclooctene ligation.
Figure 11: Semi-preparative HPLC traces (UV = top, Radio = lower) for [¹⁸F]**25**.
Figure 12: Total synthesis of [²¹¹At]-PSMA derivative [²¹¹At]**31** assembled with tetrazine-trans-cyclooctene ligation.
Figure 13: Semi-preparative HPLC traces (UV = right, Radio = left) for [²¹¹At]**31**.
Figure 14: Stability (hours) of [²¹¹At]**31** in EtOH + 5% ascorbic acid.
Figure 15: Activity/time curves obtained in healthy rats for selected organs for [¹⁸F]-PSMA derivatives.
Figure 16: Activity/time curves of [¹⁸F]**25** obtained in tumour bearing (PC3-PIP) mice for selected organs.
Figure 17: Accumulation of [¹⁸F]**25** and [¹⁸F]**30** obtained in tumour bearing (LnCAP) mice for selected organs and tumor.
Figure 18: Ex-vivo biodistribution of [²¹¹At]**31** in tumor bearing mice.

### Detailed description of the invention

The PSMA targeting urea-based ligands of the present invention are suitable for use as radiopharmaceuticals, either as imaging agents, such as diagnostic agents, or for the treatment of prostate cancer or as theranostic agents. The PSMA targeting urea-based ligands of the present invention takes advantage of a urea-based binding motif (((S)-5-amino-1-carboxypentyl)carbamoyl)-L-glutamic acid). This motif specifically interacts with the PSMA antigen binding pocket.

The internalization of the presently disclosed compounds outperform the already existing molecules such as PSMA-617 retaining the same affinity for the target.

The PSMA targeting urea-based ligands of the present invention have the following general formula (I): 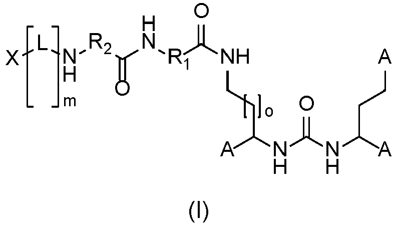 wherein:
A is independently carboxylic acid, sulphonic acid, phosphonic acid, tetrazole or isoxazole;
o is an integer selected from 1-4;
m is an integer selected from 0-10;
R₁ is -CH-CH₂-Z or -CH-CH₂-Y;
wherein Z is selected from the group consisting of: 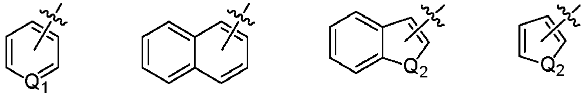
and Y is selected from the group consisting of: 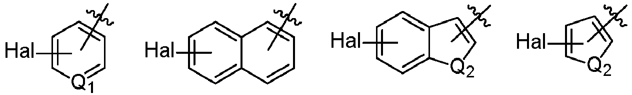 wherein
   Q₁ is -C-R³ or N, wherein R³ is H or C₁-C₅ alkyl;
   Q₂ is O, S or NH;
   Hal (halogen) is a radioisotope selected from the group consisting of radioisotopes of fluorine, radioisotopes of iodine, radioisotopes of bromine and radioisotopes of astatine;
   with the proviso that when R₂ = -CH-CH₂-Y, then R₁ must be = -CH-CH₂-Z
R₂ is -CH-CH₂-Y or -CH₂-T-;
wherein T is an aromatic monocyclic or polycyclic ring system having 6 to 14 carbon atoms, cyclopropyl, cyclobutyl, cyclopentyl, cyclohexyl, cycloheptyl or cyclooctyl;
and Y is selected from the group consisting of: 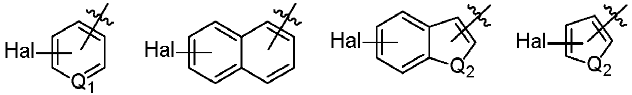 wherein
   Q₁ is -C-R³ or N, wherein R³ is H or C₁-C₅ alkyl;
   Q₂ is O, S or NH;
   Hal (halogen) is a radioisotope selected from the group consisting of radioisotopes of fluorine, radioisotopes of iodine, radioisotopes of bromine and radioisotopes of astatine;
   with the proviso that when R₁ = -CH-CH₂-Y, then R₂ must be = -CH₂-T
L is a moiety comprising groups selected from:
   -CO(CH₂)ₙNH-, -CO(CH₂CH₂O)ₙNH-, -M-, -CO(CH₂)ₙNHMₗ-, -CO(CH₂CH₂O)ₙNHMₗ-, - MnCO(CH₂)ₙNH-, -MnCO(CH₂CH₂O)ₙNH-, -LysG-, -LysJ-, -LysGCO(CH₂CH₂O)ₙNH-, -LysJCO(CH₂)ₙNH-, -LysGCO(CH₂)ₙNHMₗ-,-LysJCO(CH₂)ₙNHMₗ-, -LysGMₙ-, - LysJMₙ-, -LysGCO(CH₂)ₙNHMₗ-, -LysJCO(CH₂)ₙNHMₗ-, -LysGCO(CH₂CH₂O)ₙNHMₗ-, -LysJCO(CH₂CH₂O)ₙNHMₗ-,-LysGMₙCO(CH₂)ₙNH-, -LysJMₙCO(CH₂)ₙNH-, - LysGMₙCO(CH₂CH₂O)ₗNH-, -LysJMₙCO(CH₂CH₂O)ₗNH-, -CO(CH₂CH₂O)ₙNHLysG-, - CO(CH₂)ₙNHLysJ-, -CO(CH₂)ₙNHMₗLysG-, -CO(CH₂)ₙNHMₗLysJ-, -MₙLysG-, - MₙLysJ-, -CO(CH₂)ₙNHMₗLysG-, -CO(CH₂)ₙNHMₗLysJ-, -CO(CH₂CH₂O)ₙNHMₗLysG-, -CO(CH₂CH₂O)ₙNHMₗLysJ-,-MₙCO(CH₂)ₗNHLysG-, -MₙCO(CH₂)ₗNHLysJ-, - MₙCO(CH₂CH₂O)ₗNHLysG-, -MₙCO(CH₂CH₂O)ₗNHLysJ-,
wherein n and I are integers independently selected from 0-10;
where M is selected from one or more natural amino acids, one or more sugars, and a combination of one or more natural amino acids and one or more sugars;
Lys is a D- or L- Lysine amino acid residue modified on the side chain with G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄ wherein p is an integer selected from 0 - 5 and
R₄ is a pyridazine selected from the group consisting of: 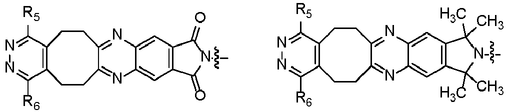 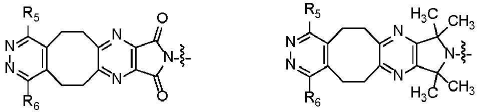 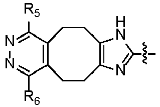
wherein R₅ and R₆ are identical or differs only in the isotope number of the labelling agent and are independently selected from 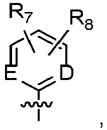 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, - WCONH(CH₂)_{b}, -WNHCO(CH₂)_{b}, where W is -(CH₂)ₕ or -(CH₂CH₂O)ₕ, where b and h are independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, - WNH(CH₂)ᵣ, -WCONH(CH₂)ᵣ, -WNHCO(CH₂)ᵣ, where W is -(CH₂)_{q} or -(CH₂CH₂O)_{q}, and r and q are independently selected from 1-25,
wherein, when R₇ is either (i) or (ii) the isotope labeling agent is selected from the group consisting of:
   ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ₋-OCH₂-COOH, wherein u is an integer selected from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, wherein u is an integer selected from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein
X is G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄ wherein p is an integer selected from 0 - 5, and R₄ is a pyridazine selected from the group consisting of: 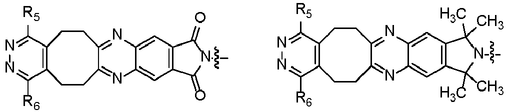 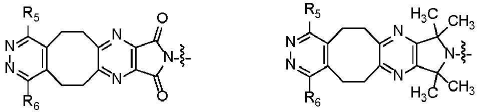 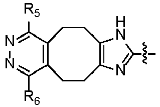 wherein R₅ and R₆ is independently selected from 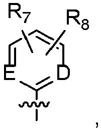 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or - (CH₂CH₂O)ₕ, where b and h are integers independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, -WCONH(CH₂)ᵣ, -WNHCO(CH₂)ᵣ, where W is -(CH₂)_{q} or -(CH₂CH₂O)_{q}, wherein and r and q are integers independently selected from 1-25,
wherein, when R₇ is either (i) or (ii) the isotope labeling agent is selected from the group consisting of:
   ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ₋-OCH₂-COOH, wherein u is an integer selected from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, and u is an integer selected from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein R₅ and R₆ are identical or differs only in the isotope number of the labelling agent;
and wherein formula (I) comprises at least one radioisotope selected from the group consisting of radioisotopes of fluorine, radioisotopes of iodine, radioisotopes of bromine and radioisotopes of astatine; and wherein at least one of L and X comprises J,
and pharmaceutically acceptable salts thereof.

In a preferred embodiment, the PSMA targeting urea-based ligands of the present invention have the following general formula (I): 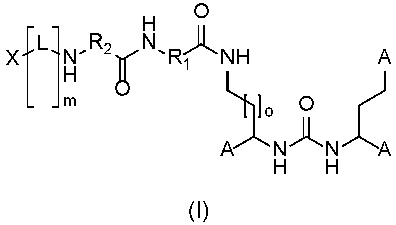 wherein:
A is independently carboxylic acid, sulphonic acid, phosphonic acid, tetrazole or isoxazole;
o is an integer selected from 1-4;
m is an integer selected from 0-10;
R₁ is -CH-CH₂-Z or -CH-CH₂-Y;
wherein Z is selected from the group consisting of: 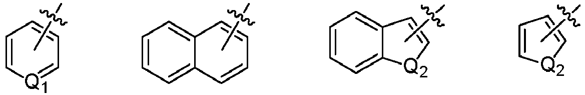
and Y is selected from the group consisting of: 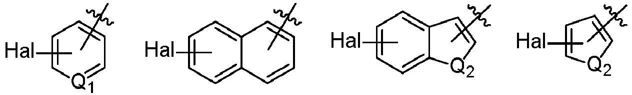 wherein
   Q₁ is -C-R³ or N, wherein R³ is H or C₁-C₅ alkyl;
   Q₂ is O, S or NH;
   Hal (halogen) is a radioisotope selected from the group consisting of radioisotopes of fluorine, radioisotopes of iodine, radioisotopes of bromine and radioisotopes of astatine;
   with the proviso that when R₂ = -CH-CH₂-Y, then R₁ must be = -CH-CH₂-Z
R₂ is -CH-CH₂-Y or -CH₂-T-;
wherein T is an aromatic monocyclic or polycyclic ring system having 6 to 14 carbon atoms, cyclopropyl, cyclobutyl, cyclopentyl, cyclohexyl, cycloheptyl or cyclooctyl; and Y is selected from the group consisting of: 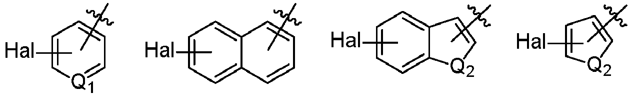 wherein
   Q₁ is -C-R³ or N, wherein R³ is H or C₁-C₅ alkyl;
   Q₂ is O, S or NH;
   Hal (halogen) is a radioisotope selected from the group consisting of radioisotopes of fluorine, radioisotopes of iodine, radioisotopes of bromine and radioisotopes of astatine;
   with the proviso that when R₁ = -CH-CH₂-Y, then R₂ must be = -CH₂-T
L is a moiety comprising groups selected from:
   -CO(CH₂)ₙNH-, -CO(CH₂CH₂O)ₙNH-, -M-, -CO(CH₂)ₙNHMₗ-, -CO(CH₂CH₂O)ₙNHMₗ-, - MnCO(CH₂)ₙNH-, -MnCO(CH₂CH₂O)ₙNH-, -LysG-, -LysJ-, -LysGCO(CH₂CH₂O)ₙNH-, -LysJCO(CH₂)ₙNH-, -LysGCO(CH₂)ₙNHMₗ-,-LysJCO(CH₂)ₙNHMₗ-, -LysGMₙ-, - LysJMₙ-, -LysGCO(CH₂)ₙNHMₗ-, -LysJCO(CH₂)ₙNHMₗ-, -LysGCO(CH₂CH₂O)ₙNHMₗ-, -LysJCO(CH₂CH₂O)ₙNHMₗ-,-LysGMₙCO(CH₂)ₙNH-, -LysJMₙCO(CH₂)ₙNH-, - LysGMₙCO(CH₂CH₂O)ₗNH-, -LysJMₙCO(CH₂CH₂O)ₗNH-, -CO(CH₂CH₂O)ₙNHLysG-, - CO(CH₂)ₙNHLysJ-, -CO(CH₂)ₙNHMₗLysG-, -CO(CH₂)ₙNHMₗLysJ-, -MₙLysG-, - MₙLysJ-, -CO(CH₂)ₙNHMₗLysG-, -CO(CH₂)ₙNHMₗLysJ-, -CO(CH₂CH₂O)ₙNHMₗLysG-, -CO(CH₂CH₂O)ₙNHMₗLysJ-,-MₙCO(CH₂)ₗNHLysG-, -MₙCO(CH₂)ₗNHLysJ-, - MₙCO(CH₂CH₂O)ₗNHLysG-, -MₙCO(CH₂CH₂O)ₗNHLysJ-,
wherein n and I are integers independently selected from 0-10;
where M is a natural amino acid;
Lys is a D- or L- Lysine amino acid residue modified on the side chain with G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄ wherein p is an integer selected from 0 - 5 and
R₄ is a pyridazine selected from the group consisting of: 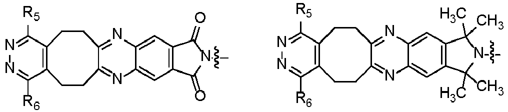 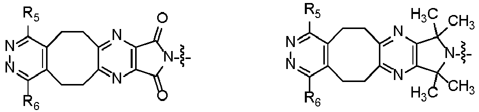 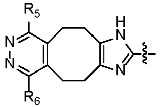
wherein R₅ and R₆ are identical or differs only in the isotope number of the labelling agent and are independently selected from 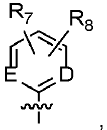 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, - WCONH(CH₂)_{b}, -WNHCO(CH₂)_{b}, where W is -(CH₂)ₕ or -(CH₂CH₂O)ₕ, where b and h are independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, - WNH(CH₂)ᵣ, -WCONH(CH₂)ᵣ, -WNHCO(CH₂)ᵣ, where W is -(CH₂)_{q} or -(CH₂CH₂O)_{q}, and r and q are independently selected from 1-25,
wherein, when R₇ is either (i) or (ii) the isotope labeling agent is selected from the group consisting of:
   ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O,¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ₋-OCH₂-COOH, wherein u is an integer selected from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, wherein u is an integer selected from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein
X is G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄ wherein p is an integer selected from 0 - 5, and R₄ is a pyridazine selected from the group consisting of: 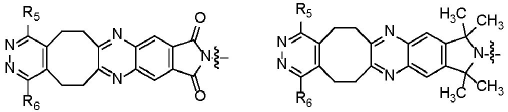 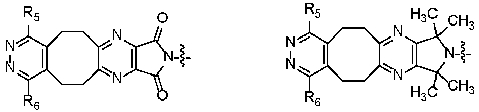 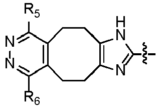 wherein R₅ and R₆ is independently selected from 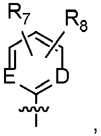 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or - (CH₂CH₂O)ₕ, where b and h are integers independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, -WCONH(CH₂)ᵣ, -WNHCO(CH₂)ᵣ, where W is -(CH₂)_{q} or -(CH₂CH₂O)_{q}, wherein and r and q are integers independently selected from 1-25,
wherein, when R₇ is either (i) or (ii) the isotope labeling agent is selected from the group consisting of:
   ¹H, ²H, ³H, ¹¹C, ¹²C, ¹³C, ¹⁴C, ¹³N, ¹⁴N, ¹⁵N, ¹⁸F, ¹⁹F ¹²³I, ¹²⁴I,¹²⁵I, ¹²⁷I, ¹³¹I, ²¹¹At, ¹⁵Q, ¹⁶Q, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mln, ¹¹⁵mln, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
   and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ--OCH₂-COOH, wherein u is an integer selected from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, and u is an integer selected from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein R₅ and R₆ are identical or differs only in the isotope number of the labelling agent;
and wherein formula (I) comprises at least one radioisotope selected from the group consisting of radioisotopes of fluorine, radioisotopes of iodine, radioisotopes of bromine and radioisotopes of astatine; and wherein at least one of L and X comprises J,
and pharmaceutically acceptable salts thereof.

Accordingly, compounds of Formula (I) always comprise at least one pyridazine. The pyridazine is part of either the L moiety or the X moiety or both the L moiety and the X moiety comprises a pyridazine. The pyridazine is denoted R4 in Formula (I) and is comprised by the J, wherein J is -CO(CH₂)ₚR₄ or-CO(CH₂)ₚC(CH₃)₂R₄ and p is an integer selected from 0 - 5. The pyridazine R₄ may be linked to a chelator or may not be linked to a chelator.

As stated above, Formula (I) moreover comprises at least one radioisotope selected from the group consisting of radioisotopes of fluorine, radioisotopes of iodine, radioisotopes of bromine and radioisotopes of astatine. These radioisotopes can be positioned on the pyridazine R4 when the pyridazine is comprised by either J (i) or J (ii), i.e. when the pyridazine is not linked to a chelator. These radioisotopes can also be positioned either on R₁ or on R₂ but not on both of R₁ and R₂.

The radioisotopes of fluorine, radioisotopes of iodine, radioisotopes of bromine and radioisotopes of astatine comprises: ¹⁸F, ¹⁹F, ¹²³I, ¹²⁴I,¹²⁵I, ¹²⁷I, ¹³¹I, ⁷⁶Br, ⁷⁷Br, ⁷⁹Br, ^{80m}Br, ⁸¹Br, and ²¹¹At.

In a preferred embodiment, the radionuclide is selected from the group consisting of ¹⁸F, ¹²⁵I, ¹²³I, ¹³¹I, ¹²⁴I, ²¹¹At, ⁷⁷Br and ^{80m}Br.

In a preferred embodiment, at least one radionuclide comprised by Formula (I) is positioned on R₇.

In a preferred embodiment, the at least one radionuclide comprised by Formula (I) is ¹⁸F and is positioned on R₇.

In one embodiment, Formula (I) comprises one radioisotope selected from the group consisting of radioisotopes of fluorine, radioisotopes of iodine, radioisotopes of bromine and radioisotopes of astatine positioned on R₁.

In another embodiment, Formula (I) comprises one radioisotope selected from the group consisting of radioisotopes of fluorine, radioisotopes of iodine, radioisotopes of bromine and radioisotopes of astatine positioned on R₂.

In another embodiment Formula (I) comprises one radioisotope selected from the group consisting of radioisotopes of fluorine, radioisotopes of iodine, radioisotopes of bromine and radioisotopes of astatine positioned on R₄, wherein R₄ is comprised by moiety L.

In another embodiment Formula (I) comprises one radioisotope selected from the group consisting of radioisotopes of fluorine, radioisotopes of iodine, radioisotopes of bromine and radioisotopes of astatine positioned on R₄, wherein R₄ is comprised by moiety X.

In one embodiment, Formula (I) comprises two radioisotopes selected from the group consisting of radioisotopes of fluorine, radioisotopes of iodine, radioisotopes of bromine and radioisotopes of astatine positioned on R₁ and R₄, wherein R₄ is comprised by moiety L.

In one embodiment, Formula (I) comprises two radioisotopes selected from the group consisting of radioisotopes of fluorine, radioisotopes of iodine, radioisotopes of bromine and radioisotopes of astatine positioned on R₁ and R₄, wherein R₄ is comprised by moiety X.

In one embodiment, Formula (I) comprises two radioisotopes selected from the group consisting of radioisotopes of fluorine, radioisotopes of iodine, radioisotopes of bromine and radioisotopes of astatine positioned on R₂ and R₄, wherein R₄ is comprised by moiety L.

In one embodiment, Formula (I) comprises two radioisotopes selected from the group consisting of radioisotopes of fluorine, radioisotopes of iodine, radioisotopes of bromine and radioisotopes of astatine positioned on R₂ and R₄, wherein R₄ is comprised by moiety X.

In a preferred embodiment Formula (I) comprises one radioisotope selected from the group consisting of radioisotopes of fluorine, radioisotopes of iodine, radioisotopes of bromine and radioisotopes of astatine positioned on R₄, wherein R₄ is comprised by moiety X or by moiety L.

In another preferred embodiment, Formula (I) comprises two radioisotopes selected from the group consisting of radioisotopes of fluorine, radioisotopes of iodine, radioisotopes of bromine and radioisotopes of astatine positioned on R₄, wherein R₄ is comprised by both moiety L and by moiety X.

In a preferred embodiment, the moiety L in formula (I) is selected from the group consisting of:
-CO(CH₂)ₙNH-, -CO(CH₂CH₂O)ₙNH-, -M-, -CO(CH₂)ₙNHMₗ-, -CO(CH₂CH₂O)ₙNHMₗ-, - MnCO(CH₂)ₙNH-, -MnCO(CH₂CH₂O)ₙNH-, -LysG-, -LysJ-, -LysGCO(CH₂CH₂O)ₙNH-, -LysJCO(CH₂)ₙNH-, -LysGCO(CH₂)ₙNHMₗ-,-LysJCO(CH₂)ₙNHMₗ-, -LysGMₙ-, - LysJMₙ-, -LysGCO(CH₂)ₙNHMₗ-, -LysJCO(CH₂)ₙNHMₗ-, -LysGCO(CH₂CH₂O)ₙNHMₗ-, -LysJCO(CH₂CH₂O)ₙNHMₗ-,-LysGMₙCO(CH₂)ₙNH-, -LysJMₙCO(CH₂)ₙNH-, - LysGMₙCO(CH₂CH₂O)ₗNH-, -LysJMₙCO(CH₂CH₂O)ₗNH-, -CO(CH₂CH₂O)ₙNHLysG-, - CO(CH₂)ₙNHLysJ-, -CO(CH₂)ₙNHMₗLysG-, -CO(CH₂)ₙNHMₗLysJ-, -MₙLysG-, - MₙLysJ-, -CO(CH₂)ₙNHMₗLysG-, -CO(CH₂)ₙNHMₗLysJ-, -CO(CH₂CH₂O)ₙNHMₗLysG-, -CO(CH₂CH₂O)ₙNHMₗLysJ-,-MₙCO(CH₂)ₗNHLysG-, -MₙCO(CH₂)ₗNHLysJ-, - MₙCO(CH₂CH₂O)ₗNHLysG-, -MₙCO(CH₂CH₂O)ₗNHLysJ-,
wherein n and I are integers independently selected from 0-10;
where M is selected from one or more natural amino acids, one or more sugars, and a combination of one or more natural amino acids and one or more sugars;
Lys is a D- or L- Lysine amino acid residue modified on the side chain with G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄, wherein p is an integer from 0 - 5 and
R₄ is a pyridazine selected from the group consisting of: 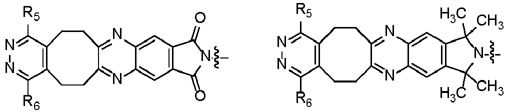 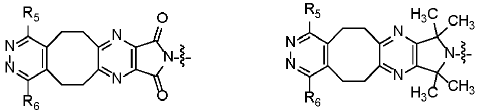 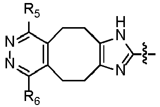
wherein R₅ and R₆ is independently selected from 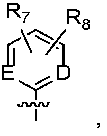 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or - (CH₂CH₂O)ₕ, where b and h are independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, -WCONH(CH₂)ᵣ, -WNHCO(CH₂)ᵣ, where W is -(CH₂)_{q} or -(CH₂CH₂O)_{q}, and r and q are independently selected from 1-25,
wherein, when R₇ is either (i) or (ii) the isotope labeling agent is selected from the group consisting of:
   ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵1, ¹²⁷I, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mln, ¹¹⁵mln, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ₋-OCH₂-COOH, wherein u is an integer from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, and u is an integer from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein R₅ and R₆ are identical or differs only in the isotope number of the labelling agent.

Formula (I) comprises a moiety M which is either one or more natural amino acids, or one or more sugars, or a combination of one or more amino acids and one or more sugars. There are 20 amino acids naturally existing in humans. All of those are chiral amino acids except from glycine, and those chiral amino acids normally have the L-configuration. However, the D-configuration are sometimes present in humans, particularly in relation to certain diseases, thus, the D-configuration of the amino acids are also comprised by the term "natural amino acid" in relation to the M moiety of Formula (I).

The compound according to Formula (I) may comprise none, one or two chelating agents.

The compound according to Formula (I) does not comprise a chelator when both L and X comprises J (i) or J (ii).

Accordingly, Formula (I) does not comprise a chelator when L is a moiety comprising J, wherein J is (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or - (CH₂CH₂O)ₕ, where b and h are independently selected from 1-25, wherein the isotope labeling agent is selected from the group consisting of: ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵1, ¹²⁷I, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mln, ¹¹⁵mln, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt; and when X L is a moiety comprising J, wherein J is (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or -(CH₂CH₂O)ₕ, where b and h are independently selected from 1-25, wherein the isotope labeling agent is selected from the group consisting of: ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵1, ¹²⁷I, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mln, ¹¹⁵mln, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt.

The compound according to Formula (I) comprises only one chelator when L is a moiety comprising a chelating agent G and X is a moiety comprising J (i) or J (ii). Also, the compound according to Formula (I) comprises only one chelator when X is a moiety comprising G and L is a moiety comprising J (i) or J (ii).

Accordingly, in one embodiment, L is a moiety comprising a chelating agent G and X is a moiety comprising J, wherein J is (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or -(CH₂CH₂O)ₕ, where b and h are independently selected from 1-25, wherein the isotope labeling agent is selected from the group consisting of: ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵1, ¹²⁷1, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mln, ¹¹⁵mln, 175_{Lu}, 177_{Lu}, 185_{Re}, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹B, ²¹² Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵Mpt, ¹⁹²Pt, ¹⁹⁴Pt, ¹⁹⁵Pt, ¹⁹⁶Pt, ¹⁹⁸Pt.

In one embodiment, X is a moiety comprising a chelating agent G and L is a moiety comprising J, wherein J is (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or - (CH₂CH₂O)ₕ, where b and h are independently selected from 1-25, wherein the isotope labeling agent is selected from the group consisting of: ¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵1, ¹²⁷1, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mln, ¹¹⁵mln, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ^{2 13}Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt.

The compound of formula 1 comprises two chelators, when L is a moiety comprising a chelating agent G and X is a moiety comprising J (iii). Also, the compound according to Formula (I) comprises two chelators when X is a moiety comprising G and L is a moiety comprising J (iii). Also, the compound according to Formula (I) comprises two chelators when X is a moiety comprising J (iii) and L is a moiety comprising J (iii).

Accordingly, in one embodiment, L is a moiety comprising a chelating agent G and X is a moiety comprising J, wherein J is (iii) an isotope labeling agent that is chelated through a chelating agent connected to the aromatic ring through a linker, wherein the linker is selected from the group consisting of -(CH₂)ᵣ, -WO(CH₂)ᵣ, - WNH(CH₂)ᵣ, -WCONH(CH₂)ᵣ, -WNHCO(CH₂)ᵣ, where W is -(CH₂)_{q} or -(CH₂CH₂O)_{q}, and r and q are independently selected from 1-25.

In one embodiment, X is a moiety comprising a chelating agent G and L is a moiety comprising J, wherein J is (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, - WCONH(CH₂)ᵣ, -WNHCO(CH₂)ᵣ, where W is -(CH₂)_{q} or -(CH₂CH₂O)_{q}, and r and q are independently selected from 1-25.

In one embodiment, L is a moiety comprising J, wherein J is (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, -WCONH(CH₂)ᵣ, -WNHCO(CH₂)ᵣ, where W is -(CH₂)_{q} or -(CH₂CH₂O)_{q}, and r and q are independently selected from 1-25; and X is a moiety comprising J, wherein J is (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, - WCONH(CH₂)ᵣ, -WNHCO(CH₂)ᵣ, where W is -(CH₂)_{q} or -(CH₂CH₂O)_{q}, and r and q are independently selected from 1-25.

In those embodiments wherein Formula (I) comprises two chelators, the chelators can be the same or they can be different.

Regardless of whether Formula (I) comprises one or two chelators, the following chelators are preferred and can be selected independently:
1,4,7,10-tetraazacyclododecane-N,N',N',N"-tetraacetic acid (DOTA),
N,N'-bis(2-hydroxy-5-(carboxyethyl)benzyl)ethylenediamine N,N'-diacetic acid (HBED-CC),
14,7-triazacyclononane-1,4,7-triacetic acid (NOTA),
2-(4.7-bis(carboxymethyl)-1,4,7-triazonan-1-yl)pentanedioic acid (NODAGA),
2-(4,7,10-tris(carboxymethyl)-1,4,7,10-tetraazacyclododecan-1- yl)pentanedioic acid (DOTAGA),
14,7-triazacyclononane phosphinic acid (TRAP),
14,7-triazacyclononane-1-methyl(2-carboxyethyl)phosphinic acid-4,7-bis(methyl(2-hydroxymethyl)phosphinic acid (NOPO),
3,6,9,15-tetraazabicyclo9.3.1.pentadeca-1 (15),11,13-triene-3,6,9- triacetic acid (PCTA),
N'-(5-acetyl (hydroxy)aminopentyl-N-(5-(4-(5- aminopentyl)(hydroxy)amino-4-oxobutanoyl)amino)pentyl-N- hydroxysuccinamide (DFO),
diethylenetriaminepentaacetic acid (DTPA),
trans-cyclohexyl-diethylenetriaminepentaacetic acid (CHX-DTPA),
1-oxa-4,7,10-triazacyclododecane-4,7,10-triacetic acid (OXO-Do3A),
p-isothiocyanatobenzyl-DTPA (SCN-BZ-DTPA),
1-(p-isothiocyanatobenzyl)-3-methyl-DTPA (1B3M),
2-(p-isothiocyanatobenzyl)-4-methyl-DTPA (1M3B),
1-(2)-methyl-4-isocyanatobenzyl-DTPA (MX-DTPA), that can comprise a metal; and pharmaceutically acceptable salts thereof.

The purpose of including one or more chelating agents into Formula (I) is either to optimize the polarity of Formula (I) or because of the technical feature provided by a metal bound by the chelator.

Thus, in some embodiments, the one or more chelating agents do not comprise any metals.
In other embodiments, the one or more chelating agents comprises a metal. If two chelators comprising a metal are present in Formula (I), the metals comprised by the chelators can be the same metal or the metal can be individually selected.

In a preferred embodiment, the one or more chelating agent comprises a metal selected from the group consisting of:⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ²¹³Bi, ⁴⁵Sc, ⁴⁷Sc, ²²⁷Th, ²³²Th, ⁹⁴Tc, ^{99m}Tc, ¹⁰³Rh, ¹⁰³mRh, ¹¹⁹Sb, ¹²¹Sb, ¹²³Sb, ¹⁹³mPt, ¹⁹⁵mPt, ¹⁹²Pt, ¹⁹⁴Pt, ¹⁹⁵Pt, ¹⁹⁶Pt, ¹⁹⁸Pt, ²²⁵Ac.

These metals are known to be chelatable and their use in therapy, imaging and/or theranostics are commonly known.

The radionuclide (Hal) is present in the R₁ group in Formula (I) or on the R2 group in Formula (I). The radionuclide is a radionuclide selected from the halogen group. This group comprises radionuclides of Fluorine (F), Chlorine (CI), Bromine (Br), Iodine (I) and Astatine (At).

In preferred embodiments, the PSMA targeting ligand according to Formula (I), is one of the following compounds: 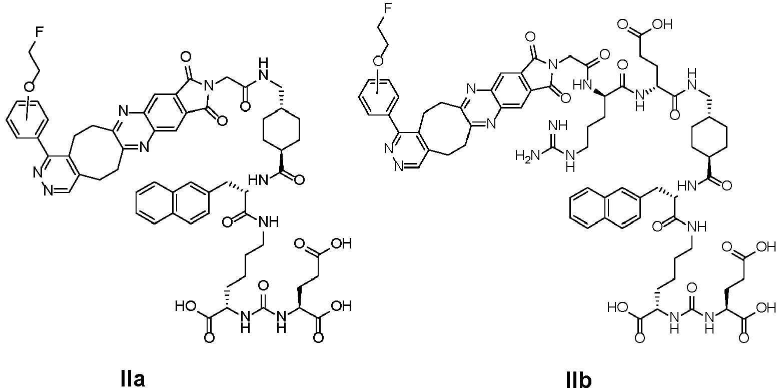 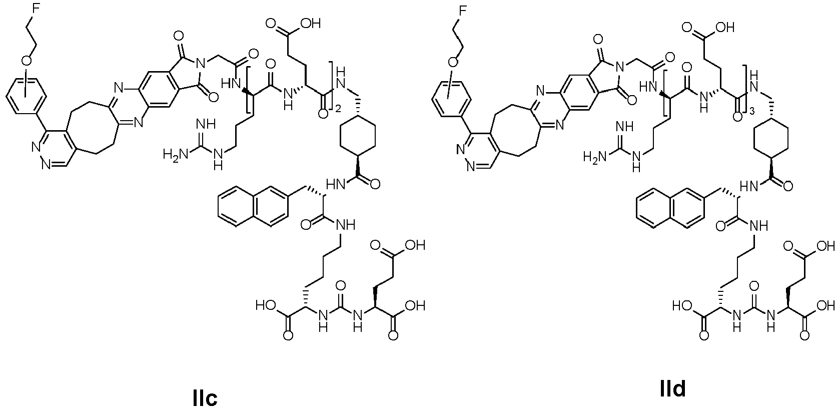 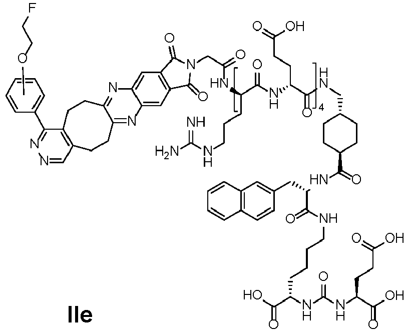 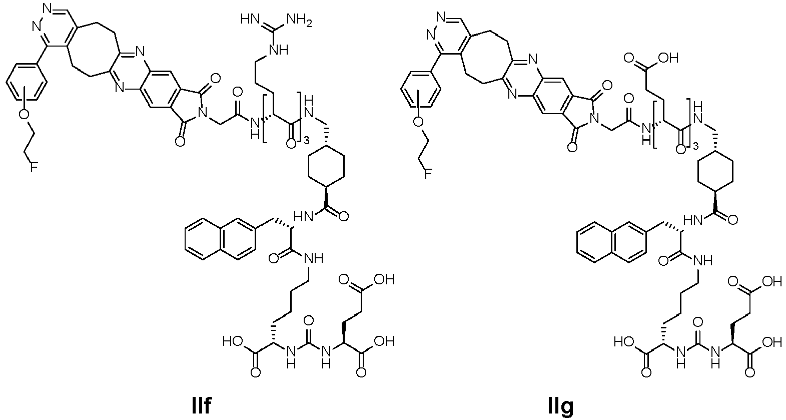 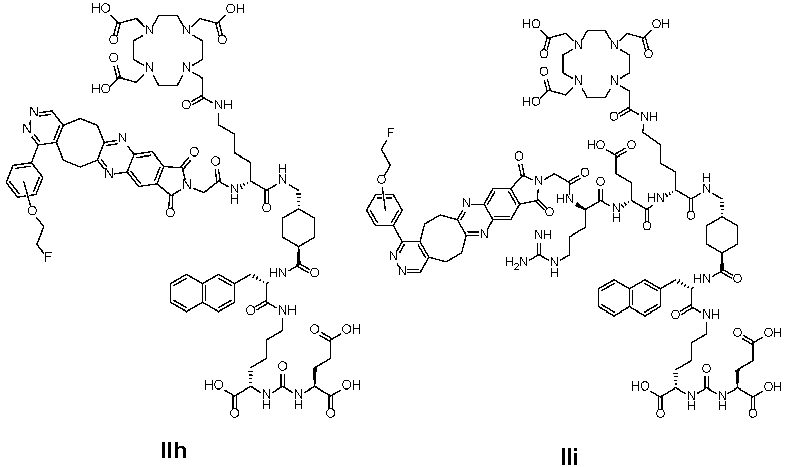 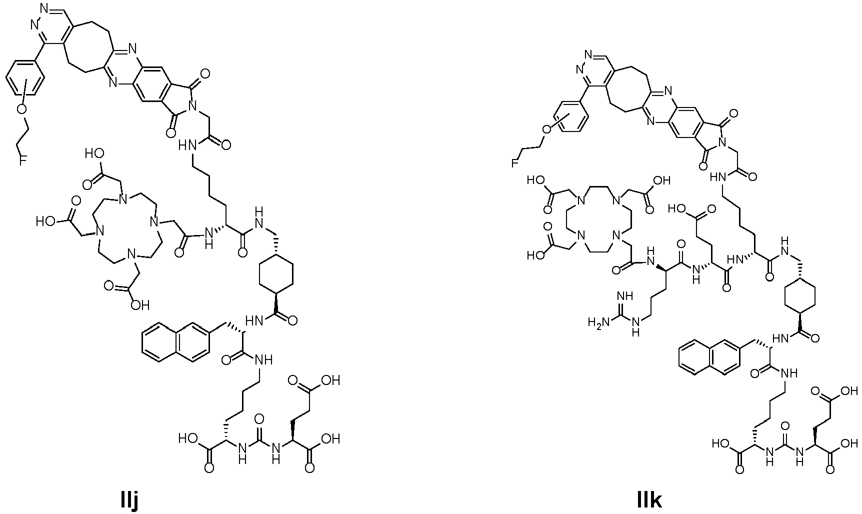 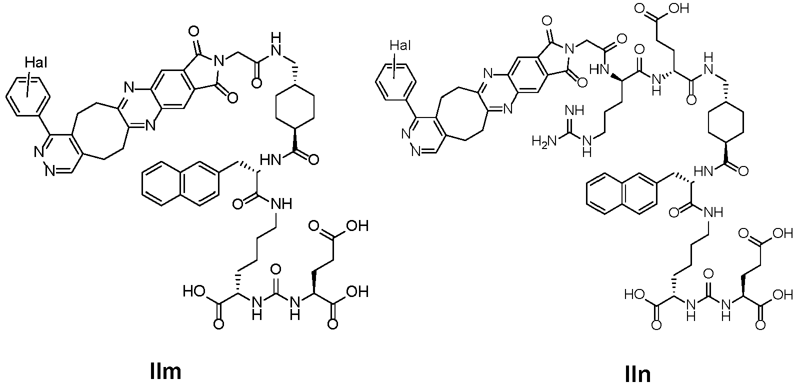 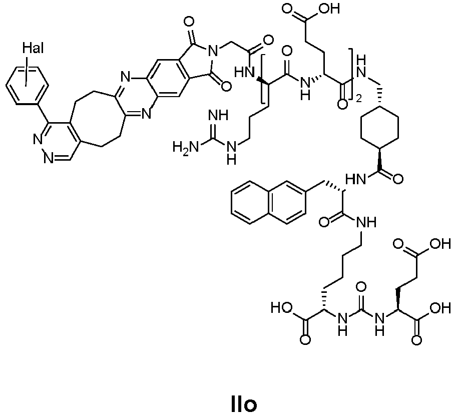 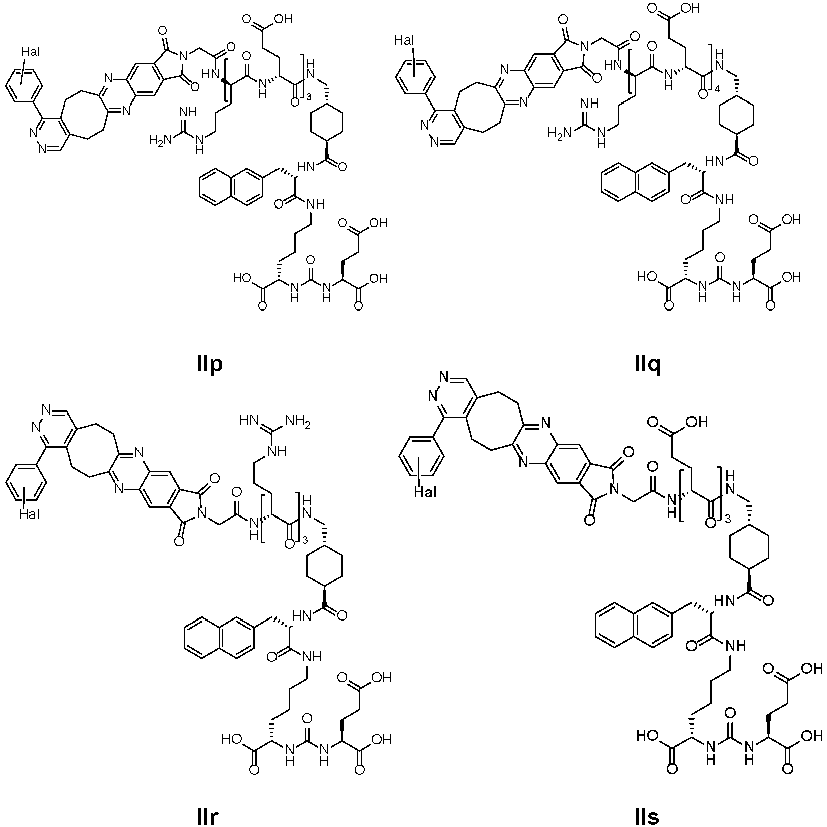 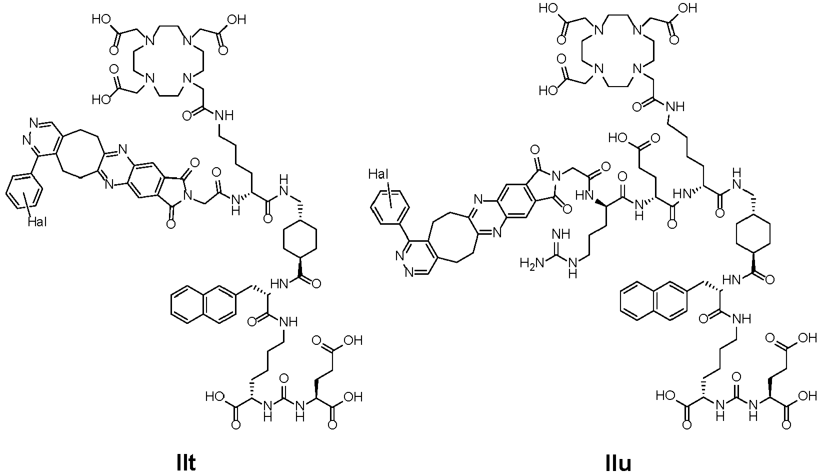 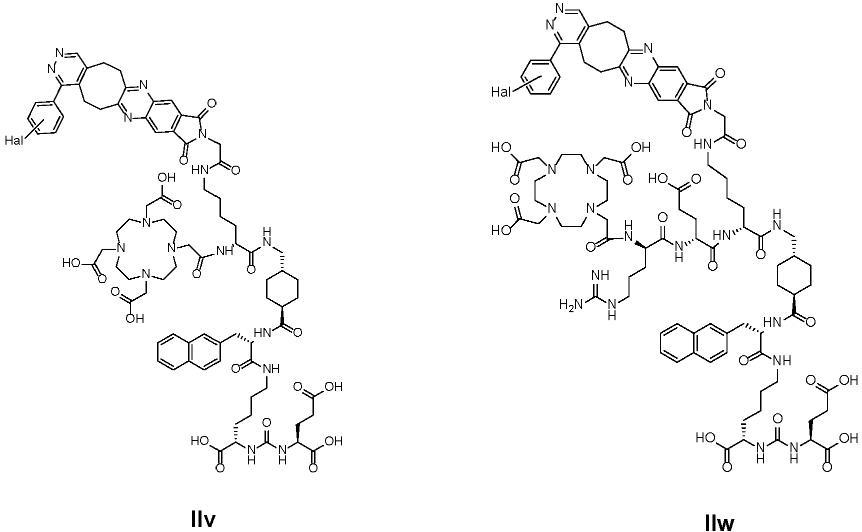 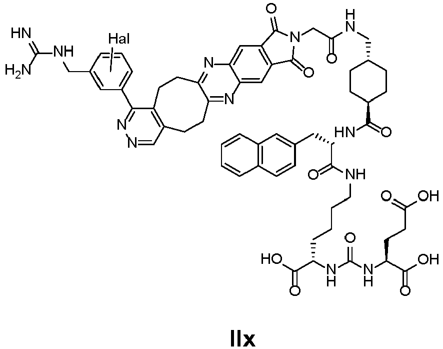 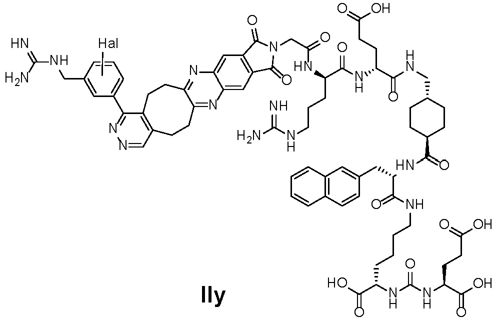 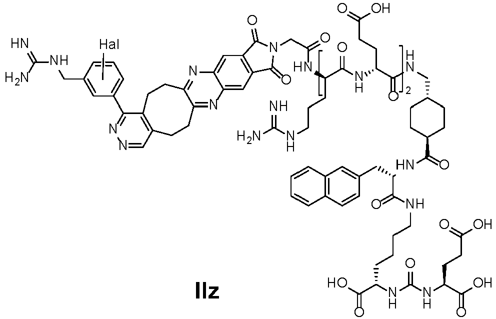 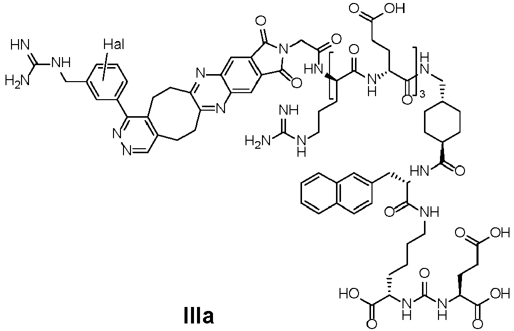 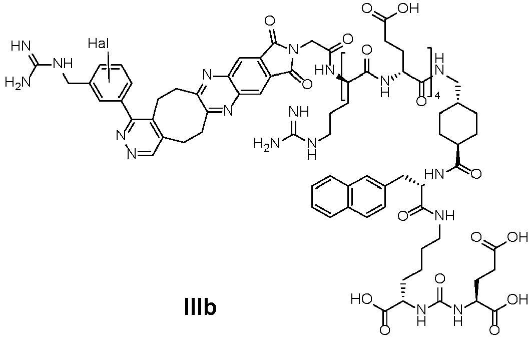 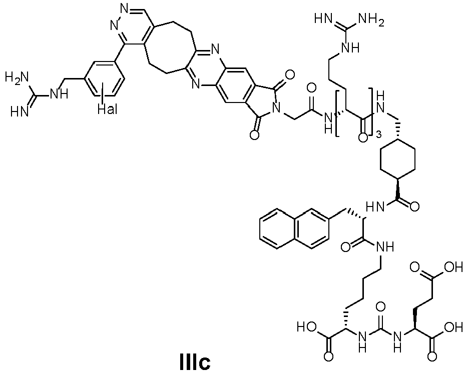 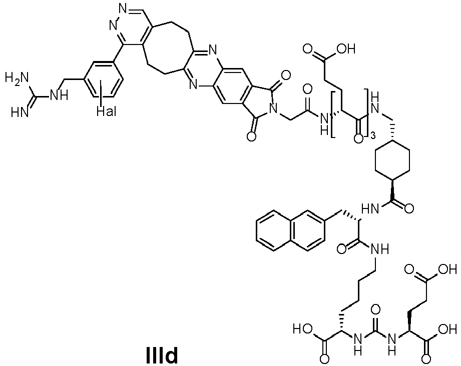 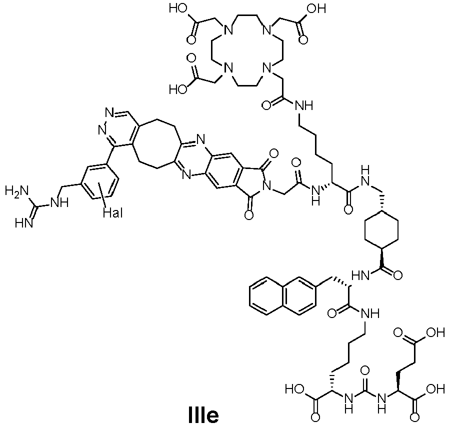 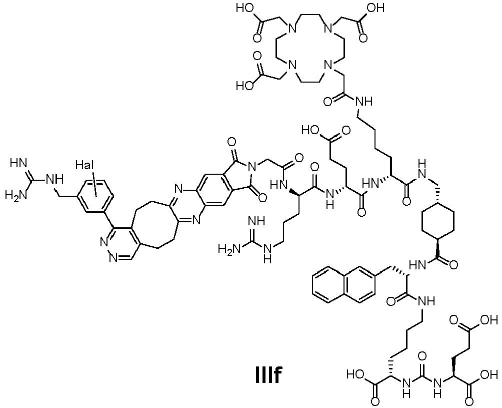 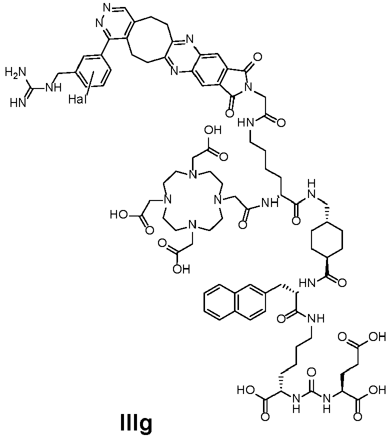 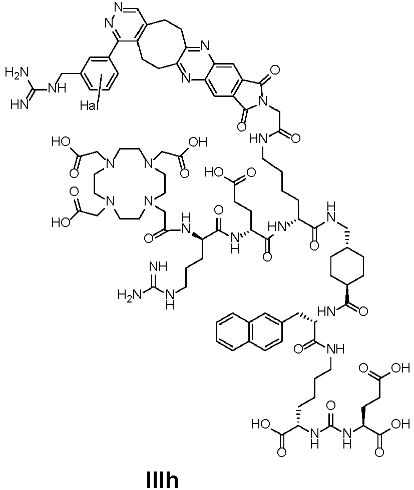 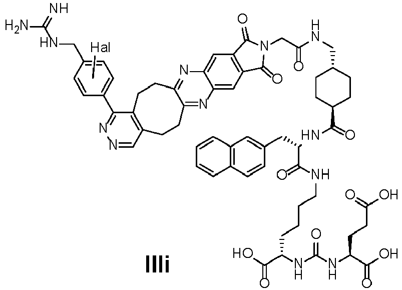 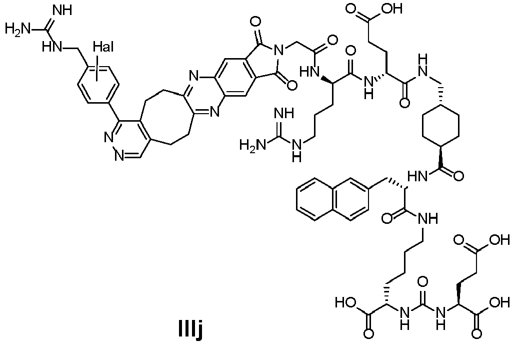 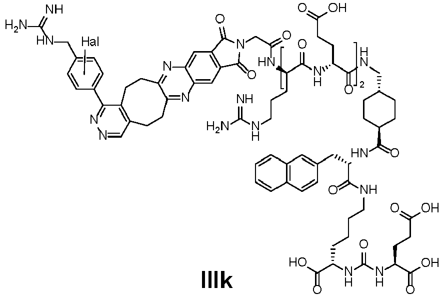 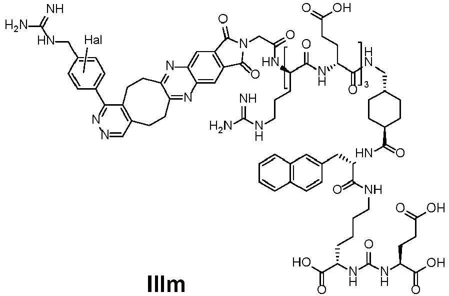 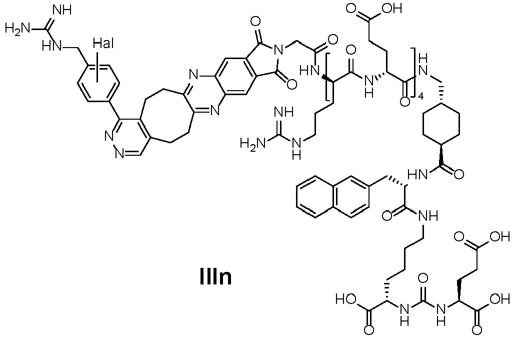 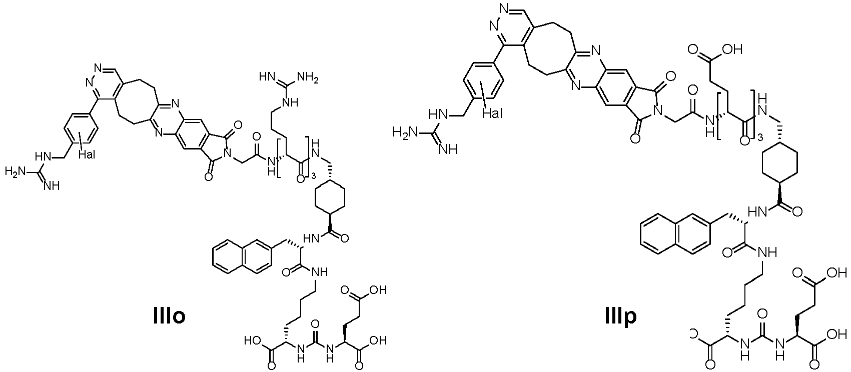 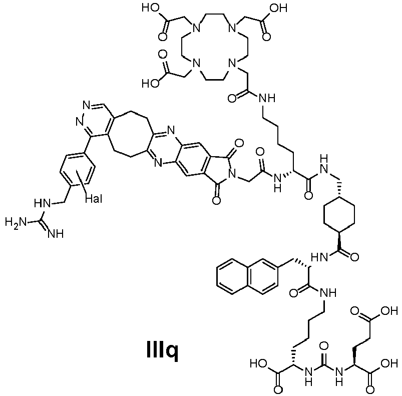 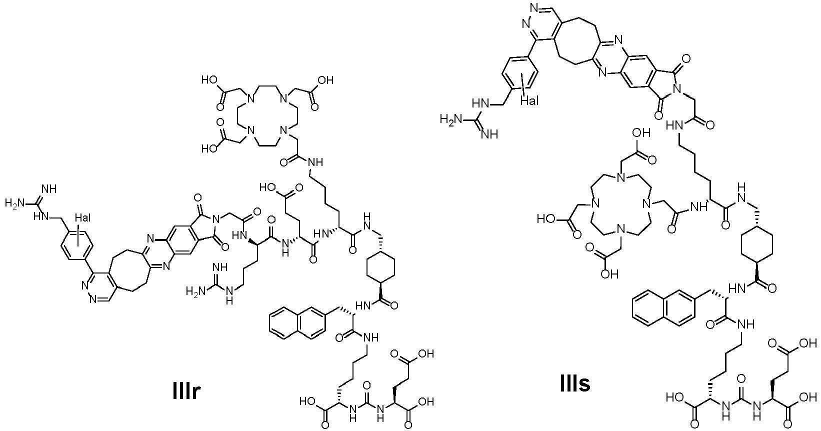 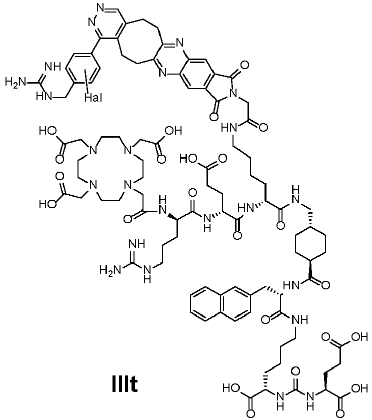 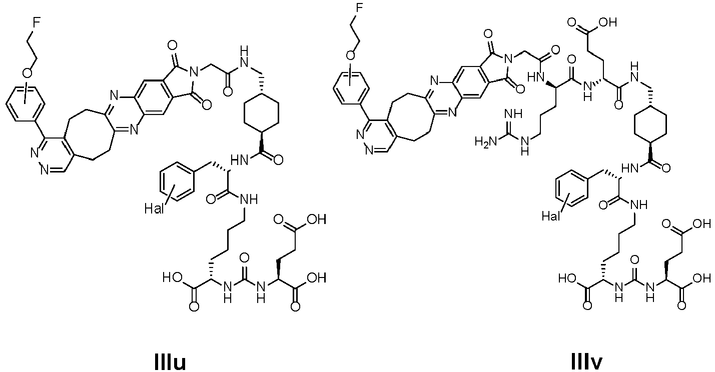 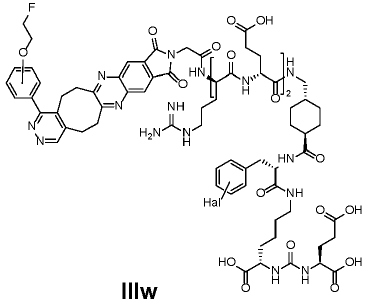 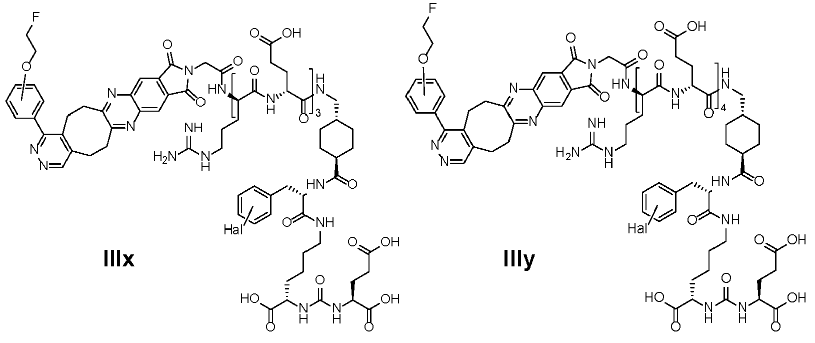 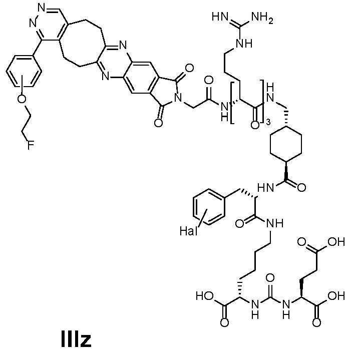 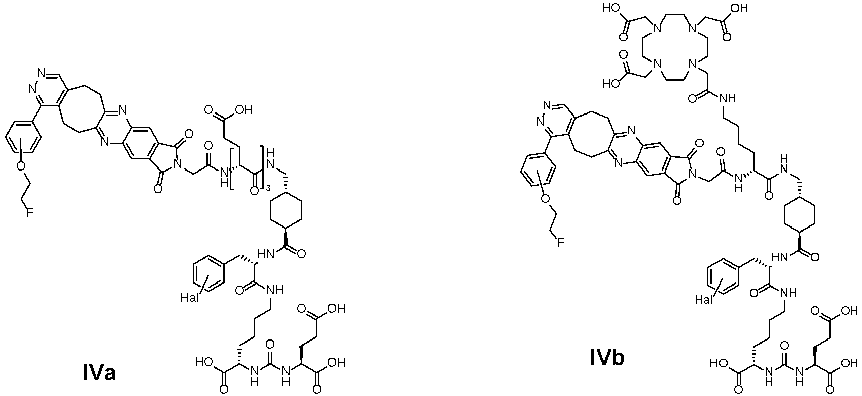 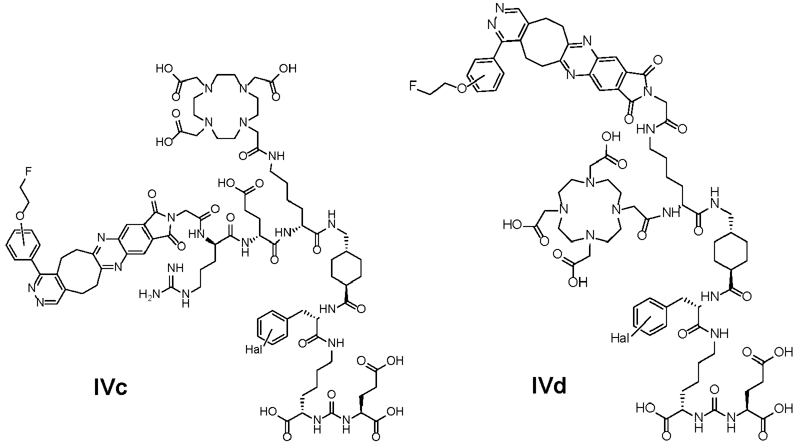 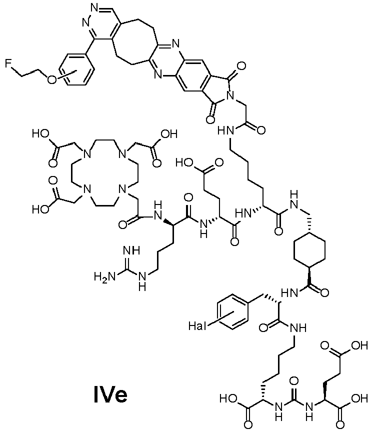
wherein F is ¹⁸F or ¹⁹F
and Hal is ¹⁸F, ¹⁹F, ¹⁸F, ¹²⁵I, ¹²³I, ¹²⁷I, ¹³¹I, ¹²⁴I, ²¹¹At, ⁷⁷Br, ⁷⁹Br, or ^{80m}Br

The PSMA targeting ligands according to formula (I) may be provided by suitable methods known in the art.

In one embodiment, the halo radionuclide is selected from a radioisotope of fluorine, iodine, bromine or astatine.

In a preferred embodiment, the halo radionuclide is one of the following radionuclides: ¹⁸F, ¹⁹F, ¹⁸F, ¹²⁵I, ¹²³I, ¹²⁷I, ¹³¹I, ¹²⁴I, ²¹¹At, ⁷⁷Br, ⁷⁹Br, or ^{80m}Br

The present invention also provides (Me)₃Sn, (Me)₃Si, B(OH)₂ and B(OR₉)₂ precursors that can be used to provide the PSMA targeting ligand according to Formula (I).

These precursors include (Me)₃Sn, (Me)₃Si, B(OH)₂ and B(OR₉)₂ with and without chelators and have the following structures: 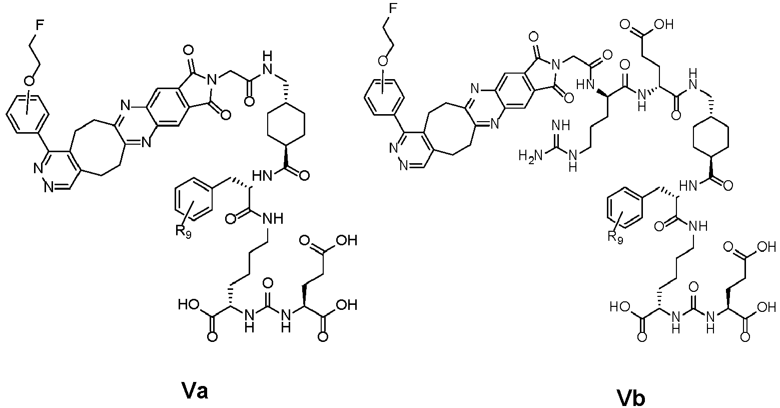 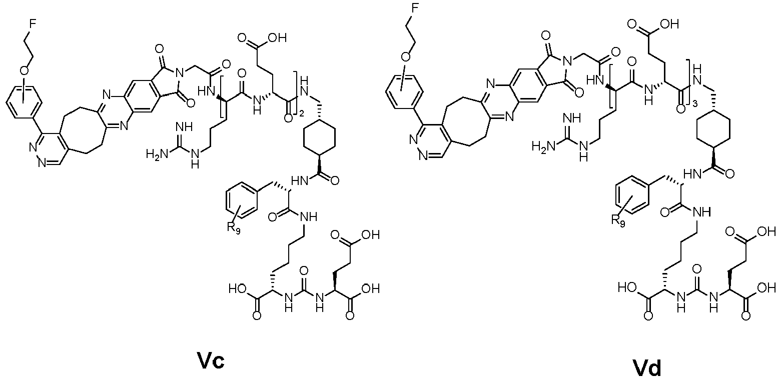 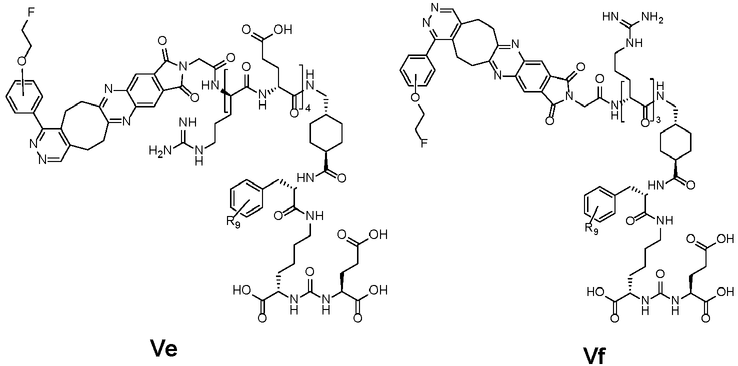 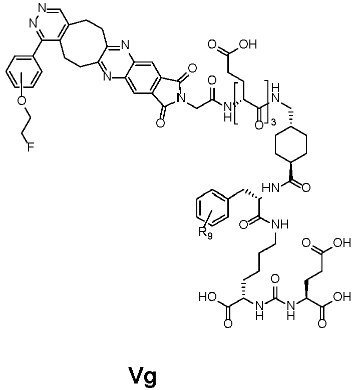 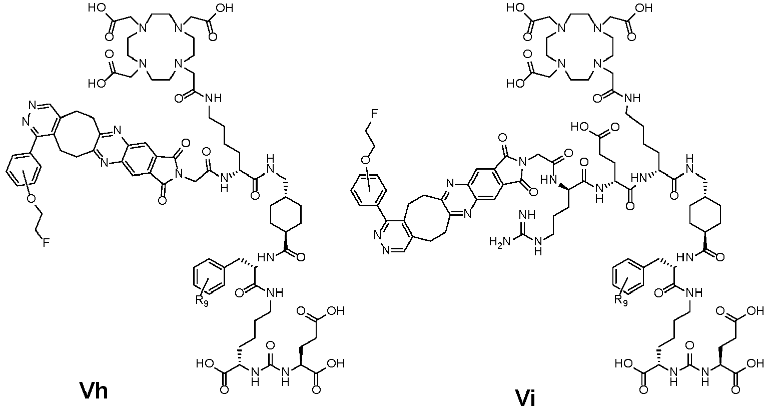 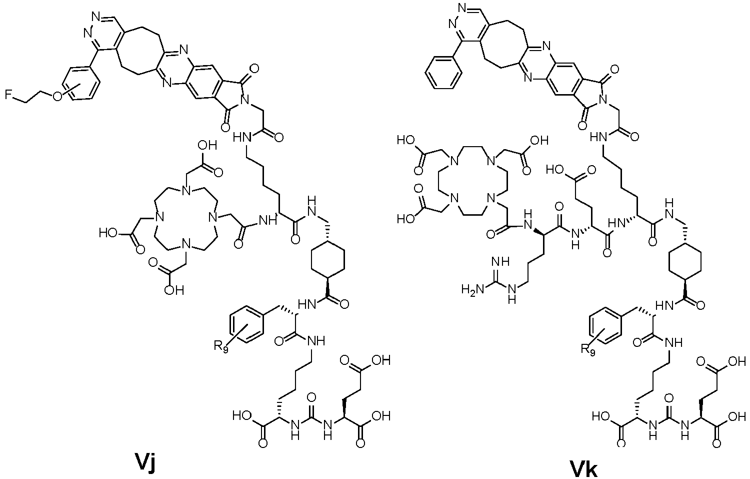 Wherein R₉ is -Si(CH₃)₃, -Si(CH₂CH₂CH₂CH₃)₃, -Sn(CH₃)₃, -Sn(CH₂CH₂CH₂CH₃)₃,-B(OH)₂, or

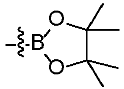

The PSMA targeting ligands of formula (I) can be used in radiotherapy, as imaging agents, such as diagnostic agents, or both i.e. as theranostic agents.

The internalization of the PSMA targeting ligands of formula (I) that was tested outperform the already existing alpha radionuclide based probes.

Formula (I) would suitably be comprised by a pharmaceutical formulation, such as a liquid pharmaceutical formulation for injection. Thus, in one aspect, the present invention relates to a pharmaceutic formulation comprising a PSMA targeting urea-based ligand according to Formula (I).
The PSMA targeting urea-based ligand of formula (I) are suitable for use as a medicament. Thus, in one aspect, the present invention relates to use of a PSMA targeting urea-based ligand according to Formula (I) as a medicament.
The specific use of Formula (I) as a medicament would depend on the specific radioisotope and optionally on the chelated metal. The potential use of the various isotopes and optionally metals, that can be comprised by Formula (I) in therapy, diagnostics, imaging and theranostic is well known and depends on the irradiation provided by the specific isotopes and metals. Therefore, the PSMA targeting urea-based ligand according to Formula (I) can be modified to suit selected specific uses depending on the choice of radioisotope and optionally on the choice of chelated metal.
Particularly, the PSMA targeting urea-based ligand according to Formula (I) is suitable for use in the treatment of prostate cancer and other cancer forms or metastases expressing PSMA. Thus, in one aspect, the present invention relates to the PSMA targeting urea-based ligand according to Formula (I) for use in the treatment of prostate cancer or other cancer forms or metastases expressing PSMA with isotopes such as ¹⁷⁷Lu, ²²⁵Ac, ²¹¹At, ¹³¹I, ²¹²Pb.
Since PSMA is not exclusively relevant in relation to prostate cancer, the PSMA targeting urea-based ligand according to Formula (I) is as such suitable for use as a radiopharmaceutical. Thus, in one aspect, the present invention relates to the PSMA targeting urea-based ligand according to Formula (I) for use as a radiopharmaceutical with isotopes such as ¹⁷⁷Lu, ²²⁵Ac, ²¹¹At, ¹³¹I, ²¹²Pb, ⁶⁷Cu.

Moreover, the PSMA targeting urea-based ligand according to Formula (I) is suitable for use as an imaging agent. Thus, in one aspect, the present invention relates to the PSMA targeting urea-based ligand according to Formula (I) for use as an imaging agent with isotopes such as ¹⁸F, ⁶⁸Ga, ⁶⁴Cu, ²⁰³Pb.

Also, the PSMA targeting urea-based ligand according to Formula (I) is suitable for use as a theranostic agent i.e. an agent that combines therapy and diagnostic. This would for instance be when the chelator comprises a metal suitable for imaging and R₄ and/or R₁ or R₂ comprises a radionuclide suitable for therapy. Or when the chelator comprises a metal suitable for therapy and R₄ and/or R₁ or R₂ comprises a radionuclide suitable for imaging. Thus, in one aspect, the present invention relates to the PSMA targeting urea-based ligand according to Formula (I) for use as a theranostic agent such as with the theranostic pairs ¹⁸F/²¹¹At, ¹²³I/¹³¹I, ⁶⁴/Cu/⁶⁷Cu, ⁶⁸Ga/¹⁷⁷Lu ⁶⁸Ga/²²⁵Ac, ²⁰³Pb/²¹²Pb.

### Examples:

### Example 1: Synthesis of general reagents

### 3-(4-(2-Fluoroethoxy)phenyl)-1,2,4,5-tetrazine (Compound 1)

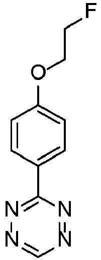

The compound was synthesized as previously described in the literature to afford 0.36 g (37%) of the desired compound as a red oil.¹³ R_{f}= 0.33 (Heptane/EtOAc 80/20); ¹H NMR (400 MHz, CDCl₃) δ 10.07 (s, 1H), 8.53 (d, *J* = 8.9 Hz, 2H), 7.06 (d, *J* = 8.9 Hz, 2H), 4.89 - 4.78 (m, 1H), 4.74 - 4.62 (m, 1H), 4.43 - 4.29 (m, 1H), 4.27 - 4.16 (m, 1H); ¹³C NMR (101 MHz, CDCl₃) δ 166.06, 162.56, 157.40, 130.26, 124.56, 115.38, 81.63 (d, *J* = 171.5 Hz), 67.29 (d, *J* = 20.6 Hz).

### 2-(4-(1,2,4,5-Tetrazin-3-yl)phenoxy)ethyl 4-nitrobenzenesulfonate (Compound 2)

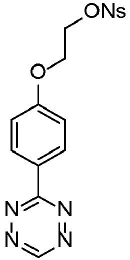

The compound was synthesized as described in the literature 0.12 g (65%) of the desired product as a red solid.¹³ R_{f}= 0.41 (Heptane/EtOAc 50/50); ¹H NMR (400 MHz, DMSO) δ 10.52 (s, 1H), 8.97 - 8.31 (m, 5H), 8.33 - 7.87 (m, 2H), 7.11 (d, *J* = 8.9 Hz, 2H), 4.97 - 4.50 (m, 2H), 4.48 - 4.06 (m, 2H); ¹³C NMR (101 MHz, DMSO) δ 165.57, 161.88, 158.28, 154.85, 147.71, 130.25, 127.38, 125.26, 123.79, 116.05, 75.01, 67.33.

### 3-(4-iodophenyl)-1,2,4,5-tetrazine (Compound 3)

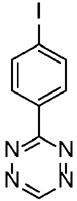

The compound was synthesized as previously described.¹⁴ 4-iodobenzonitrile (0.92 g, 4.0 mmol), CH₂Cl₂ (0.26 mL, 4.0 mmol), sulfur (0.26 g, 1.0 mmol), and ethanol (4.0 mL) were mixed together in a microwave reaction vial. Hydrazine monohydrate (1.55 mL, 32.00 mmol) was added dropwise with stirring. The vessel was sealed, and the reaction mixture was heated to 50 °C for 24 h. The reaction was diluted with 3 mL of CH₂Cl₂, and sodium nitrite (2.76 g, 40.00 mmol) in 30 mL of H₂O was added dropwise to the mixture under cooling. Excess acetic acid (14 mL) was then added slowly, during which the solution turned bright red in color. The reaction mixture was extracted with CH₂Cl₂ (3 × 30 mL). The organic phase was dried over MgSO₄ and concentrated under reduced pressure. The resulting residue was purified using flash chromatography (n-Heptane/EtOAc 95/5) to afford 0.36 g (29%) of the desired compound as a red oil (mixture of rotamers). The crude was purified using flash chromatography (90/10 n-Heptane/EtOAc) to yield 0.30 mg (27%) of a pink solid. Rf = 0.37 (n-Heptane:10%EtOAC); ¹H NMR (400 MHz, Chloroform-*d*) δ 10.24 (s, 1H), 8.39 - 8.32 (m, 2H), 8.02 - 7.95 (m, 2H).; ¹³C NMR (101 MHz, CDCl₃) δ 166.36, 158.10, 138.90, 131.24, 129.75, 101.22.

### 3-(4-trimethyltin)-6-methyl-1,2,4,5-tetrazine (Compound 4)

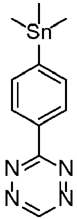

The compound was synthesized s previously described.¹⁴ Pd(PPh₃)₄ (19.4 mg, 10%) and hexamethylditin (87 µL, 0.42 mmol, 2.5 equiv.) were successively added to a microwave vial equipped with a stir bar which was then sealed and purged with N₂. **3** (0.05 g, 0.17 mmol) in dry THF (2.5 mL) was added via a syringe and the reaction allowed to stir at 65 °C in a microwave for 2 hours. The reaction was allowed to cool to room temperature and unsealed before being quenched with saturated aqueous KF (1 mL). The solution was extracted with CH₂Cl₂ washed with brine (2x5 mL), dried over MgSO₄, filtered and concentrated under reduced pressure. The tetrazine was then purified using flash chromatography (90/10 n-Heptane/EtOAc) to yield 0.27 g (61%) of a pink solid. Rf = 0.43 (n-Heptane:20%EtOAc); ¹H NMR (400 MHz, Chloroform-*d*) δ 10.20 (s, 1H), 8.58 - 8.54 (m, 2H), 7.84 - 7.67 (m, 2H), 0.37 (s, 9H).; ¹³C NMR (101 MHz, Chloroform-*d*) δ 167.00, 157.96, 150.34, 136.90, 130.29, 127.35, -9.33.; HPLC-MS [M+H]⁺ m/z calc. for [C₁₁H₁₅SnN₄]⁺: 323.04; Found: 323.38.

### Synthesis of 2,5-dioxopyrrolidin-1-yl (E)-2-(1,3-dioxo-1,3,6,7,10, 11-hexahydro-2H-cycloocta[bjpyrrolo[3,4-gjquinoxalin-2-yl)acetate (Compound 5)

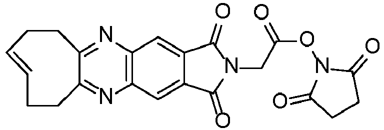

The compound was synthesized as previously reported to give 0.056 g (72%) of the desired compound as a yellow solid (PCT/EP2023/055930). R_{f} = 0.41 (n-Heptane/EtOAc 50/50); ¹H NMR (400 MHz, CDCl₃) δ 8.56 - 8.47 (m, 2H), 6.10 (td, J = 12.1, 5.9 Hz, 1H), 5.25 - 5.07 (m, 1H), 4.87 (s, 2H), 3.40 - 3.30 (m, 2H), 3.30 - 3.21 (m, 1H), 3.15 - 3.04 (m, 1H), 2.92 - 2.68 (m, 6H), 2.45 (q, *J =* 11.9 Hz, 1H), 2.33 (dt, *J =* 12.1, 6.1 Hz, 1H); ¹³C NMR (101 MHz, CDCl₃) δ 168.16, 165.58, 163.15, 161.67, 161.31, 143.40, 143.24, 136.54, 132.67, 130.16, 130.00, 126.26, 126.07, 46.74, 41.81, 37.05, 34.19, 28.26, 25.55.

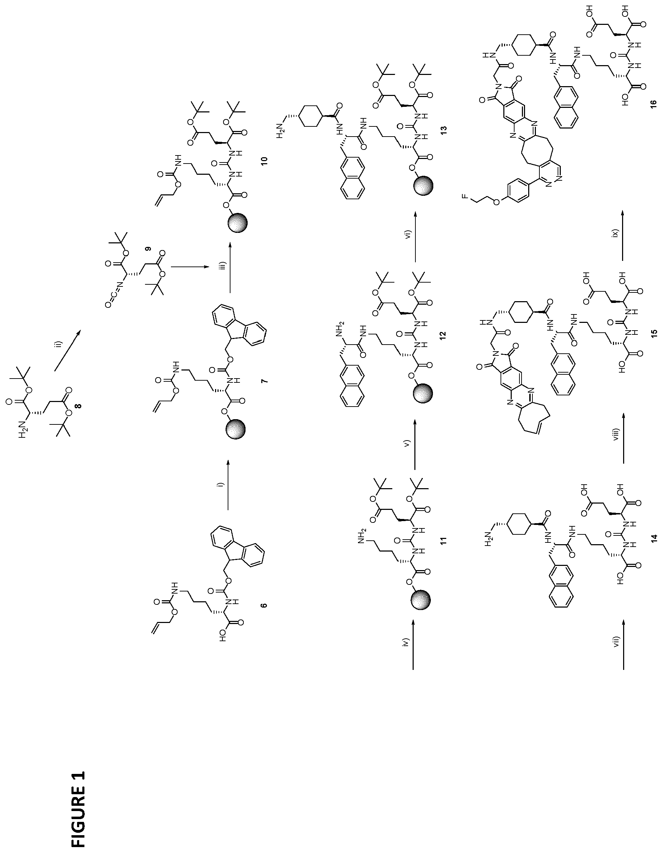

### Example 2: Synthesis of PSMA-NH-T4CO (Compound 15, Figure 1)

Figure 1 is a scheme showing the synthesis of compound **16.** The grey dot in the intermediate structures symbolizes a resin. The following reagents and conditions were used in the steps i - ix: i) 2CT-resin, DIPEA, CH₂Cl₂, rt, 12 h, ii) Triphosgene, DIPEA, CH₂Cl₂, 0 °C, 20 min.; iii) DCM, 0 °C to rt, 12 h; iv) Pd(PPh₃)₄, Morpholine, CH₂Cl₂, rt, 4 h; v) a) Fmoc-L-Nal-OH, HATU, DIPEA, DMF, rt, 2 h, b) 50% piperidine in DMF, rt, 20 min.; vi) N-Fmoc-tranexamic acid, HATU, DIPEA, DMF, rt, 2 h, b) 50% piperidine in DMF, rt, 20 min.;vii) TFA, TIPS, H₂O, rt, 2 h; viii) **5,** 4-methylmorpholine, DMF, rt, 6 h, ix) **1,** ACN, H₂O, TFA, rt, 5 h.

### Synthesis of PSMA-NH₂ (Compound 14, Scheme 1)

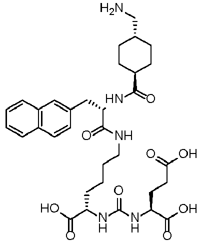

The resin-bound and tert-butyl-protected binding motif **(11)** was synthesized as previously described.⁵ Relative to the resin (0.22 mmol), 4 equiv. of Fmoc-L-2-Nal-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized Glutamate-urea-Lysine and shaken for 1 hour. The Fmoc-protecting group was cleaved with a mixture of DMF and piperidine (1:1). The product **(12)** was reacted with 4 equiv. of trans-4-(Fmoc-aminomethyl)cyclohexanecarboxylic acid (N-Fmoctranexamic acid) activated with 3.92 equiv. of HATU and 4 equiv. of DIPEA. Deprotection of the Fmoc group with a mixture of DMF and piperidine (1:1) gave **13.** The product was cleaved from the resin and deprotected with trifluoroacetic acid (TFA), triisopropylsilane (TIPS) and water (95:2.5:2.5). Preparative HPLC afforded 0.110 g of the desired compound **14.** ¹H NMR (600 MHz, DMSO) δ 12.74 - 12.36 (m, 2H), 12.14 (s, 1H), 7.98 - 7.93 (m, 2H), 7.86 (dd, *J* = 7.7, 1.6 Hz, 1H), 7.82 - 7.77 (m, 2H), 7.69 (d, *J* = 1.7 Hz, 1H), 7.60 (s, 3H), 7.49 - 7.43 (m, 2H), 7.40 (dd, *J* = 8.4, 1.7 Hz, 1H), 6.34 - 6.25 (m, 2H), 4.55 (td, *J* = 9.0, 5.1 Hz, 1H), 4.11 (td, *J* = 8.3, 5.2 Hz, 1H), 4.03 (td, *J* = 8.2, 5.2 Hz, 1H), 3.12 (dd, J*=* 13.7, 5.0 Hz, 1H), 3.06 (dq, *J*= 13.0, 6.6 Hz, 1H), 2.99 (dq, *J* = 13.0, 6.7 Hz, 1H), 2.93 (dd, *J* = 13.7, 9.5 Hz, 1H), 2.66 - 2.59 (m, 3H), 2.31 - 2.18 (m, 2H), 2.09 (ddt, *J =* 12.0, 7.5, 3.6 Hz, 1H), 1.93 (dddd, *J =* 14.1, 9.3, 6.7, 5.2 Hz, 1H), 1.77 - 1.56 (m, 5H), 1.55 - 1.19 (m, 9H), 1.05 (qd, *J* = 13.0, 3.5 Hz, 1H), 0.93 - 0.81 (m, 2H); ESI-MS [M+H]⁺= 656.3.

### Synthesis of PSMA-NH-T4CO (Compound 15, Figure 1)

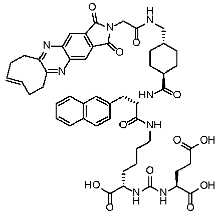

To a solution of **14** (0.03 g, 0.046 mmol) and **5** (0.02 g, 0.046 mmol) in dry DMF (3 mL) under argon, was added 4-methylmorpholine (0.03 mL, 0.27 mmol). The reaction was stirred at room temperature for 6 h. The mixture was then diluted with 10 mL of mobile phase A (1%TFA in H₂O) and purified by preparative HPLC. The collected fractions were freeze-dried to give 0.03 g (64%) of the desired compound as a white solid. ESI-MS [M+H]⁺= 976.4.

### Example 3: Synthesis of PSMA-NH-T4CO-FTz (Compound 16, Figure 1)

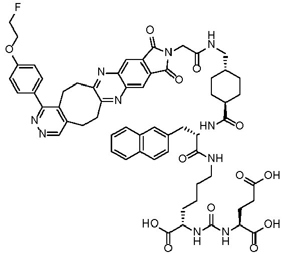

To a solution of **15** (0.005 g, 0.0051 mmol) in ACN (2 mL) and 1% TFA in H₂O (2 mL) was added **1** (0.001 g, 0.0051 mmol). The reaction was stirred at room temperature for 24 h. The mixture was directly purified by preparative HPLC. The collected fractions were freeze-dried to give 0.003 g (50%) of the desired compound as a white solid. ESI-MS [M+H]⁺= 1165.3.

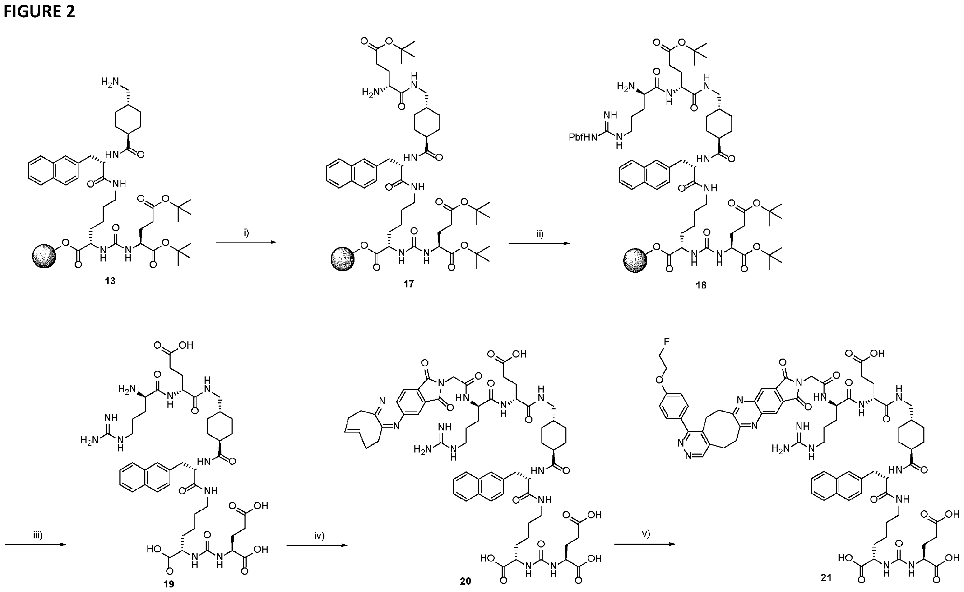

### Example 4: Synthesis of PSMA-AA-NH-T4CO (Compound 20, Figure 2)

Figure 2 is a scheme showing the synthesis of compound 21. The grey dot in the intermediate structures symbolizes a resin. The following reagents and conditions were used in steps i) - v): i)a) Fmoc-D-Glu-OH, HATU, DIPEA, DMF, rt, 2 h, b) 50% piperidine in DMF, rt, 20 min.; ii)a) Fmoc-D-Arg(Pbf)-OH, HATU, DIPEA, DMF, rt, 2 h, b) 50% piperidine in DMF, rt, 20 min.; iii) TFA, TIPS, H₂O, rt, 2 h; iv) **5,** 4-methylmorpholine, DMF, rt, 6 h; v) **1,** ACN, H₂O, TFA, rt, 5 h.

### Synthesis of PSMA-AA-NH₂ (Compound 19, Figure 2)

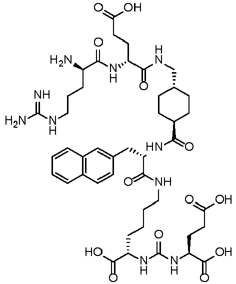

Relative to the resin and **13** (0.22 mmol), 4 equiv. of Fmoc-D-Glu-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After2 min, the solution was added to the resin-immobilised Glutamate-urea-Lysine and shaken for 1 hour. The Fmoc-protecting group was cleaved with a mixture of DMF and piperidine (1:1). The product **(17)** was reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave **18.** The product was cleaved from the resin and deprotected with trifluoroacetic acid (TFA), triisopropylsilane (TIPS) and water (95:2.5:2.5). Preparative HPLC afforded 0.09 g of the desired compound **19.** ESI-MS [M+H]⁺= 941.3.

### Synthesis of PSMA-AA-NH-T4CO (Compound 20, Figure 2)

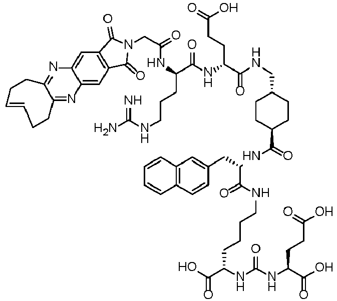

To a solution of **19** (0.035 g, 0.030 mmol) and **5** (0.013 g, 0.030 mmol) in dry DMF (3 mL) under argon, was added 4-methylmorpholine (0.03 mL, 0.27 mmol). The reaction was stirred at room temperature for 6 h. The mixture was then diluted with 10 mL of mobile phase A (1%TFA in H₂O) and purified by preparative HPLC. The collected fractions were freeze-dried to give 0.011 g (28%) of the desired compound as a white solid. ESI-MS [M+H]⁺= 1261.1.

### Example 5: Synthesis of PSMA-NH-AA-T4CO-FTz (21, Figure 2)

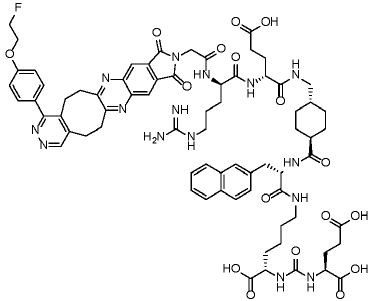

To a solution of **20** (0.005 g, 0.0036 mmol) in ACN (2 mL) and 1% TFA in H₂O (2 mL) was added **1** (0.0008 g, 0.0036 mmol). The reaction was stirred at room temperature for 24 h. The mixture was directly purified by preparative HPLC. The collected fractions were freeze-dried to give 0.002 g (38%) of the desired compound as a white solid. ESI-MS [M+H]⁺= 1450.6.

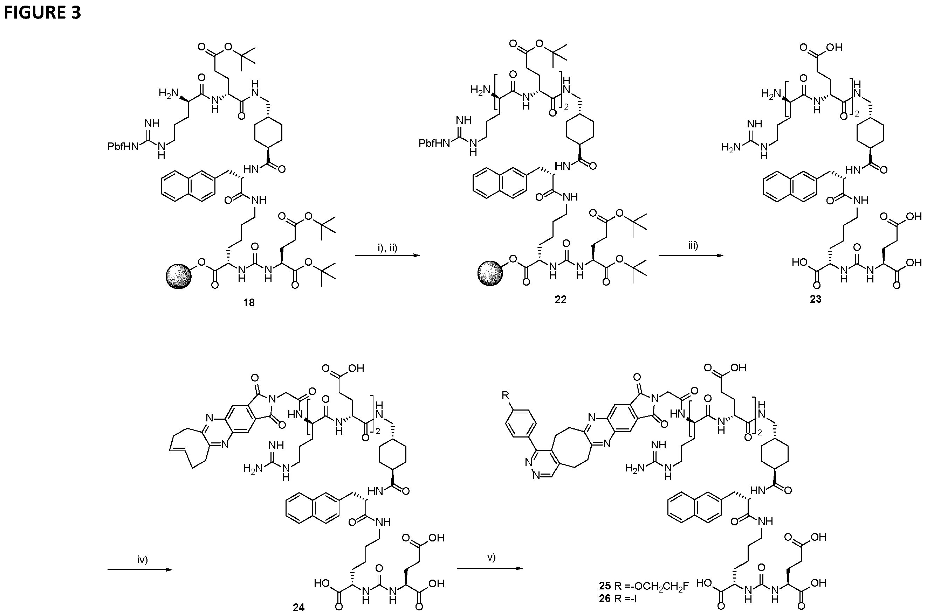

### Example 6: Synthesis of PSMA-AAAA-NH-T4CO (Compound 24, Figure 3)

Figure 3 is a scheme showing the synthesis of compounds 25 and 26. The grey dot in the intermediate structures symbolizes a resin. In compound 25 R = OCH₂CH₂F. In compound 26 R = I. The following reagents and conditions were used in steps i) - v): i)a) Fmoc-D-Glu-OH, HATU, DIPEA, DMF, rt, 2 h, b) 50% piperidine in DMF, rt, 20 min.; ii)a) Fmoc-D-Arg(Pbf)-OH, HATU, DIPEA, DMF, rt, 2 h, b) 50% piperidine in DMF, rt, 20 min.; iii) TFA, TIPS, H₂O, rt, 2 h; iv) **5,** 4-methylmorpholine, DMF, rt, 6 h; v) **1** or **3,** ACN, H₂O, TFA, rt, 5 h.

### Synthesis of PSMA-AAAA-NH₂ (Compound 23, Figure 3)

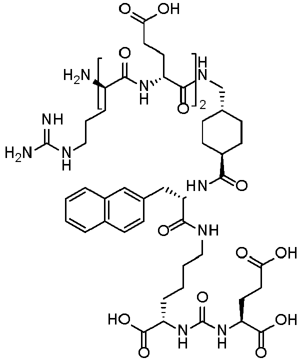

Relative to the resin and **18** (0.22 mmol), 4 equiv. of Fmoc-D-Glu-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After2 min, the solution was added to the resin-immobilized Glutamate-urea-Lysine and shaken for 1 hour. The Fmoc-protecting group was cleaved with a mixture of DMF and piperidine (1:1). The product was reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave **22.** The product was cleaved from the resin and deprotected with trifluoroacetic acid (TFA), triisopropylsilane (TIPS) and water (95:2.5:2.5). Preparative HPLC afforded 0.07 g of the desired compound **23.** ESI-MS [M+H]⁺= 1226.8.

### Synthesis of PSMA-AAAA-NH-T4CO (Compound 24, Figure 3)

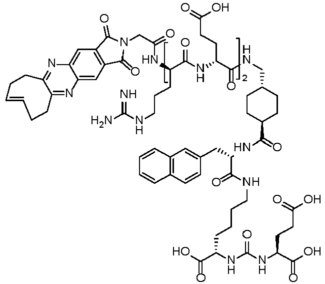

To a solution of **23** (0.026 g, 0.016 mmol) and 5 (0.007 g, 0.016 mmol) in dry DMF (3 mL) under argon, was added 4-methylmorpholine (0.016 mL, 0.15 mmol). The reaction was stirred at room temperature for 6 h. The mixture was then diluted with 10 mL of mobile phase A (1%TFA in H₂O) and purified by preparative HPLC. The collected fractions were freeze-dried to give 0.015 g (51%) of the desired compound as a white solid. ESI-MS [M+H]⁺ = 1546.3.

### Example 7: Synthesis of PSMA-NH-AAAA-T4CO-FTz (Compound 25, Figure 3)

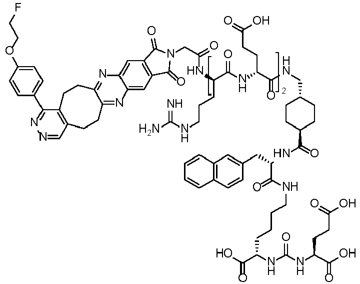

To a solution of **24** (0.00 g, 0.0023 mmol) in ACN (2 mL) and 1% TFA in H₂O (2 mL) was added **1** (0.0005 g, 0.0023 mmol). The reaction was stirred at room temperature for 24 h. The mixture was directly purified by preparative HPLC. The collected fractions were freeze-dried to give 0.002 g (45%) of the desired compound as a white solid. ESI-MS [M+H]⁺= 1736.6.

### Example 8: Synthesis of PSMA-NH-AAAA-T4CO-ITz (Compound 26, Figure 3)

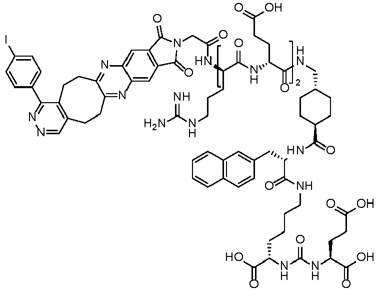

To a solution of **24** (0.004 g, 0.0023 mmol) in ACN (2 mL) and 1% TFA in H₂O (2 mL) was added **3** (0.0005 g, 0.0023 mmol). The reaction was stirred at room temperature for 24 h. The mixture was directly purified by preparative HPLC. The collected fractions were freeze-dried to give 0.002 g (44%) of the desired compound as a white solid. ESI-MS [M+H]⁺= 1799.6.

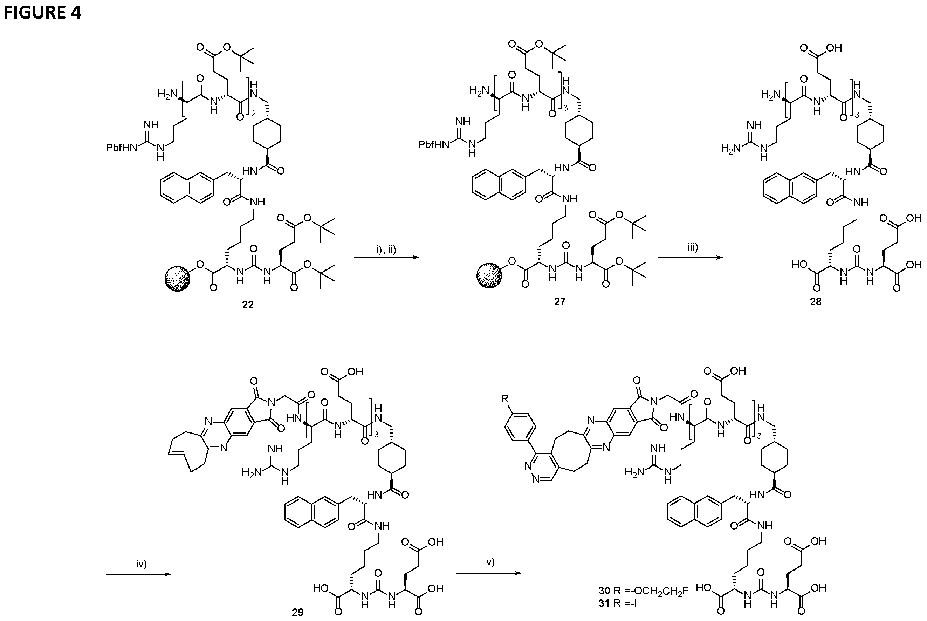

### Example 9: Synthesis of PSMA-AAAAAA-NH-T4CO (Compound 29, Figure 4)

Figure 4 is a scheme showing the synthesis of compounds 30 and 31. The grey dot in the intermediate structures symbolizes a resin. In compound 30 R = OCH₂CH₂F. In compound 31 R = I. The following reagents and conditions were applied in steps i) - v): i)a) Fmoc-D-Glu-OH, HATU, DIPEA, DMF, rt, 2 h, b) 50% piperidine in DMF, rt, 20 min.; ii)a) Fmoc-D-Arg(Pbf)-OH, HATU, DIPEA, DMF, rt, 2 h, b) 50% piperidine in DMF, rt, 20 min.; iii) TFA, TIPS, H₂O, rt, 2 h; iv) **5,** 4-methylmorpholine, DMF, rt, 6 h; v) 1 or 3, ACN, H₂O, TFA, rt, 5 h.

### Synthesis of PSMA-AAAAAA-NH₂ (Compound 28, Figure 4)

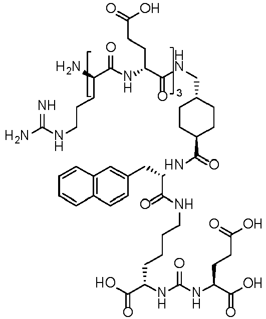

Relative to the resin and **22** (0.22 mmol), 4 equiv. of Fmoc-D-Glu-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After2 min, the solution was added to the resin-immobilized Glutamate-urea-Lysine and shaken for 1 hour. The Fmoc-protecting group was cleaved with a mixture of DMF and piperidine (1:1). The product was reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave **27.** The product was cleaved from the resin and deprotected with trifluoroacetic acid (TFA), triisopropylsilane (TIPS) and water (95:2.5:2.5). Preparative HPLC afforded 0.11 g of the desired compound **28.** ESI-MS [M+H]⁺= 1226.8.

### Synthesis of PSMA-AAAAAA-NH-T4CO (Compound 29, Figure 4)

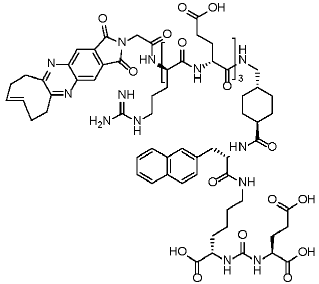

To a solution of **28** (0.11 g, 0.056 mmol) and **5** (0.024 g, 0.056 mmol) in dry DMF (6 mL) under argon, was added 4-methylmorpholine (0.074 mL, 0.67 mmol). The reaction was stirred at room temperature for 6 h. The mixture was then diluted with 10 mL of mobile phase A (1%TFA in H₂O) and purified by preparative HPLC. The collected fractions were freeze-dried to give 0.040 g (33%) of the desired compound as a white solid. ESI-MS [M+H]⁺= 1831.7.

### Example 10: Synthesis of PSMA-NH-AAAAAA-T4CO-FTz (Compound 30, Figure 4)

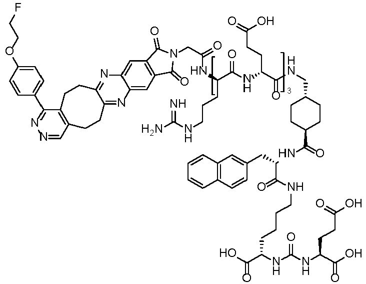

To a solution of **29** (0.005 g, 0.0023 mmol) in ACN (2 mL) and 1% TFA in H₂O (2 mL) was added **1** (0.0005 g, 0.0023 mmol). The reaction was stirred at room temperature for 24 h. The mixture was directly purified by preparative HPLC. The collected fractions were freeze-dried to give 0.004 g (73%) of the desired compound as a white solid. ESI-MS [M+H]⁺= 1010.9.

### Example 11: Synthesis of PSMA-NH-AAAAAA-T4CO-ITz (Compound 31, Figure 4)

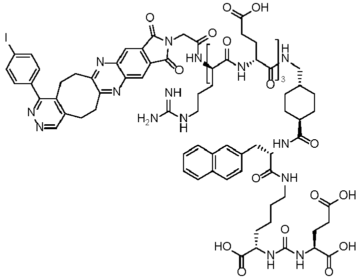

To a solution of **29** (0.01 g, 0.0046 mmol) in ACN (2 mL) and 1% TFA in H₂O (2 mL) was added **3** (0.001 g, 0.0046 mmol). The reaction was stirred at room temperature for 24 h. The mixture was directly purified by preparative HPLC. The collected fractions were freeze-dried to give 0.006 g (54%) of the desired compound as a white solid. ESI-MS [M+2H]⁺= 1042.9.

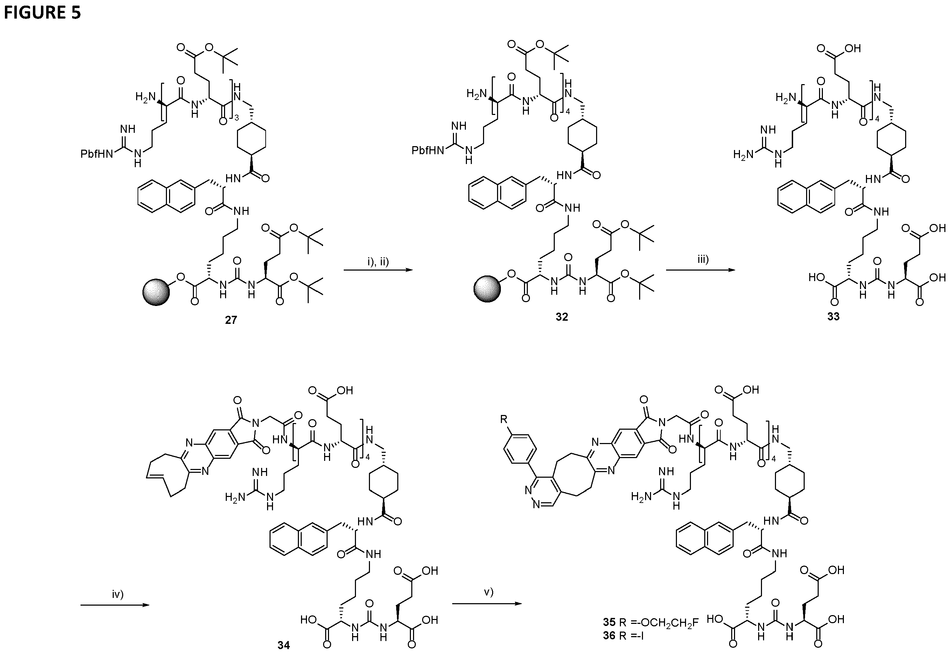

### Example 12: Synthesis of PSMA-AAAAAAAA-NH-T4CO (Compound 34, Figure 5)

Figure 5 is a scheme showing the synthesis of compounds 35 and 36. The grey dot in the intermediate structures symbolizes a resin. In compound 35 R = OCH₂CH₂F. In compound 36 R = I. The following reagents and conditions were applied in steps i) iv): i)a) Fmoc-D-Glu-OH, HATU, DIPEA, DMF, rt, 2 h, b) 50% piperidine in DMF, rt, 20 min.; ii)a) Fmoc-D-Arg(Pbf)-OH, HATU, DIPEA, DMF, rt, 2 h, b) 50% piperidine in DMF, rt, 20 min.; iii) TFA, TIPS, H₂O, rt, 2 h; iv) **5,** 4-methylmorpholine, DMF, rt, 6 h; v) **1** or **3,** ACN, H₂O, TFA, rt, 5 h.

### Synthesis of PSMA-AAAAAAAA-NH₂ (Compound 33, Figure 5)

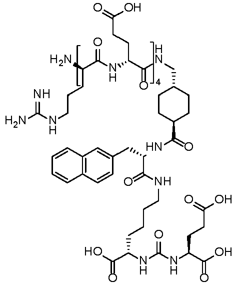

Relative to the resin and **27** (0.21 mmol), 4 equiv. of Fmoc-D-Glu-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After2 min, the solution was added to the resin-immobilised Glutamate-urea-Lysine and shaken for 1 hour. The Fmoc-protecting group was cleaved with a mixture of DMF and piperidine (1:1). The product reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave **32**. The product was cleaved from the resin and deprotected with trifluoroacetic acid (TFA), triisopropylsilane (TIPS) and water (95:2.5:2.5). Preparative HPLC afforded 0.12 g of the desired compound **33.** ESI-MS [M+H]⁺= 1796.9.

### Synthesis of PSMA-AAAAAAAA-NH-T4CO (Compound 34, Figure 5)

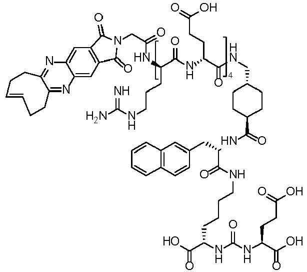

To a solution of **33** (0.11 g, 0.046 mmol) and **5** (0.021 g, 0.046 mmol) in dry DMF (6 mL) under argon, was added 4-methylmorpholine (0.076 mL, 0.69 mmol). The reaction was stirred at room temperature for 6 h. The mixture was then diluted with 10 mL of mobile phase A (1%TFA in H₂O) and purified by preparative HPLC. The collected fractions were freeze-dried to give 0.025 g (21%) of the desired compound as a white solid. ESI-MS [M+2H]⁺ = 1058.9.

### Example 13: Synthesis of PSMA-NH-AAAAAAAA-T4CO-FTz (Compound 35, Figure 5)

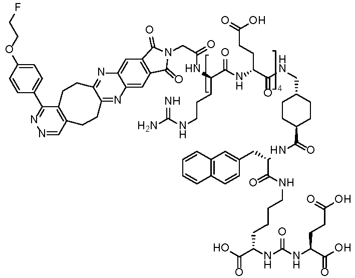

To a solution of **34** (0.005 g, 0.002 mmol) in ACN (2 mL) and 1% TFA in H₂O (2 mL) was added **1** (0.0005 g, 0.002 mmol). The reaction was stirred at room temperature for 24 h. The mixture was directly purified by preparative HPLC. The collected fractions were freeze-dried to give 0.004 g (73%) of the desired compound as a white solid. ESI-MS [M+2H]⁺= 1153.9.

### Example 14: Synthesis of PSMA-NH-AAAAAAAA-T4CO-ITz (Compound 36, Figure 5)

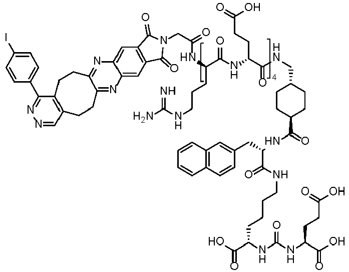

To a solution of **34** (0.01 g, 0.0046 mmol) in ACN (2 mL) and 1% TFA in H₂O (2 mL) was added 3 (0.001 g, 0.0046 mmol). The reaction was stirred at room temperature for 24 h. The mixture was directly purified by preparative HPLC. The collected fractions were freeze-dried to give 0.006 g (54%) of the desired compound as a white solid. ESI-MS [M+2H]⁺= 1186.2.

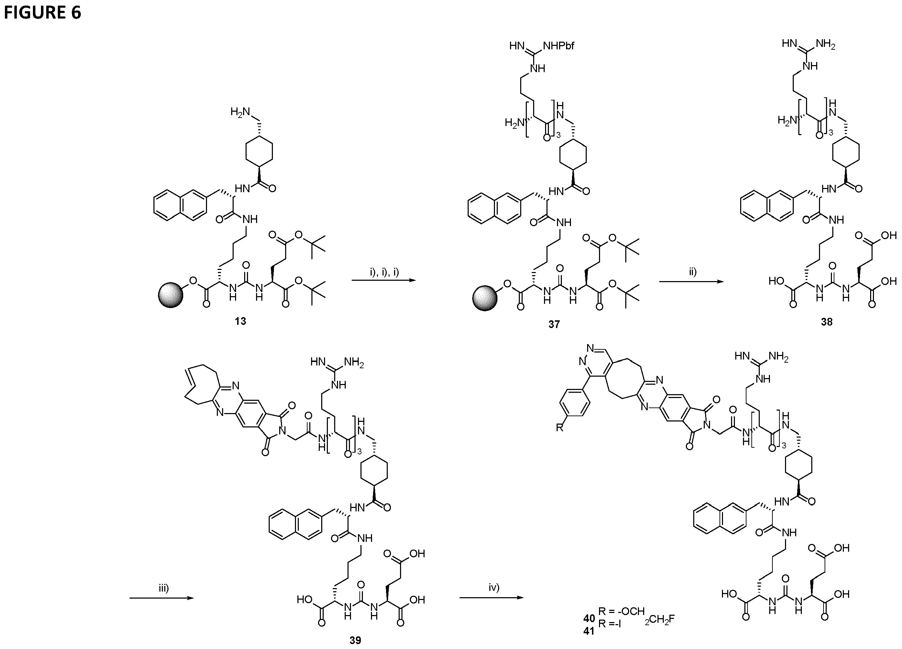

### Example 15: Synthesis of PSMA-3Arg-NH-T4CO (Compound 39, Figure 6)

Figure 6 is a scheme showing the synthesis of compounds 40 and 41. The grey dot in the intermediate structures symbolizes a resin. In compound 40 R = OCH₂CH₂F. In compound 41 R = I. The following reagents and conditions were applied in steps i) - iv): i)a) Fmoc-D-Arg(Pbf)-OH, HATU, DIPEA, DMF, rt, 2 h, b) 50% piperidine in DMF, rt, 20 min.; ii) TFA, TIPS, H₂O, rt, 2 h; iii) **5,** 4-methylmorpholine, DMF, rt, 6 h; iv) **1** or **3,** ACN, H₂O, TFA, rt, 5 h.

### Synthesis of PSMA-3Arg-NH₂ (Compound 38, Figure 6)

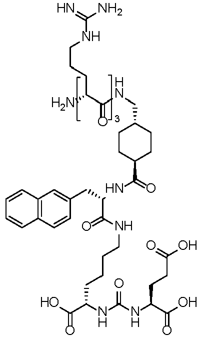

Relative to the resin and **13** (0.22 mmol), 4 equiv. of Fmoc-D-Arg(Pbf)-OH were activated with 3.92 equiv. of HATU and 4 equiv. of DIPEA. Deprotection of the Fmoc group with a mixture of DMF and piperidine (1:1) gave the deprotected intermediate. This process was repeated three times to give **37.** The product was cleaved from the resin and deprotected with trifluoroacetic acid (TFA), triisopropylsilane (TIPS) and water (95:2.5:2.5). Preparative HPLC afforded 0.12 g of the desired compound **38.** ESI-MS [M+H]⁺= 1125.0.

### Synthesis of PSMA-3Arg-NH-T4CO (Compound 39, Figure 6)

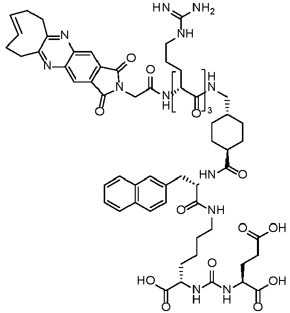

To a solution of **38** (0.07 g, 0.044 mmol) and 5 (0.019 g, 0.044 mmol) in dry DMF (5 mL) under argon, was added 4-methylmorpholine (0.044 mL, 0.40 mmol). The reaction was stirred at room temperature for 6 h. The mixture was then diluted with 10 mL of mobile phase A (1%TFA in H₂O) and purified by preparative HPLC. The collected fractions were freeze-dried to give 0.042 g (53%) of the desired compound as a white solid. ESI-MS [M+H]⁺ = 1444.6.

### Example 16: Synthesis of PSMA-3Arg-NH-T4CO-FTz (Compound 40, Figure 6)

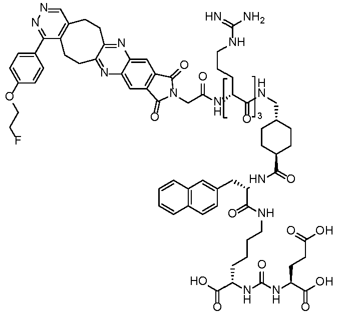

To a solution of **39** (0.02 g, 0.0011 mmol) in ACN (2 mL) and 1% TFA in H₂O (2 mL) was added **1** (0.0005 g, 0.0023 mmol). The reaction was stirred at room temperature for 24 h. The mixture was directly purified by preparative HPLC. The collected fractions were freeze-dried to give 0.0015 g (67%) of the desired compound as a white solid. ESI-MS [M+2H]⁺= 817.4.

### Example 17: Synthesis of PSMA-3Arg-NH-T4CO-ITz (Compound 41, Figure 6)

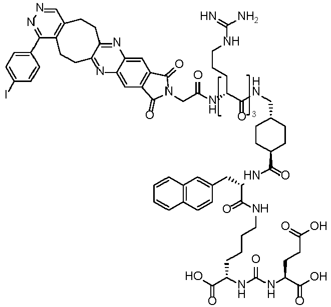

To a solution of **39** (0.015 g, 0.0084 mmol) in ACN (2 mL) and 1% TFA in H₂O (2 mL) was added **3** (0.0024 g, 0.0084 mmol). The reaction was stirred at room temperature for 24 h. The mixture was directly purified by preparative HPLC. The collected fractions were freeze-dried to give 0.006 g (42%) of the desired compound as a white solid. ESI-MS [M+2H]⁺= 849.4.

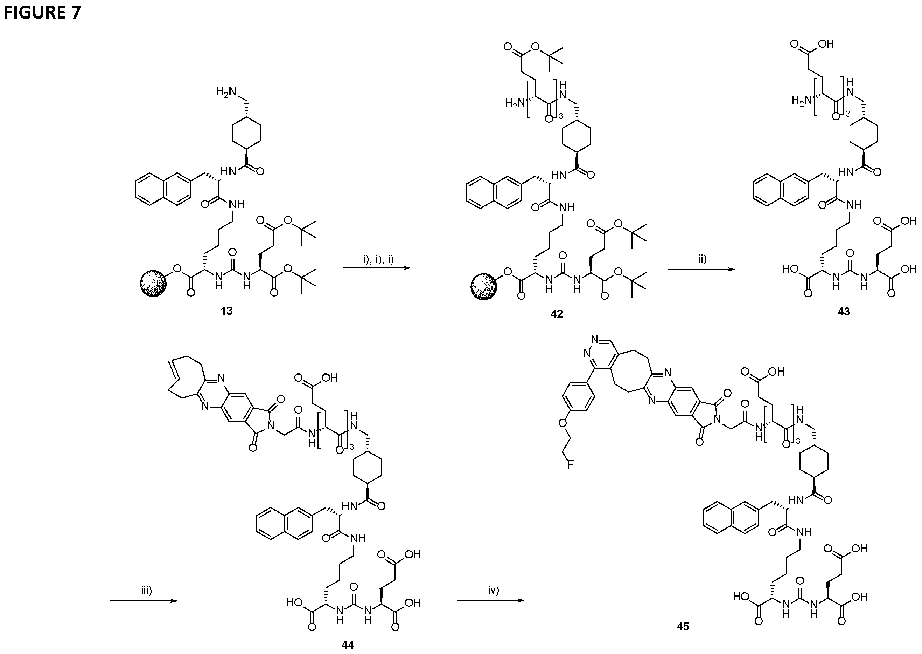

### Example 18: Synthesis of PSMA-3AGlu-NH-T4CO (Compound 44, Figure 7)

Figure 7 is a scheme showing the synthesis of compounds 45. The grey dot in the intermediate structures symbolizes a resin. The following reagents and conditions in steps i) - iv): i)a) Fmoc-D-Glu-OH, HATU, DIPEA, DMF, rt, 2 h, b) 50% piperidine in DMF, rt, 20 min.; ii) TFA, TIPS, H₂O, rt, 2 h; iii) **5,** 4-methylmorpholine, DMF, rt, 6 h; iv) 1, ACN, H₂O, TFA, rt, 5 h.

### Synthesis of PSMA-3AGlu-NH₂ (Compound 43, Figure 7)

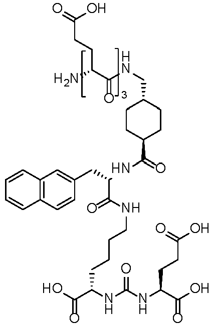

Relative to the resin and **13** (0.32 mmol), 4 equiv. of Fmoc-D-Glu-OH were activated with 3.92 equiv. of HATU and 4 equiv. of DIPEA. Deprotection of the Fmoc group with a mixture of DMF and piperidine (1:1) gave the deprotected intermediate. This process was repeated three times to give **42.** The product was cleaved from the resin and deprotected with trifluoroacetic acid (TFA), triisopropylsilane (TIPS) and water (95:2.5:2.5). Preparative HPLC afforded 0.11 g of the desired compound **43.** ESI-MS [M+H]⁺= 1043.4.

### Synthesis of PSMA-3Glu-NH-T4CO (Compound 44, Figure 7)

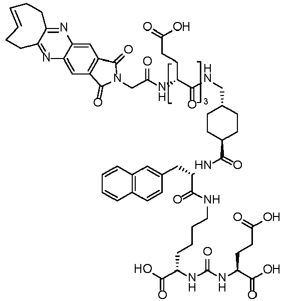

To a solution of **43** (0.054 g, 0.046 mmol) and **5** (0.020 g, 0.046 mmol) in dry DMF (5 mL) under argon, was added 4-methylmorpholine (0.051 mL, 0.46 mmol). The reaction was stirred at room temperature for 6 h. The mixture was then diluted with 10 mL of mobile phase A (1%TFA in H₂O) and purified by preparative HPLC. The collected fractions were freeze-dried to give 0.024 g (38%) of the desired compound as a white solid. ESI-MS [M-H]⁻ = 1361.4.

### Example 19: Synthesis of PSMA-3Glu-NH-T4CO-FTz (Compound 45, Figure 7)

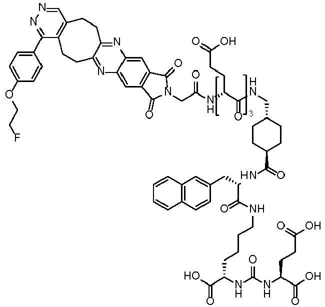

To a solution of **44** (0.005 g, 0.0037 mmol) in ACN (2 mL) and 1% TFA in H₂O (2 mL) was added **1** (0.0008 g, 0.0037 mmol). The reaction was stirred at room temperature for 24 h. The mixture was directly purified by preparative HPLC. The collected fractions were freeze-dried to give 0.004 g (70%) of the desired compound as a white solid. ESI-MS [M+2H]⁺= 817.4.

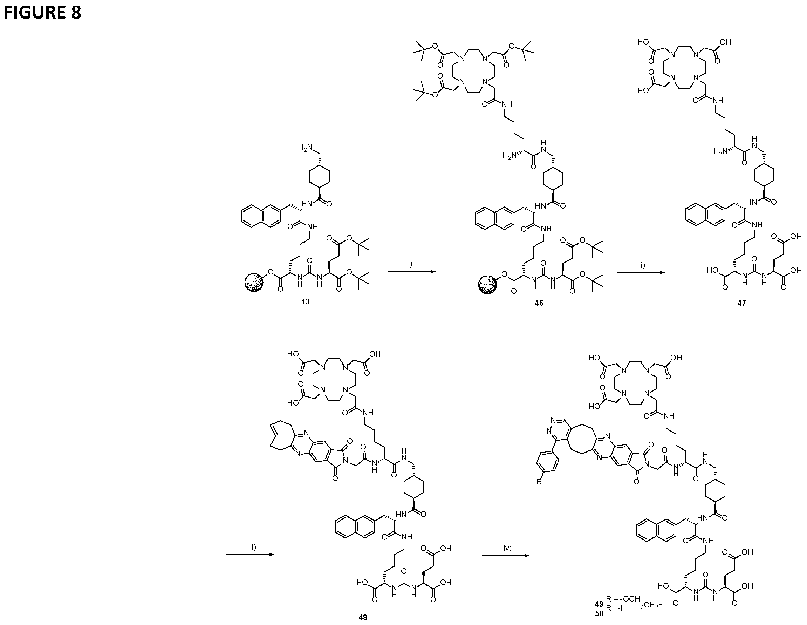

### Example 20: Synthesis of PSMA-Lys-DOTA-NH-T4CO (Compound 48, Figure 8)

Figure 8 is a scheme showing the synthesis of compounds 49 and 50. The grey dot in the intermediate structures symbolizes a resin. In compound 49 R = OCH₂CH₂F. In compound 50 R = I. The following reagents and conditions in steps i) - iv): i)a) Fmoc-tBuDOTA-L-Lys-OH, HATU, DIPEA, DMF, rt, 2 h, b) 50% piperidine in DMF, rt, 20 min.; ii) TFA, TIPS, H₂O, rt, 2 h; iii) **5,** 4-methylmorpholine, DMF, rt, 6 h; iv) **1** or **3,** ACN, H₂O, TFA, rt, 5 h.

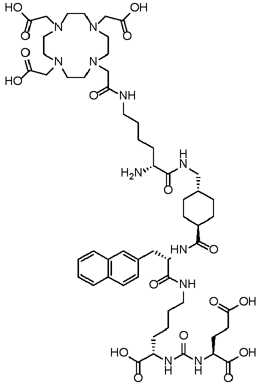

Relative to the resin and **13** (0.32 mmol), 1.4 equiv. of Fmoc-tBuDOTA-L-Lys-OH were activated with 1.2 equiv. of HATU and 4.2 equiv. of DIPEA. Deprotection of the Fmoc group with a mixture of DMF and piperidine (1:1) gave the deprotected intermediate **46.** Half of the product was cleaved from the resin and deprotected with trifluoroacetic acid (TFA), triisopropylsilane (TIPS) and water (95:2.5:2.5). Preparative HPLC afforded 0.04 g of the desired compound **47.** ESI-MS [M+H]⁺= 1170.5.

### Synthesis of PSMA-Lys-DOTA-NH-T4CO (Compound 48, Figure 8)

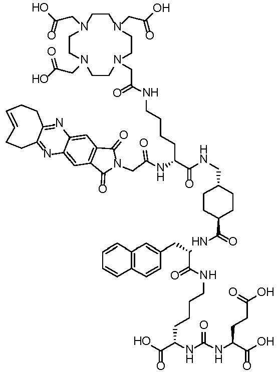

To a solution of **47** (0.04 g, 0.034 mmol) and **5** (0.015 g, 0.034 mmol) in dry DMF (5 mL) under argon, was added 4-methylmorpholine (0.041 mL, 0.37 mmol). The reaction was stirred at room temperature for 6 h. The mixture was then diluted with 10 mL of mobile phase A (1%TFA in H₂O) and purified by preparative HPLC. The collected fractions were freeze-dried to give 0.025 g (49%) of the desired compound as a white solid. ESI-MS [M-H]⁻= 1490.7.

### Example 21: Synthesis of PSMA-Lys-DOTA-NH-T4CO-FTz (Compound 49, Figure 8)

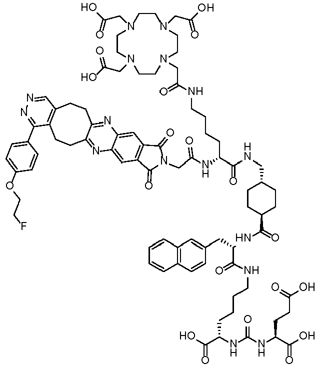

To a solution of **48** (0.003 g, 0.002 mmol) in ACN (2 mL) and 1% TFA in H₂O (2 mL) was added **1** (0.0005 g, 0.002 mmol). The reaction was stirred at room temperature for 24 h. The mixture was directly purified by preparative HPLC. The collected fractions were freeze-dried to give 0.002 g (59%) of the desired compound as a white solid. ESI-MS [M+H]⁺= 1680.7.

### Example 22: Synthesis of PSMA-Lys-DOTA-NH-T4CO-ITz (Compound 50, Figure 8)

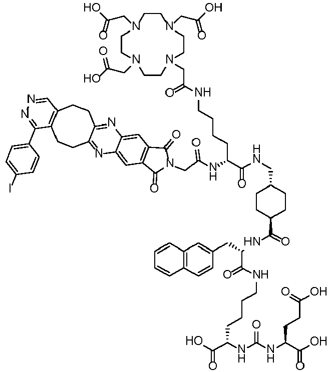

To a solution of **48** (0.005 g, 0.003 mmol) in ACN (2 mL) and 1% TFA in H₂O (2 mL) was added **3** (0.0009 g, 0.003 mmol). The reaction was stirred at room temperature for 24 h. The mixture was directly purified by preparative HPLC. The collected fractions were freeze-dried to give 0.002 g (59%) of the desired compound as a white solid. ESI-MS [M+H]⁺= 1774.5

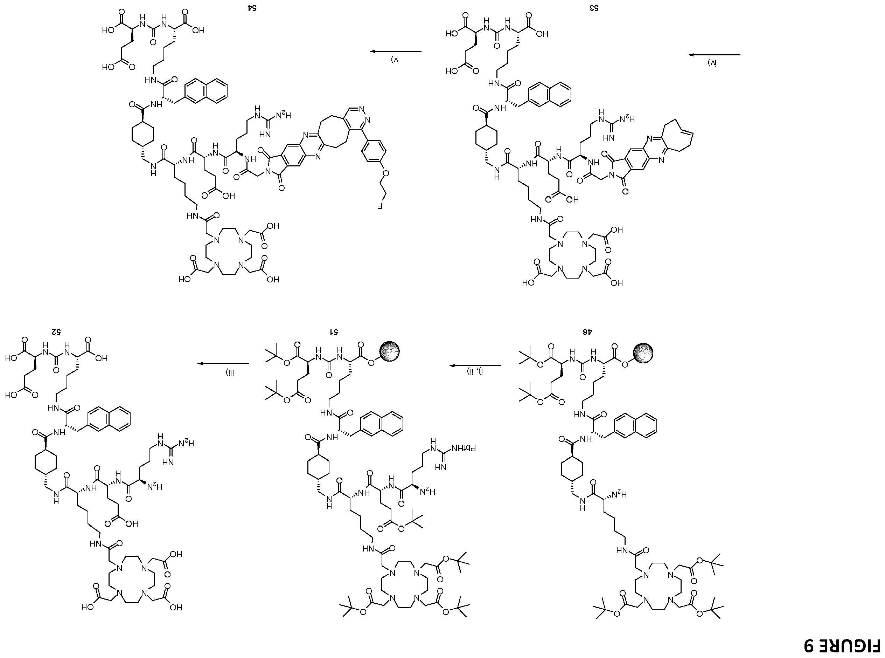

### Example 23: Synthesis of PSMA-Lys-DOTA-AA-NH-T4CO (Compound 52, Figure 9)

Figure 9 is a scheme showing the synthesis of compound 54. The grey dot in the intermediate structures symbolizes a resin. The following reagents and conditions in steps i) - v): i)a) Fmoc-D-Glu-OH, HATU, DIPEA, DMF, rt, 2 h, b) 50% piperidine in DMF, rt, 20 min.; ii)a) Fmoc-D-Arg(Pbf)-OH, HATU, DIPEA, DMF, rt, 2 h, b) 50% piperidine in DMF, rt, 20 min.; iii) TFA, TIPS, H₂O, rt, 2 h; iv) **5,** 4-methylmorpholine, DMF, rt, 6 h; v) 1, ACN, H₂O, TFA, rt, 5 h.

### Synthesis of PSMA-Lys-DOTA-AA-NH₂ (Compound 52, Figure 9)

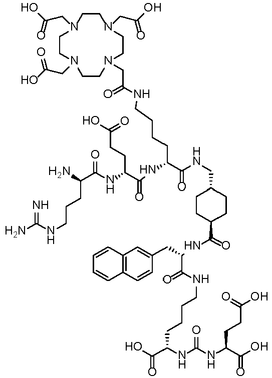

Relative to the resin and **46** (0.16 mmol), 4 equiv. of Fmoc-D-Glu-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized Glutamate-urea-Lysine and shaken for 1 hour. The Fmoc-protecting group was cleaved with a mixture of DMF and piperidine (1:1). The product reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave **51.** The product was cleaved from the resin and deprotected with trifluoroacetic acid (TFA), triisopropylsilane (TIPS) and water (95:2.5:2.5). Preparative HPLC afforded 0.03 g of the desired compound **52.** ESI-MS [M+H]⁺= 1455.6.

### Synthesis of PSMA-Lys-DOTA-AA-NH-T4CO (Compound 53, Figure 9)

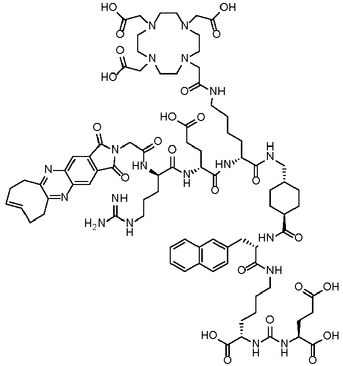

To a solution of **52** (0.035 g, 0.021 mmol) and **5** (0.009 g, 0.021 mmol) in dry DMF (5 mL) under argon, was added 4-methylmorpholine (0.027 mL, 0.25 mmol). The reaction was stirred at room temperature for 6 h. The mixture was then diluted with 10 mL of mobile phase A (1%TFA in H₂O) and purified by preparative HPLC. The collected fractions were freeze-dried to give 0.016 g (43%) of the desired compound as a white solid. ESI-MS [M-H]⁻= 1775.9.

### Example 24: Synthesis of PSMA-Lys-DOTA-AA-NH-T4CO-FTz (Compound 54, Figure 9)

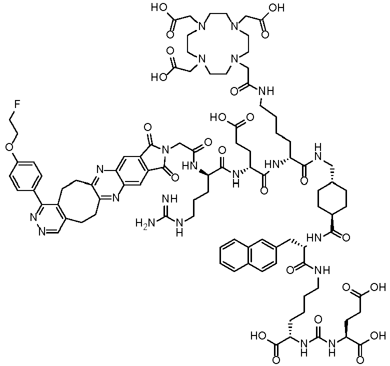

To a solution of **53** (0.005 g, 0.003 mmol) in ACN (2 mL) and 1% TFA in H₂O (2 mL) was added **1** (0.0009 g, 0.003 mmol). The reaction was stirred at room temperature for 24 h. The mixture was directly purified by preparative HPLC. The collected fractions were freeze-dried to give 0.004 g (72%) of the desired compound as a white solid. ESI-MS [M+H]⁺= 1965.8.

### Example 25: Radiolabeling of [¹⁸F]1 before IEDDA conjugation

The radiolabeling was performed according to published procedure.¹³ The aqueous [¹⁸F]fluoride solution received from the cyclotron was passed through a Sep-Pak Light QMA cartridge preconditioned with 5 mL 0.5 M K₃PO₄. [¹⁸F]Fluoride was eluted from the QMA cartridge into a 4 mL v-shaped vial using Bu₄NOTf solution (20 mM in MeOH, 1 mL). The eluate was dried at 100°C for 5 min under nitrogen or helium flow. After MeOH had evaporated, acetonitrile (0.5 mL) was added to the same vial and evaporated under the same conditions to remove traces of water. Two additions of acetonitrile were performed. Nosyl precursor **2** (1.5 mg) was dissolved in anhydrous acetonitrile (0.3 mL), diluted with tBuOH (0.7 mL) and added to the dried [¹⁸F]fluoride residue. After reacting for 5 min at 100 °C, the reaction was cooled to 80 °C with ambient air flow, diluted with water (2 mL) and purified by semipreparative HPLC: Discovery HS F5 5 µm, 250 mm × 10 mm column, isocratic elution with 50% acetonitrile in 20 mM citrate buffer pH 6.1, elution speed 5 mL/min. The product peak (retention time 9.0 min) was collected, diluted with water (50-100 mL) and passed through a Sep-pak Plus C18 Short solid phase extraction cartridge (Waters, USA) that was preconditioned by flushing it with EtOH/water mixture (1/1 v/v, 10 mL). The Sep-pak cartridge was then flushed with extra water (5 mL), blown with nitrogen, eluted with 1-2 mL of organic solvent (ACN or EtOH) and diluted with water to achieve the necessary concentration of the organic solvent for the next step. [¹⁸F]**1** was obtained in a radiochemical yield of 16±10% (n=4) and >95% radiochemical purity.

### Example 26: Radiolabeling of [²¹¹At]4 before IEDDA conjugation

5 µL of a solution of precursor **4** (5mg/ml in MeOH) was added to concentrated (from CHCl₃) [²¹¹At], to which was added 15 uL MeOH, 1 µL AcOH and 1 µL NCS solution (20mg/ml in MeOH). The reaction was left for 10 min. The reaction mixture was diluted with H₂O, containing 0.1% TFA and the compound was purified with HPLC. The isolated fraction was used directly in the next step. [²¹¹At]**4** was obtained in a radiochemical yield of 67±5% (n=5) and >95% radiochemical purity.

### Isolated RCY and activities for [²¹¹At]4

| Reaction | MBq isolated (after HPLC) | RCY % | RCP % |
|---|---|---|---|
| 1 | 56.8 | 72 | >95 |
| 2 | 31.3 | 60 | >95 |
| 3 | 36.3 | 62 | >95 |
| 4 | 101 | 68 | >95 |
| 5 | 75.3 | 78 | >95 |

### Example 27: IEDDA conjugation of TCO functionalized PSMA derivatives with [¹⁸F]1

### Click:

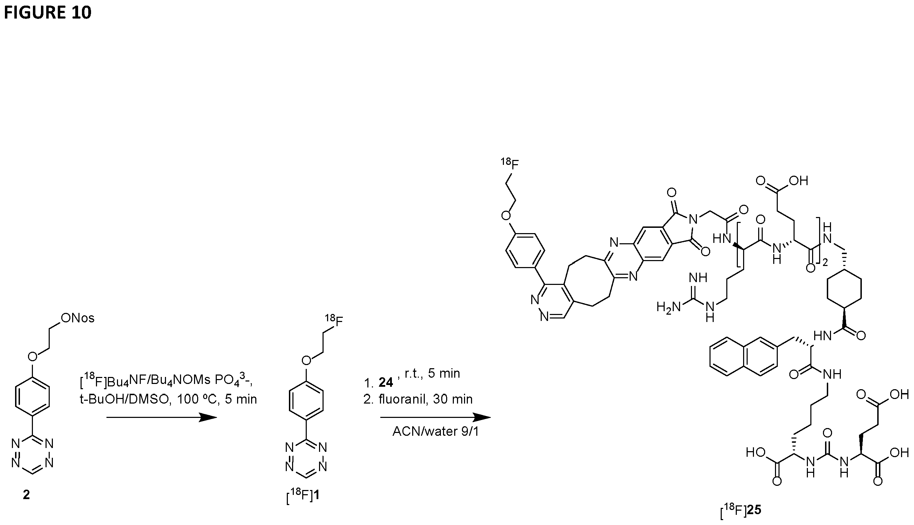

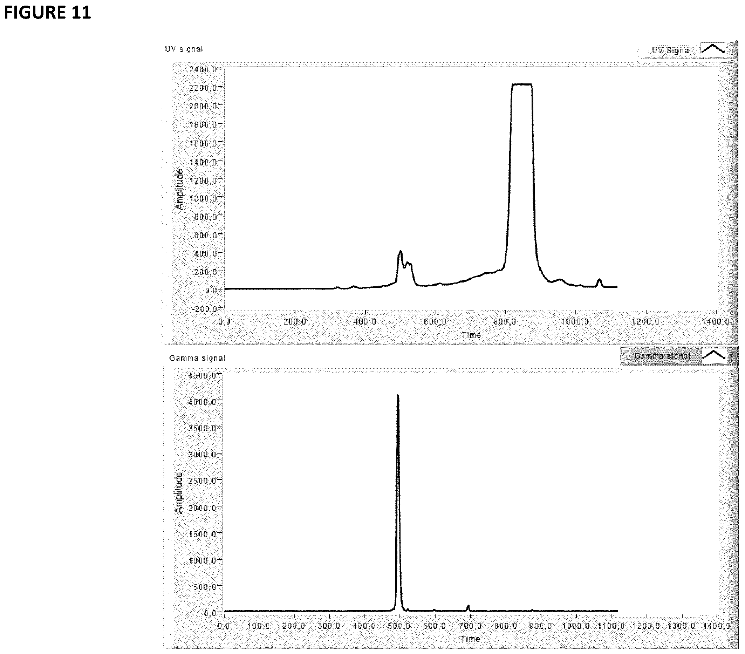

To the solution of 3-(4-(2-[¹⁸F]fluoroethoxy)phenyl)-1,2,4,5-tetrazine ([¹⁸F]**1**) in acetonitrile (1 mL) was added 20-100 µL of PSMA modified derivatives (**15, 20, 24**, **29, 34, 39, 43, 47, 51**) solution in DMSO (2-5 mg/mL, total added mass 100-200 µg). Full consumption of [¹⁸F]**1** was confirmed by radio-HPLC after 5 min. Figure 10 shows the full synthesis starting from the nosyl precursor **2**. Figure 11 shows semipreparative HPLC traces for [¹⁸F]**25**.

### Oxidation:

After the click step, 50 µL fluoranil solution (20 mg/mL in acetonitrile) was added to the click mixture. The mixture was left standing at room temperature for 30 min following the procedure previously reported (PCT/EP2023/055930).

### Optional Purification

Crude oxidized [¹⁸F]-labeled PSMA derivative was diluted with water (3 mL) and purified by semipreparative HPLC (method described below). The product peak (Table 1) was collected, diluted with water (50-100 mL) and passed through a Sep-pak Plus C18 Short solid phase extraction cartridge (Waters, USA) that was preconditioned by flushing it with EtOH/water mixture (1/1 v/v, 10 mL). The Sep-pak cartridge was then flushed with extra water (5 mL), blown with nitrogen and eluted with 1 mL of ethanol. The conditions employed were: HPLC method: Luna C18(2) 5 µm column, 250x10 mm, eluted with a gradient of acetonitrile in water with 0.1% TFA (v/v) in both. Gradient: 0 min - 30% acetonitrile, 11.7 min - 55% acetonitrile, 16.7 min - 55% acetonitrile, 30 min - 90% acetonitrile. Elution flow 4 mL/min

| [¹⁸F]PSMA Derivatives | Precursor | Retention time, min | RCY from [¹⁸F]**1**, % | RCP, % |
|---|---|---|---|---|
| [¹⁸F]**16** | **15** | 12.1 | 55 | >95 |
| [¹⁸F]**21** | **20** | 9.8 | 16 | >95 |
| [¹⁸F]**25** | **24** | 8.2 | 48 | >95 |
| [¹⁸F]**30** | **29** | 7.6 | 48 | >95 |
| [¹⁸F]**35** | **34** | 7.2 | 46 | >95 |
| [¹⁸F]**40** | **39** | 8.3 | 36 | >95 |
| [¹⁸F]**44** | **43** | 9.8 | 23 | >95 |
| [¹⁸F]**48** | **47** | 8.8 | 43 | >95 |
| [¹⁸F]**53** | **51** | 7.8 | 29 | >95 |

### Formulation for experiments in rats:

Ethanolic solution of purified [¹⁸F]-labeled PSMA derivative was evaporated to dryness under nitrogen flow at 40°C. The residue was resolubilized in 35 µL ethanol and diluted with 350 µL phosphoric acid (0.085% in water v/v) and 350 µL citrate buffer (20 mM, pH 6.1). The resulting solution (pH 4.5) was used for injections.

### Example 28: IEDDA conjugation of TCO functionalized PSMA derivatives with [²¹¹At]4

### Tetrazine labeling:

### Click

To the MeCN/H₂O solution of [²¹¹At]**4** (250 uL) was added 45 uL 29 in MeCN/H₂O (0.4 mg/450 uL, 33% MeCN in H₂O).

### Oxidation

After the click step, 50 µL fluoranil solution (20 mg/mL in acetonitrile) was added to the click mixture. The mixture was left standing at room temperature for 30 min following the procedure previously reported (PCT/EP2023/055930).

### Optional Purification

The compound was purified via HPLC. The product was trapped on C18 cartridge and eluted with 1 mL EtOH.

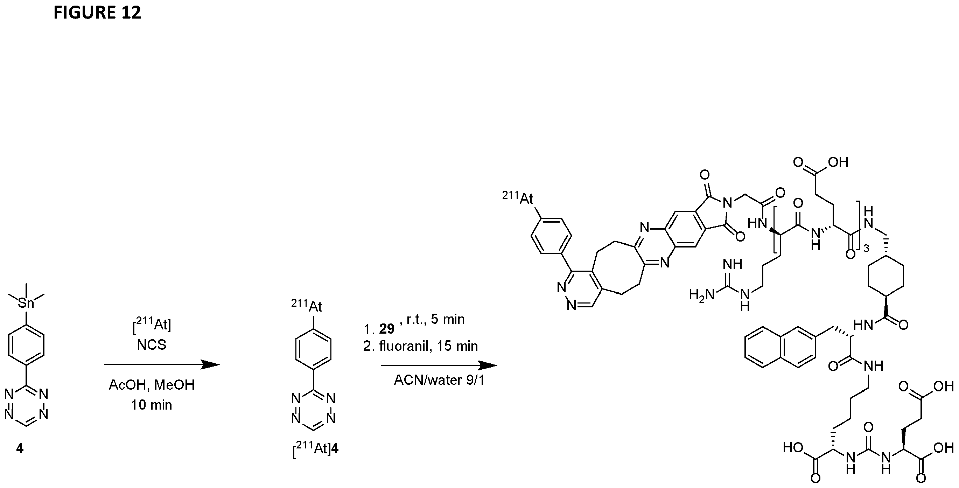

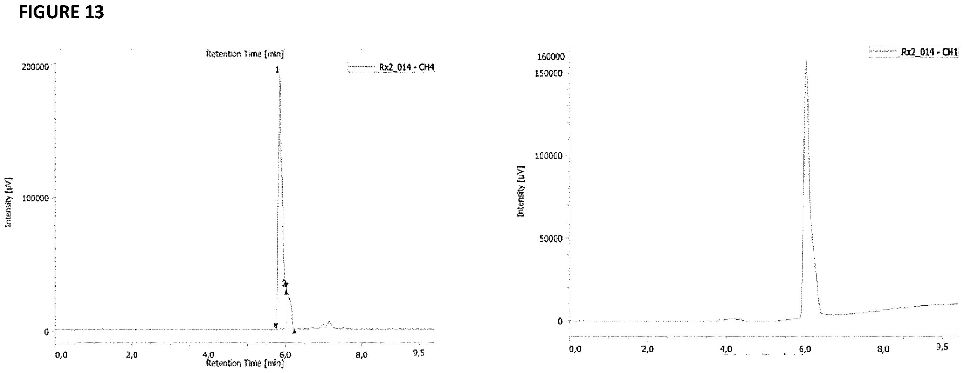

Figure 12 shows the full synthesis starting from precursor **4.** Figure 13 shows semipreparative HPLC traces for [²¹¹At]**31**.

### Isolated RCY and activities for [²¹¹At]31

| Reaction | MBq isolated (after HPLC) | RCY from [²¹¹At]**4**, % | MBq isolated (after C18) | RCY% from [²¹¹At]**4**, % | RCP, % |
|---|---|---|---|---|---|
| 1 | 17.6 | 35 | 3.81 | 28 | >95 |
| 2 | 51 | 67 | 1.2 | 2 | >95 |
| 3 | 44 | 76 | 14.6 | 25 | >95 |

### Formulation for experiments in rats:

Ethanolic solution of purified [²¹¹At]**31** was dried under a stream of N₂ and diluted to the correct concentration EtOH containing 5% ascorbic acid (v/w%. Before administration, the ethanolic solution was diluted with PBS, to 5% EtOH, containing 2 kBq/µL of [²¹¹At]**31.**

### Example 29: General procedure for evaluation of stability of [²¹¹At] labeled derivative

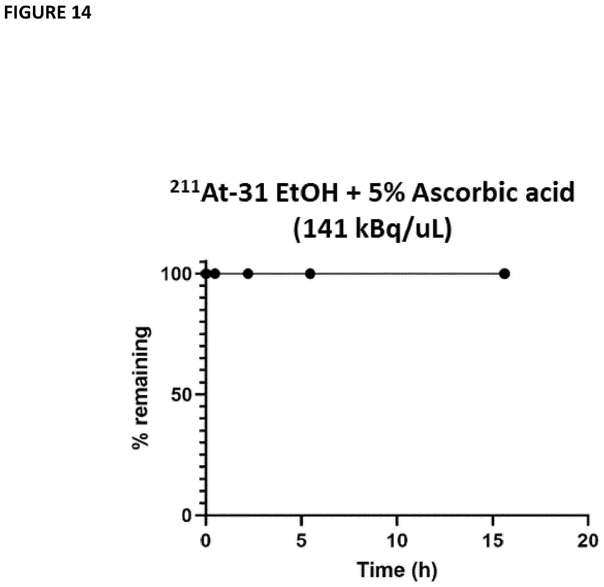

The stability of the compound was measured in neat EtOH, containing 5% ascorbic acid (v/w%) and followed over the course of 900 minutes. The results for [²¹¹At]**31** are reported in Figure 14.

### Example 30: General procedure for autoradiography for affinity measurement

The affinity of PSMA derivatives towards PSMA was measured in an in vitro autoradiography competition assay on rat brain slices against [¹¹¹In]In-PSMA-617 as a radioligand/
[¹¹¹In]In-PSMA-617 was prepared by mixing 2 µL of 2 mg/mL vipivotide tetraxetan (MedChemExpress, USA) solution in milliQ water with ¹¹¹InCl₃ (5-20 MBq) buffered to pH 5.5 with ammonium acetate (-0.2M final ammonium acetate concentration, 40-70 µL final volume) and heating the resulting mixture at 60°C for 5 min. This method provided [¹¹¹In]-PSMA-617 in >95% radiochemical yield, as determined by reverse-phase HPLC.

Rat brain slices were obtained from female Sprague-Dawley rats (weight~250g; Taconic Biosciences). The rats were sacrificed by decapitation, brains were extracted, frozen at -80 °C and stored at the same temperature. To prepare slices for autoradiography, brains were cut into halves along the sagittal symmetry plane. Each half was mounted, lateral side up, on the cryostat sample holder pretreated by Tissue-Tek OCT compound and fixed by freezing in the cryostat. After fixing, brains were cut at -22 °C into sagittal slices 20 µm thick using a Leica microtome, and the slices were thaw-mounted on Superfrost (70 × 22 mm, Fischer) adhesive slides, 3-4 slices per slide. Only slices containing midbrain were used because midbrain structures express relatively high levels of PSMA.¹⁵ The slices were allowed to dry and were then put into storage boxes and kept at -80 °C until they were used.

Saturation assay to measure the affinity of [¹¹¹In]In-PSMA-617 towards PSMA in brain slices and competition assay to measure the affinity of PSMA derivatives towards the same target were designed according to the guidelines of Hein et al.¹⁶ Briefly, for saturation assay, the slides were incubated for 1.5 h with increasing concentrations (0.1-30 nM) of [¹¹¹In]In-PSMA-617 in the assay buffer (170 mM Tris-HCl pH 7.2, 5 mM MgCl₂, 1% BSA). For competition assay, the slides were incubated with 2 nM [¹¹¹In]In-PSMA-617 and increasing concentrations (0.1-30 nM) of investigated PSMA derivatives for the same amount of time in the same assay buffer. Non-specific binding was determined by adding 5 µM 2-(phosphonomethyl)pentanedioic acid (PMPA) to [¹¹¹In]In-PSMA-617 solutions.

Incubations were performed by creating a hydrophobic barrier along the edges of the slides with a PAP pen, adding 1 mL of the corresponding incubation solution per slide and then gently shaking the slides at room temperature in a closed plastic box. However, for low (<0.3 nM) concentrations of [¹¹¹In]In-PSMA-617 in the saturation assay, the slides were incubated in a bath filled with [¹¹¹In]In-PSMA-617 solution in the assay buffer instead (>20 mL / slide). Immediately before the incubation, 1 mL assay buffer was applied to each slide, the slides were shaken at room temperature for 15 min as described above, and then assay buffer was replaced with the corresponding incubation solution.

After the incubation, the slides were rinsed by dipping them into ice-cold assay buffer for 5 min and then into ice-cold distilled water for 0.5 min. Afterwards, the slices were dried in air stream and exposed against phosphor screens (Packard Instruments Co) for 24-72 h along with a calibration curve prepared by spotting 1 µL aliquots of [¹¹¹In]In-PSMA-617 solutions with known concentrations (0.1-30 nM) on a silica TLC strip.

The screens were then read by a Cyclone Plus storage phosphor system (Packard Instruments Co). Quantification of readings was done with Optiquant software (version 3.00, Packard Instruments Co). Namely, elliptic regions of interest (ROIs) encompassing the midbrain and cerebellar nuclei were drawn inside the brain slice images, while circular ROIs were drawn around calibration curve spots, and average exposure was calculated for both ROI categories. Exposure values were then recalculated into fmol [¹¹¹In]In-PSMA-617 per µL tissue (equivalent to nM) by fitting a linear function onto the calibration curve values. The fmol/µL values were then used for non-linear regression in GraphPad Prism (v9.4.1) to calculate Bₘₐₓ and K_{D} of [¹¹¹In]In-PSMA-617 in the saturation assay and IC50 of PSMA derivatives in the competitions assays. IC50 values were automatically recalculated into Kᵢ values by GraphPad Prism using the Cheng-Prusoff equation. The values for all the tested compounds, except for [¹⁸F]**40**, were in line with results published for PSMA-617 suggesting that the affinity for the target is retained.

| [¹⁸F]PSMA Derivatives | Precursor | Affinity (Kᵢ, nM) |
|---|---|---|
| [¹⁸F]**16** | **15** | ND |
| [¹⁸F]**21** | **20** | ND |
| [¹⁸F]**25** | **24** | 3.12 ± 1.66 (2) |
| [¹⁸F]**30** | **29** | 3.10 ± 0.28 (2) |
| [¹⁸F]**35** | **34** | 7.04 ± 4.44 (2) |
| [¹⁸F]**40** | **39** | 47.45 |
| [¹⁸F]**44** | **43** | 1.33 ± 0.94 (2) |
| [¹⁸F]**48** | **47** | 3.73 ± 1,21 |
| [¹⁸F]**52** | **51** | 4.93 ± 1.64 |

Data are presented as means ± standard deviations. Number of independent determinations (if > 1) is shown in parentheses.

### Example 31: General procedure for internalization assay

Poly-L-Lysine (300 µL of a 0.01% w/v in H₂O) is added to each well of a 24-well plate (Greiner Bio). The plate is left at room temperature for 20-30 minutes. Afterwards, the poly-L-lysine solution is removed, and the wells washed with 500 µL of PBS. Afterwards, 1 mL of LNCaP cells suspension is placed in each well to obtain 10⁵ cells/well. The 24-well plates are left in the incubator overnight until the start of the internalisation experiment. Subsequently, the previously plated cells were incubated at 37 °C with the selected ¹⁸ F-labelled PSMA derivative in 250 µL growth medium for 45 min (c = 32 nM). For the determination of specific uptake, 2-PMPA was added into half of the wells in an excess concentration of 500 µM/well. After incubation, cellular uptake was interrupted by removing the medium and washing the cells with ice-cold PBS (3 × 1 mL). The surface bound radioactivity was removed by washing with 50 mM glycine (pH 2.8, 2 × 500 µL). Finally, the internalized fraction was determined by lysis of the cells with 0.3 M NaOH (1 × 500 µL). Fractions collected from the surface bound activity and lysates were collected and measured in a gamma counter. All counts were normalized to the starting activity and specific surface binding as well as specific internalization were determined by subtracting the non-specific binding obtained from the wells treated with 2-PMPA. Results were calculated and expressed as %AA/10⁵ cells (% applied activity/105 cells, n = 4). [⁶⁸Ga]-PSMA617 was selected as a reference to compare internalization values. The experiment was conducted two times on different compounds. The data are shown below in separate tables. PSMA-617 was used as a reference and internal control for both experiments. The data shows that the compounds presented here have a better internalization compared to PSMA-617. The higher internalization could have a big impact on the therapeutic effect of such molecules.

| | [¹⁸F]**16** | [¹⁸F]**25** | [¹⁸F]**30** | [¹⁸F]**35** | [¹⁸F]**44** | [¹⁸F]**40** | [⁶⁸Ga]-PSMA-617 |
|---|---|---|---|---|---|---|---|
| Cell surface | 0.36 ± 0.03 | 0.70 ± 0.04 | 0.64 ± 0.10 | 1.50 ± 0.09 | 0.69 ± 0.07 | 5.15 ± 0.87 | 0.36 ± 0.03 |
| Cell surface blocked | 0.09 ± 0.01 | 0.10 ± 0.01 | 0.08 ± 0.01 | 0.11 ± 0.01 | 0.03 ± 0.01 | 4.70 ± 1.05 | 0.03± 0.01 |
| Internalized | 1.98 ±0.17 | 0.90 ± 0.04 | 0.54 ± 0.06 | 1.28 ± 0.07 | 1.17 ± 0.02 | 4.53 ± 0.55 | 0.27± 002 |
| Internalized blocked | 0.78 ± 0.11 | 0.12 ± 0.01 | 0.07 ± 0.01 | 0.09 ± 0.01 | 0.04 ± 0.01 | 4.10 ± 0.65 | 0.03 ± 0.01 |

| | [⁶⁸Ga]**49** | [⁶⁸Ga]**50** | [⁶⁸Ga]**54** | [⁶⁸Ga]-PSMA-617 |
|---|---|---|---|---|
| Cell surface | 0.51 ± 0.02 | 0.40 ± 0.07 | 0.54 ± 0.04 | 0.36 ± 0.03 |
| Cell surface blocked | 0.05 ± 0.01 | 0.08 ± 0.01 | 0.05 ± 0.01 | 0.03± 0.01 |
| Internalized | 0.55 ±0.04 | 0.80 ± 0.05 | 0.26 ± 0.04 | 0.13± 001 |
| Internalized blocked | 0.13 ± 0.01 | 0.27 ± 0.06 | 0.08 ± 0.01 | 0.02 ± 0.01 |

### Example 32: In vivo Positron Emission Tomography (PET) imaging in rats of [¹⁸F]-labeled PSMA derivatives

### Animals

Long-Evans female rats weighing 200 - 300 g (Charles River, Calco, Italy), were housed in a cage of 2-3 rats per cage and kept in a climate-controlled facility with a 12-hr light/dark cycle. The cages had environmental enrichment (nest box, biting stick). All rats were fed *ad libitum* with commercial breeding diet (1310 FORTI-Avlsfoder, Brogaarden, Altromin International) and had free access to water. All the experiments were performed in accordance with the European Commission's Directive 2010/63/EU, FELASA and ARRIVE guidelines for animal research and, with approval from The Danish Council for Animal Ethics (license numbers 2017-15-0201-01283 and 2017-15-0201-01375) together with the Department of Experimental Medicine, University of Copenhagen.

### PET procedure

The scans were performed on the Siemens HRRT (High-Resolution Research Tomography) (CPS Innovations/Siemens, Malvern, PA, USA). On the day of experiment, the rats were weighed and then transported to the scanner at least 2 h before the scan. Anesthesia was induced using 3% isoflurane in oxygen flow. All rats were placed in a 2 × 2 custom made rat holder, enabling simultaneous scanning of four rats 17-19. While in the custom-made rat holder, the rats were kept under anesthesia with a constant flow of isoflurane (-2% isoflurane in oxygen). The rats were cannulated in the dorsal or lateral tail vein with BD Neoflon 24G vein catheter and flushed with a solution of 4 unit/mL heparin in saline. The rats were placed in the scanner and kept warm using an infrared lamp or heating pads and checked for respiration throughout the entire scan. The tracer (5-25 MBq in 0.5-1.0 mL) was injected through the catheter and a 90-min PET acquisition (emission scan) was started at the time of injection. A rotating point source ¹³⁷Cs transmission scan ²⁰ was carried out before or after each emission scan.

### Image reconstruction

PET list-mode emission files were reconstructed with the aid of ordinary Poisson 3D subset expectation maximization (OP-3D-OSEM) algorithm, including modeling the point-spread function, with 16 subsets, ten iterations, and standard corrections. The transmission attenuation map was regenerated using maximum posteriori algorithm. Emission data were binned into time frames of increasing lengths: 6 × 10 s, 8 × 30 s, 5 × 60 s and 16 × 300 s. Each reconstructed time frame image consisted of 207 planes of 256 × 256 voxels of 1.22 × 1.22 × 1.22 mm size.¹⁹

### Data analysis

To analyze the data, the program PMOD (PMOD Technologies, Zürich, Switzerland) was used.

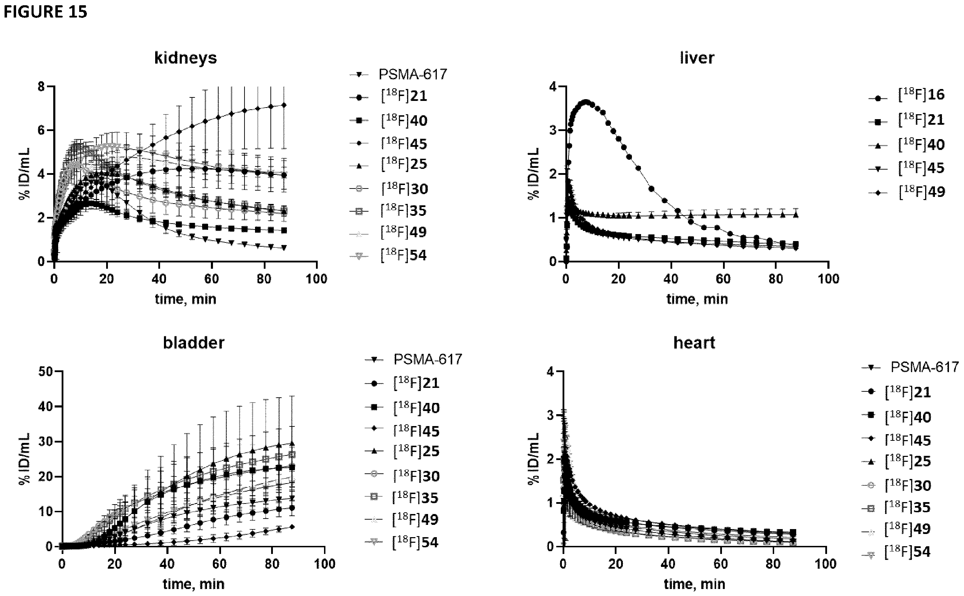

Figure 15 shows the results of these studies. Surprisingly by increasing the number of amino acids in the linker the excretion is shifted from the liver to the kidneys and bladder. This effect is more pronounced for 2 to 6 amino acids. Compound [¹⁸F]**16** (no amino acids) was excreted mainly through the liver while [¹⁸F] **21** (2 amino acids) was not.

### Examples 33: In vivo PET/CT imaging in tumor bearing mice (PC3-PIP) of [¹⁸F]labeled PSMA derivatives

### Animals

For evaluation, NOD-SCID mice 6-7 weeks of age (Janvier, Europe) were inoculated with PC3-PIP (PSMA-positive) cells into the left shoulder and with PC3-flu (PSMA-negative) cells into the right shoulder. Imaging experiments were performed once both tumor xenografts reached the size of at least 100 mm³. Mouse body weight was 22-29 g on the day of the imaging.

### PET/CT procedure

The mice were anesthetized with a mixture of 1.5-2% isoflurane and 100% oxygen before injection of 0.1 ml bolus containing 5-9 MBq of [¹⁸F]-labeled PSMA derivative into the tail vein. To assess the specificity of tracer binding to its respective target (PSMA), for half of the mice the injected tracer was mixed with 100 µg of cold competing ligand (MEF98). PET acquisition (90 min) was started immediately after [¹⁸ F]FlutraPros injection. Additional PET acquisitions were performed 4 h (15 min long) and 8 h (30 min long) post-injection, with mice re-anesthetized before scanning as described above.

### MEF98 PSMA ligand

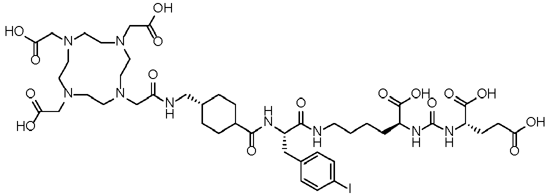

PET/CT imaging was performed on a Siemens INVEON multimodality preclinical scanner in a docked mode (Siemens, Knoxville, Tennessee, USA). The imaging protocol included a two-bed CT for anatomic orientation performed with full rotation, 360-degree projections, and exposure of 80 kV and 500 µA. A static CT scan was immediately followed by the PET scan. CT and PET images were co-registered using a transformation.

### Image reconstruction

Reconstruction of PET data was performed using an OSEM3D/SP-MAP algorithm (2 x OSEM iterations and 18 x MAP iterations) with scatter correction and a matrix size 128x128, resulting in a final target resolution of 1.5 mm. List-mode data from the dynamic scan were binned into time frames of increasing lengths: 3 × 10 s, 3 × 30 s, 3 × 60 s, 5 × 300 s, 3 × 600 s and 2 × 900 s.

### Data analysis

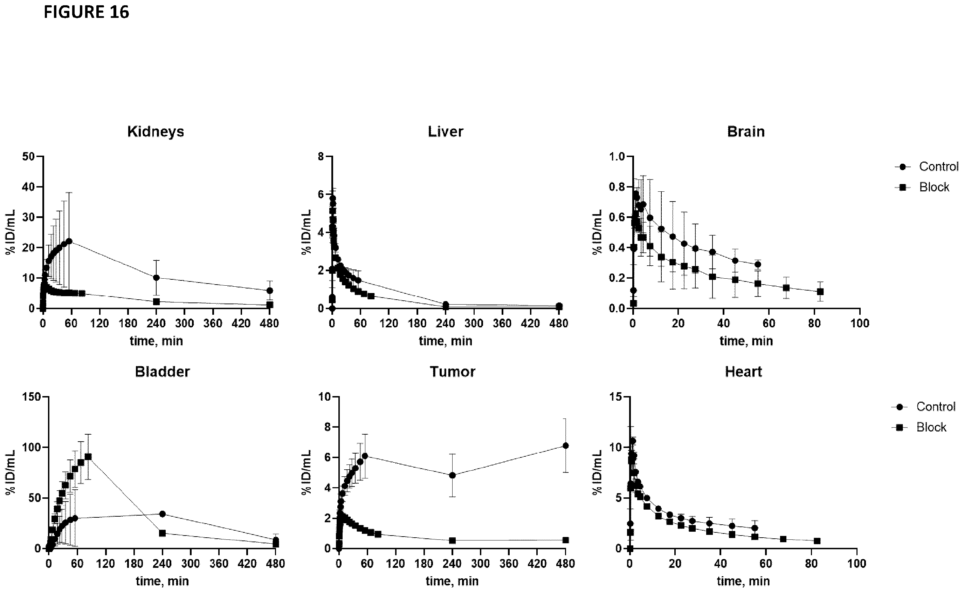

To analyze the data, the program PMOD (PMOD Technologies, Zürich, Switzerland) was used. Normalized maximum intensity projection (MIP) images were prepared using summed PET data for 60-90 min post-injection. Volumes of interest (VOls) for kidneys and PC3-PIP tumor were drawn using the isocontouring function in PMOD. Figure 16 shows the results obtained with [¹⁸F]**25**.

### Examples 34: In vivo PET/CT imaging in tumor bearing mice (LnCap) of [¹⁸F]labeled PSMA derivatives.

### Animals

For evaluation, NOD-SCID mice 6-7 weeks of age (Janvier, Europe) were inoculated with PC3-PIP (PSMA-positive) cells into the left shoulder and with PC3-flu (PSMA-negative) cells into the right shoulder. Imaging experiments were performed once both tumor xenografts reached the size of at least 100 mm³. Mouse body weight was 22-29 g on the day of the imaging.

### PET/CT procedure

The mice were anesthetized with a mixture of 1.5-2% isoflurane and 100% oxygen before injection of 0.1 ml bolus containing 5-9 MBq of [¹⁸F]-labeled PSMA derivative into the tail vein. To assess the specificity of tracer binding to its respective target (PSMA), for half of the mice the injected tracer was mixed with 100 µg of cold competing ligand (MEF98). PET acquisition (90 min) was started immediately after [¹⁸ F]FlutraPros injection. Additional PET acquisitions were performed 4 h (15 min long) and 8 h (30 min long) post-injection, with mice re-anesthetized before scanning as described above. PET/CT imaging was performed on a Siemens INVEON multimodality preclinical scanner in a docked mode (Siemens, Knoxville, Tennessee, USA). The imaging protocol included a two-bed CT for anatomic orientation performed with full rotation, 360-degree projections, and exposure of 80 kV and 500 µA. A static CT scan was immediately followed by the PET scan. CT and PET images were co-registered using a transformation.

### Image reconstruction

Reconstruction of PET data was performed using an OSEM3D/SP-MAP algorithm (2 x OSEM iterations and 18 x MAP iterations) with scatter correction and a matrix size 128x128, resulting in a final target resolution of 1.5 mm. List-mode data from the dynamic scan were binned into time frames of increasing lengths: 3 × 10 s, 3 × 30 s, 3 × 60 s, 5 × 300 s, 3 × 600 s and 2 × 900 s.

### Data analysis

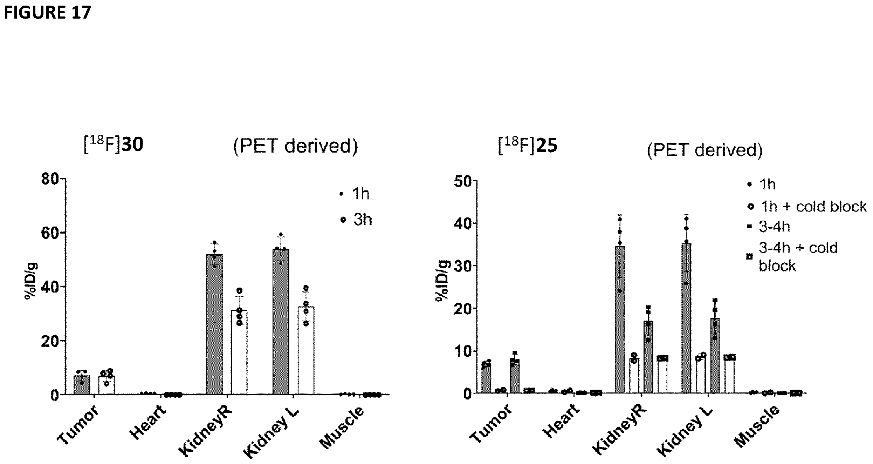

To analyze the data, the program PMOD (PMOD Technologies, Zürich, Switzerland) was used. Normalized maximum intensity projection (MIP) images were prepared using summed PET data for 60-90 min post-injection. Volumes of interest (VOls) for kidneys and PC3-PIP tumor were drawn using the isocontouring function in PMOD. Figure 17 shows the results obtained with [¹⁸F]**25** and [¹⁸F]**30**.

### Examples 34: Ex vivo biodistribution in tumor bearing mice of [²¹¹At]-labeled PSMA derivatives.

### Animals

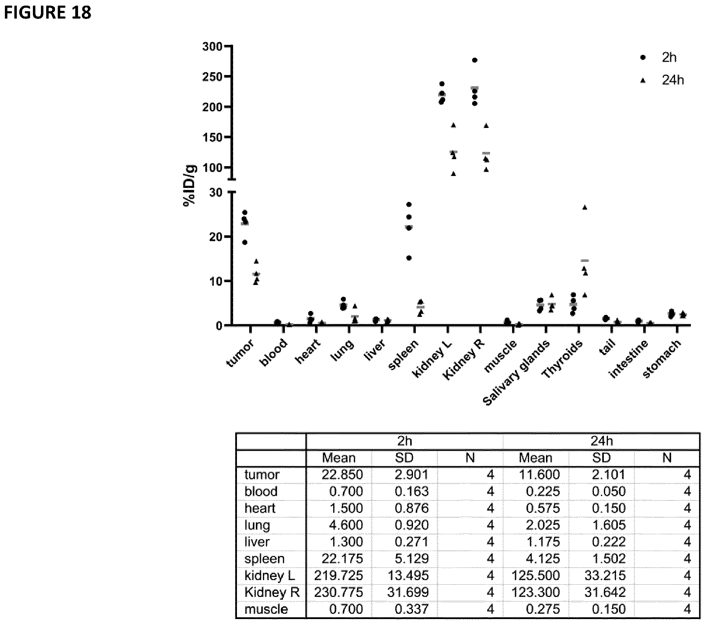

For the biodistribution, 7- to 8-wk-old male BALC/c *nu*/*nu* mice (BALB/cAnN-*Foxn1*^{nu/nu}/Rj, Janvier, France) were subcutaneously inoculated on their right flank with 5 × 10⁶ LNCaP cells (1:1 in PBS:Matrigel). Once the tumor size was ca. 1 cm³ mice were injected via the tail vein with 200 kBq of [²¹¹At]31. Mice were sacrificed at 2 and 24 h post-injection (n = 4 - 6 per time point) and organs of interest harvested. Organs included: blood, heart, lungs, liver, salivary glands, thyroids (trachea), spleen, stomach, small intestine, kidneys, tail, and grafted LNCaP tumor. After weighing, each organ was measured in an automated γ-counter (Hidex AMG, Hidex, Finland) with an energy window of 60 - 100 keV and a 80 keV peak position. %!D/g were calculated using the amount of solution injected and animal/organ weight. All counts were decay corrected and standardized to the injection solution. Figure 18 shows the results obtained with [²¹¹At]**31**.

### References

1. Bray, F.; Ferlay, J.; Soerjomataram, I.; Siegel, R. L.; Torre, L. A.; Jemal, A. Global cancer statistics 2018: GLOBOCAN estimates of incidence and mortality worldwide for 36 cancers in 185 countries. CA: A Cancer Journal for Clinicians 2018, 68, 394-424.
2. Antonarakis, E. S.; Feng, Z.; Trock, B. J.; Humphreys, E. B.; Carducci, M. A.; Partin, A. W.; Walsh, P. C.; Eisenberger, M. A. The natural history of metastatic progression in men with prostate-specific antigen recurrence after radical prostatectomy: long-term follow-up. BJU International 2012, 109, 32-39.
3. de Bono, J. S.; Oudard, S.; Ozguroglu, M.; Hansen, S.; Machiels, J.-P.; Kocak, I.; Gravis, G.; Bodrogi, I.; Mackenzie, M. J.; Shen, L.; Roessner, M.; Gupta, S.; Sartor, A. O. Prednisone plus cabazitaxel or mitoxantrone for metastatic castration-resistant prostate cancer progressing after docetaxel treatment: a randomised open-label trial. The Lancet 2010, 376, 1147-1154.
4. David, T.; Jean-Marc, F.; Manuel, B.; Palma, R.; Rodney, J. H.; Uwe, H. PSMA-Targeted Radionuclide Therapy and Salivary Gland Toxicity: Why Does It Matter? Journal of Nuclear Medicine 2018, 59, 747.
5. Martina, B.; Martin, S.; Ulrike, B.-W.; Ali, A.-O.; Clemens, K.; Walter, M.; Uwe, H.; Klaus, K.; Matthias, E. Preclinical Evaluation of a Tailor-Made DOTA-Conjugated PSMA Inhibitor with Optimized Linker Moiety for Imaging and Endoradiotherapy of Prostate Cancer. Journal of Nuclear Medicine 2015, 56, 914.
6. Sartor, O.; de Bono, J.; Chi, K. N.; Fizazi, K.; Herrmann, K.; Rahbar, K.; Tagawa, S. T.; Nordquist, L. T.; Vaishampayan, N.; El-Haddad, G.; Park, C. H.; Beer, T. M.; Armour, A.; Pérez-Contreras, W. J.; DeSilvio, M.; Kpamegan, E.; Gericke, G.; Messmann, R. A.; Morris, M. J.; Krause, B. J. Lutetium-177-PSMA-617 for Metastatic Castration-Resistant Prostate Cancer. New England Journal of Medicine 2021, 385, 1091-1103.
7. Marcu, L.; Bezak, E.; Allen, B. J. Global comparison of targeted alpha vs targeted beta therapy for cancer: In vitro, in vivo and clinical trials. Critical Reviews in Oncology/Hematology2018, 123, 7-20.
8. Feuerecker, B.; Tauber, R.; Knorr, K.; Heck, M.; Beheshti, A.; Seidl, C.; Bruchertseifer, F.; Pickhard, A.; Gafita, A.; Kratochwil, C.; Retz, M.; Gschwend, J. E.; Weber, W. A.; D'Alessandria, C.; Morgenstern, A.; Eiber, M. Activity and Adverse Events of Actinium-225-PSMA-617 in Advanced Metastatic Castration-resistant Prostate Cancer After Failure of Lutetium-177-PSMA. European Urology 2021, 79, 343-350.
9. Albertsson, P.; Bäck, T.; Bergmark, K.; Hallqvist, A.; Johansson, M.; Aneheim, E.; Lindegren, S.; Timperanza, C.; Smerud, K.; Palm, S. Astatine-211 based radionuclide therapy: Current clinical trial landscape. Front Med (Lausanne) 2022, 9, 1076210.
10. Mease, R. C.; Kang, C. M.; Kumar, V.; Banerjee, S. R.; Minn, I.; Brummet, M.; Gabrielson, K. L.; Feng, Y.; Park, A.; Kiess, A. P.; Sgouros, G.; Vaidyanathan, G.; Zalutsky, M. R.; Pomper, M. G. An Improved 211At-Labeled Agent for PSMA-Targeted α-Therapy. Journal of Nuclear Medicine 2022, 63, 259-267.
11. Otaru, S.; Paulus, A.; Imlimthan, S.; Kuurne, I.; Virtanen, H.; Liljenbäck, H.; Tolvanen, T.; Auchynnikava, T.; Roivainen, A.; Helariutta, K.; Sarparanta, M.; Airaksinen, A. J. Development of [18F]AmBF3 Tetrazine for Radiolabeling of Peptides: Preclinical Evaluation and PET Imaging of [18F]AmBF3-PEG7-Tyr3-Octreotide in an AR42J Pancreatic Carcinoma Model. Bioconjugate Chemistry 2022, 33, 1393-1404.
12. Allott, L.; Amgheib, A.; Barnes, C.; Braga, M.; Brickute, D.; Wang, N.; Fu, R.; Ghaem-Maghami, S.; Aboagye, E. O. Radiolabelling an 18F biologic via facile IEDDA "click" chemistry on the GE FASTLab™ platform. Reaction Chemistry & Engineering 2021, 6, 1070-1078.
13. García-Vázquez, R.; Jorgensen, J. T.; Bratteby, K. E.; Shalgunov, V.; Hvass, L.; Herth, M. M.; Kjær, A.; Battisti, U. M. Development of 18F-Labeled Bispyridyl Tetrazines for In Vivo Pretargeted PET Imaging. In Pharmaceuticals, 2022; Vol. 15.
14. García-Vázquez, R.; Battisti, U. M.; Jorgensen, J. T.; Shalgunov, V.; Hvass, L.; Stares, D. L.; Petersen, I. N.; Crestey, F.; Löffler, A.; Svatunek, D.; Kristensen, J. L.; Mikula, H.; Kjaer, A.; Herth, M. M. Direct Cu-mediated aromatic 18F-labeling of highly reactive tetrazines for pretargeted bioorthogonal PET imaging. Chemical Science 2021, 12, 11668-11675.
15. Guilarte, T. R.; McGlothan, J. L.; Foss, C. A.; Zhou, J.; Heston, W. D.; Kozikowski, A. P.; Pomper, M. G. Glutamate carboxypeptidase II levels in rodent brain using [125I]DCIT quantitative autoradiography. Neuroscience Letters 2005, 387, 141-144.
16. Hein, P.; Michel, M. C.; Leineweber, K.; Wieland, T.; Wettschureck, N.; Offermanns, S. Receptor and Binding Studies. In Practical Methods in Cardiovascular Research, Dhein, S.; Mohr, F. W.; Delmar, M., Eds. Springer Berlin Heidelberg: Berlin, Heidelberg, 2005; pp 723-783.
17. Keller, S. H.; L'Estrade, E. N.; Dall, B.; Palner, M.; Herth, M. Quantification Accuracy of a New HRRT High Throughput Rat Hotel Using Transmission-Based Attenuation Correction: A Phantom Study. Ieee Nucl Sci Conf R 2016**.**
18. Casado-Sainz, A.; Gudmundsen, F.; Baerentzen, S. L.; Lange, D.; Ringsted, A.; Martinez-Tajada, I.; Medina, S.; Lee, H.; Svarer, C.; Keller, S. H.; Schain, M.; Kjaerby, C.; Fisher, P. M.; Cumming, P.; Palner, M. Nigro-striatal dopamine activation lowers behavioral and neuronal phenotypes associated with obsessive-compulsive disorder. BioRxiv 2021**.**
19. Shalgunov, V.; Xiong, M.; L'Estrade, E. T.; Raval, N. R.; Andersen, !. V.; Edgar, F. G.; Speth, N. R.; Baerentzen, S. L.; Hansen, H. D.; Donovan, L. L.; Nasser, A.; Peitersen, S. T.; Kjaer, A.; Knudsen, G. M.; Syvanen, S.; Palner, M.; Herth, M. M. Blocking of efflux transporters in rats improves translational validation of brain radioligands. EJNMMI Res 2020, 10, 124.
20. Keller, S. H.; Svarer, C.; Sibomana, M. Attenuation correction for the HRRT PET-scanner using transmission scatter correction and total variation regularization. IEEE Trans Med Imaging 2013, 32, 1611-21.

## Claims

1. PSMA targeting urea-based ligand of formula (I): 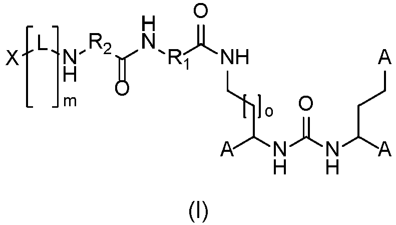 wherein:
A is independently carboxylic acid, sulphonic acid, phosphonic acid, tetrazole or isoxazole;
o is an integer selected from 1-4;
m is an integer selected from 0-10;
R₁ is -CH-CH₂-Z or -CH-CH₂-Y;
wherein Z is selected from the group consisting of: 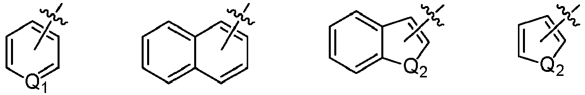
and Y is selected from the group consisting of: 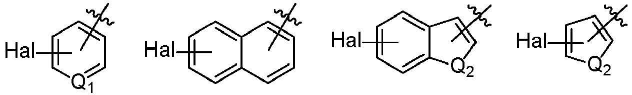 wherein
Q₁ is -C-R³ or N, wherein R³ is H or C₁-C₅ alkyl;
Q₂ is O, S or NH;
Hal (halogen) is a radioisotope selected from the group consisting of radioisotopes of fluorine, radioisotopes of iodine, radioisotopes of bromine and radioisotopes of astatine;
with the proviso that when R₂ = -CH-CH₂-Y, then R₁ must be = -CH-CH₂-Z
R₂ is -CH-CH₂-Y or -CH₂-T-;
wherein T is an aromatic monocyclic or polycyclic ring system having 6 to 14 carbon atoms, cyclopropyl, cyclobutyl, cyclopentyl, cyclohexyl, cycloheptyl or cyclooctyl; and Y is selected from the group consisting of: 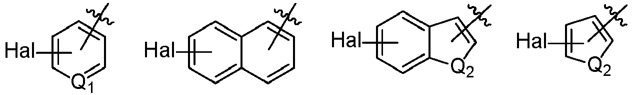 wherein
Q₁ is -C-R³ or N, wherein R³ is H or C₁-C₅ alkyl;
Q₂ is O, S or NH;
Hal (halogen) is a radioisotope selected from the group consisting of radioisotopes of fluorine, radioisotopes of iodine, radioisotopes of bromine and radioisotopes of astatine;
with the proviso that when R₁ = -CH-CH₂-Y, then R₂ must be = -CH₂-T
L is a moiety comprising groups selected from:
-CO(CH₂)ₙNH-, -CO(CH₂CH₂O)ₙNH-, -M-, -CO(CH₂)ₙNHMₗ-, -CO(CH₂CH₂O)ₙNHMₗ-, -MnCO(CH₂)ₙNH-, -MnCO(CH₂CH₂O)ₙNH-, -LysG-, -LysJ-, -LysGCO(CH₂CH₂O)ₙNH-, -LysJCO(CH₂)ₙNH-, -LysGCO(CH₂)ₙNHMₗ-,-LysJCO(CH₂)ₙNHMₗ-, -LysGMₙ-, -LysJMₙ-, -LysGCO(CH₂)ₙNHMₗ-, -LysJCO(CH₂)ₙNHMₗ-, -LysGCO(CH₂CH₂O)ₙNHMₗ-, -LysJCO(CH₂CH₂O)ₙNHMₗ-,-LysGMₙCO(CH₂)ₙNH-, -LysJMₙCO(CH₂)ₙNH-, -LysGMₙCO(CH₂CH₂O)ₗNH-, -LysJMₙCO(CH₂CH₂O)ₗNH-, -CO(CH₂CH₂O)ₙNHLysG-, -CO(CH₂)ₙNHLysJ-, -CO(CH₂)ₙNHMₗLysG-, -CO(CH₂)ₙNHMₗLysJ-, -MₙLysG-, -MₙLysJ-, -CO(CH₂)ₙNHMₗLysG-, -CO(CH₂)ₙNHMₗLysJ-, -CO(CH₂CH₂O)ₙNHMₗLysG-, -CO(CH₂CH₂O)ₙNHMₗLysJ-,-MₙCO(CH₂)ₗNHLysG-, -MₙCO(CH₂)ₗNHLysJ-, -MₙCO(CH₂CH₂O)ₗNHLysG-, -MₙCO(CH₂CH₂O)ₗNHLysJ-,
wherein n and I are integers independently selected from 0-10;
where M is selected from one or more natural amino acids, one or more sugars, and a combination of one or more natural amino acids and one or more sugars;
Lys is a D- or L- Lysine amino acid residue modified on the side chain with G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄ wherein p is an integer selected from 0 - 5 and
R₄ is a pyridazine selected from the group consisting of: 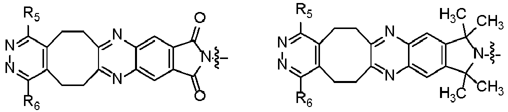 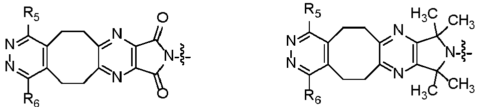 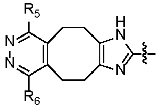
wherein R₅ and R₆ are identical or differs only in the isotope number of the labelling agent and are
independently selected from 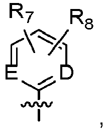 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b},-WCONH(CH₂)_{b}, -WNHCO(CH₂)_{b}, where W is -(CH₂)ₕ or -(CH₂CH₂O)ₕ , where b and h are independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ,-WNH(CH₂)ᵣ, -WCONH(CH₂)ᵣ, -WNHCO(CH₂)ᵣ, where W is -(CH₂)_{q} or -(CH₂CH₂O)_{q}, and r and q are independently selected from 1-25,
wherein, when R₇ is either (i) or (ii) the isotope labeling agent is selected from the group consisting of:
¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O,¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb, ²⁰⁸Pb, ²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ--OCH₂-COOH, wherein u is an integer selected from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, wherein u is an integer selected from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein
X is G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄ wherein p is an integer selected from 0-5, and R₄ is a pyridazine selected from the group consisting of: 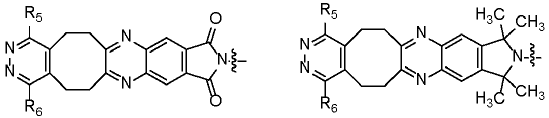 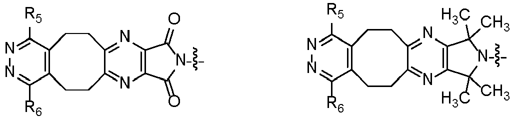 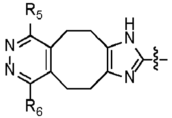
wherein R₅ and R₆ is independently selected from 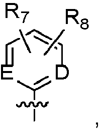 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or-(CH₂CH₂O)ₕ, where b and h are integers independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, -WCONH(CH₂)ᵣ, -WNHCO(CH₂)ᵣ, where W is -(CH₂)_{q} or -(CH₂CH₂O)_{q}, wherein and r and q are integers independently selected from 1-25,
wherein, when R₇ is either (i) or (ii) the isotope labeling agent is selected from the group consisting of:
¹H, ²H, ³H, ¹¹C, ¹²C, ¹³C, ¹⁴C, ¹³N, ¹⁴N, ¹⁵N, ¹⁸F, ¹⁹F ¹²³I, ¹²⁴I,¹²⁵I, ¹²⁷I, ¹³¹I, ²¹¹At, ¹⁵O, ¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy , ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh, ¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mpt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ--OCH₂-COOH, wherein u is an integer selected from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, and u is an integer selected from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein R₅ and R₆ are identical or differs only in the isotope number of the labelling agent;
and wherein formula (I) comprises at least one radioisotope selected from the group consisting of radioisotopes of fluorine, radioisotopes of iodine, radioisotopes of bromine and radioisotopes of astatine; and wherein at least one of L and X comprises J,
and pharmaceutically acceptable salts thereof.

2. PSMA targeting urea-based ligand according to claim 1, wherein L is selected from the group consisting of:
-CO(CH₂)ₙNH-, -CO(CH₂CH₂O)ₙNH-, -M-, -CO(CH₂)ₙNHMₗ-, -CO(CH₂CH₂O)ₙNHMₗ-, -MnCO(CH₂)ₙNH-, -MnCO(CH₂CH₂O)ₙNH-, -LysG-, -LysJ-, -LysGCO(CH₂CH₂O)ₙNH-, -LysJCO(CH₂)ₙNH-, -LysGCO(CH₂)ₙNHMₗ-,-LysJCO(CH₂)ₙNHMₗ-, -LysGMₙ-, -LysJMₙ-, -LysGCO(CH₂)ₙNHMₗ-, -LysJCO(CH₂)ₙNHMₗ-, -LysGCO(CH₂CH₂O)ₙNHMₗ-, -LysJCO(CH₂CH₂O)ₙNHMₗ-,-LysGMₙCO(CH₂)ₙNH-, -LysJMₙCO(CH₂)ₙNH-, -LysGMₙCO(CH₂CH₂O)ₗNH-, -LysJMₙCO(CH₂CH₂O)ₗNH-, -CO(CH₂CH₂O)ₙNHLysG-, -CO(CH₂)ₙNHLysJ-, -CO(CH₂)ₙNHMₗLysG-, -CO(CH₂)ₙNHMₗLysJ-, -MₙLysG-, -MₙLysJ-, -CO(CH₂)ₙNHMₗLysG-, -CO(CH₂)ₙNHM,LysJ-, -CO(CH₂CH₂O)ₙNHMₗLysG-, -CO(CH₂CH₂O)ₙNHMₗLysJ-,-MₙCO(CH₂)ₗNHLysG-,-MₙCO(CH₂)ₗNHLysJ-, -MₙCO(CH₂CH₂O)ₗNHLysG-, -MₙCO(CH₂CH₂O)ₗNHLysJ-,
wherein n and I are integers independently selected from 0-10;
where M is selected from one or more natural amino acids, one or more sugars, and a combination of one or more natural amino acids and one or more sugars;
Lys is a D- or L- Lysine amino acid residue modified on the side chain with G or J, wherein G is either a chelating agent that can comprise a metal, or a tetrazine or a metabolic derivative thereof, and
J is -CO(CH₂)ₚR₄ or-CO(CH₂)ₚC(CH₃)₂R₄, wherein p is an integer from 0 - 5 and
R₄ is a pyridazine selected from the group consisting of: 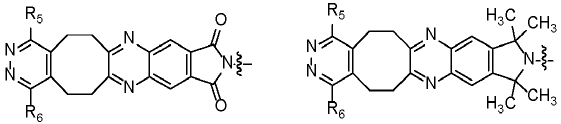 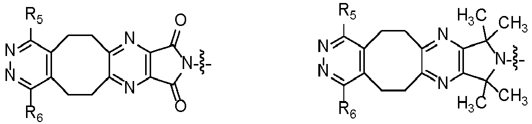 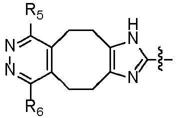
wherein R₅ and R₆ is independently selected from 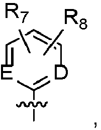 wherein the curly sign indicates the link to the six-membered aromatic ring of the pyridazine, and wherein R₇ is -H, or (i) an isotope labeling agent directly connected to the aromatic ring, or (ii) an isotope labeling agent connected to the aromatic ring via a linker, wherein the linker is selected from the group consisting of -(CH₂)_{b}, WO(CH₂)_{b} -WNH(CH₂)_{b}, -WCONH(CH₂)_{b}, -WNHCO(CH₂)_{b}, where W is -(CH₂)ₕ or - (CH₂CH₂O)ₕ, where b and h are independently selected from 1-25, or (iii) an isotope labeling agent that is chelated through a chelating agent connected to the aromatic ring through a linker, wherein the linker is selected from the group consisting of -(CH₂)ᵣ, -WO(CH₂)ᵣ, -WNH(CH₂)ᵣ, -WCONH(CH₂)ᵣ, -WNHCO(CH₂)ᵣ, where W is -(CH₂)_{q} or -(CH₂CH₂O)_{q}, and r and q are independently selected from 1-25,
wherein, when R₇ is either (i) or (ii) the isotope labeling agent is selected from the group consisting of:
¹H, ²H, ³H, ¹¹C, ¹²C, ¹³C, ¹⁴C, ¹³N, ¹⁴N, ¹⁵N, ¹⁸F, ¹⁹F, ¹²³I, ¹²⁴I,¹²⁵I, ¹²⁷I, ¹³¹I, ²¹¹At, ¹⁵O,¹⁶O, ¹⁷O, ¹⁸O, ⁴³Sc, ⁴⁴Sc, ⁴⁵Sc, ⁴⁵Ti, ⁴⁶Ti, ⁴⁷Ti, ⁴⁸Ti, ⁴⁹Ti, ⁵⁰Ti, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷⁶Br, ⁷⁷Br, ⁷⁹Br, ^{80m}Br, ⁸¹Br, ⁷²As, ⁷⁵As, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰⁶Pb, ²⁰⁷Pb,²⁰⁸Pb,²¹²Pb, ²⁰⁹Bi, ²¹²Bi, ²¹³Bi, ³¹P, ³²P, ³³P, ³²S, ³⁵S, ⁴⁵Sc, ⁴⁷Sc, ⁸⁴Sr, ⁸⁶Sr, ⁸⁷Sr, ⁸⁸Sr, ⁸⁹Sr, ¹⁶⁵Ho, ¹⁶⁶Ho, ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ²²⁷Th, ²³²Th, ⁵¹Cr, ⁵²Cr, ⁵³Cr, ⁵⁴Cr, ⁷³Se, ⁷⁴Se, ⁷⁵Se, ⁷⁶Se, ⁷⁷Se, ⁷⁸Se, ⁸⁰Se, ⁸²Se, ⁹⁴Tc, ^{99m}Tc, ¹⁰³Rh,¹⁰³mRh, ¹¹⁹Sb, ¹²¹Sb, ¹²³Sb, ¹³⁵La, ¹³⁸La, ¹³⁹La, ¹⁶²Er, ¹⁶⁴Er, ¹⁶⁵Er, ¹⁶⁶Er, ¹⁶⁷Er, ¹⁶⁸Er, ¹⁷⁰Er, ¹⁹³mPt, ¹⁹⁵mPt, ¹⁹²Pt, ¹⁹⁴Pt, ¹⁹⁵Pt, ¹⁹⁶Pt, ¹⁹⁸Pt,
and wherein E and D are independently selected from: -CH and -N- ,
and wherein R₈ is H or a moiety selected from the group consisting of: a hydroxy group, a sulfonamide, a carboxyl group, a sulfonyl group, amine, a substituted amine with 1-5 polyethylene glycol unit(s), a -(O-CH₂-CH₂)ᵤ--OCH₂-COOH, wherein u is an integer from 1-5, Methyl, Ethyl, Propyl, optionally substituted heteroaryl, and optionally substituted arylalkyl; wherein optionally substituted means one or more substituents selected from: a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)ᵤ-OCH₂-COOH, and u is an integer from 1-5; or H, Methyl, Ethyl, Propyl, optionally substituted heteroaryl, and optionally substituted arylalkyl; wherein optionally substituted means one or more substituents selected from a halogen, a hydroxy group, a sulfonamide, a carboxyl group, a sulfonyl group, and amine,
and wherein R₅ and R₆ are identical or differs only in the isotope number of the labelling agent.

3. PSMA targeting urea-based ligand according to claim 1, wherein M is a polar natural amino acid or its D-enantiomer.

4. PSMA targeting urea-based ligand according to any of claims 1 to 3 wherein the chelating agent comprised by G is selected form the group consisting of:
1,4,7,10-tetraazacyclododecane-N,N',N',N"-tetraacetic acid (DOTA),
N,N'-bis(2-hydroxy-5-(carboxyethyl)benzyl)ethylenediamine N,N'-diacetic acid (HBED-CC),
14,7-triazacyclononane-1,4,7-triacetic acid (NOTA),
2-(4.7-bis(carboxymethyl)-1,4,7-triazonan-1-yl)pentanedioic acid (NODAGA),
2-(4,7,10-tris(carboxymethyl)-1,4,7,10-tetraazacyclododecan-1- yl)pentanedioic acid (DOTAGA),
14,7-triazacyclononane phosphinic acid (TRAP),
14,7-triazacyclononane-1-methyl(2-carboxyethyl)phosphinic acid-4,7-bis(methyl(2-hydroxymethyl)phosphinic acid (NOPO),
3,6,9,15-tetraazabicyclo9.3.1.pentadeca-1 (15),11,13-triene-3,6,9- triacetic acid (PCTA),
N'-(5-acetyl (hydroxy)aminopentyl-N-(5-(4-(5- aminopentyl)(hydroxy)amino-4-oxobutanoyl)amino)pentyl-N- hydroxysuccinamide (DFO), diethylenetriaminepentaacetic acid (DTPA),
trans-cyclohexyl-diethylenetriaminepentaacetic acid (CHX-DTPA),
1-oxa-4,7,10-triazacyclododecane-4,7,10-triacetic acid (OXO-Do3A),
p-isothiocyanatobenzyl-DTPA (SCN-BZ-DTPA),
1-(p-isothiocyanatobenzyl)-3-methyl-DTPA (1B3M),
2- (p-isothiocyanatobenzyl)-4-methyl-DTPA (1M3B),
1-(2)-methyl-4-isocyanatobenzyl-DTPA (MX-DTPA).

5. PSMA targeting urea-based ligand according to claim 4, wherein the chelating agent comprises a metal.

6. PSMA targeting urea-based ligand according to claim 5, wherein the metal is selected from:
⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁸⁶Y, ⁸⁹Y, ⁹⁰Y, ⁸⁹Zr, ⁹⁰Zr, ⁹¹Zr, ⁹²Zr, ⁹⁴Zr, ¹⁴⁹Tb, ¹⁵²Tb, ¹⁵⁹Tb, ¹⁶¹Tb, ¹¹¹In, ¹¹³In, ¹¹⁴mIn, ¹¹⁵mIn, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl , ²⁰⁶Pb, ²⁰⁷Pb,²⁰⁸Pb,²¹²Pb, ²⁰⁹Bi, ²¹²Bi, ²¹³Bi, ⁴⁵Sc, ⁴⁷Sc, ²²⁷Th, ²³²Th, ⁹⁴Tc, ^{99m}Tc, ¹⁰³Rh,¹⁰³mRh, ¹¹⁹Sb, ¹²¹Sb, ¹²³Sb, ¹⁹³mPt, ¹⁹⁵mPt, ¹⁹²Pt, ¹⁹⁴Pt, ¹⁹⁵Pt, ¹⁹⁶Pt , ¹⁹⁸Pt, ²²⁵Ac.

7. PSMA targeting urea-based ligand according to any of claims 1 to 6, wherein at least one radionuclide is selected is from the group consisting of ¹⁸F, ¹²⁵I, ¹²³I, ¹³¹I, ¹²⁴I, ²¹¹At, ⁷⁷Br and ^{80m}Br.

8. PSMA targeting urea-based ligand according to claim 7, wherein at least one radionuclide is positioned on R₄

9. PSMA targeting urea-based ligand according to claim 7, wherein at least one radionuclide is positioned on R₁ or R₂

10. PSMA targeting urea-based ligand according to claims 7, wherein a radionuclide is positioned on R₄ and on R₁ or R₂

11. PSMA targeting urea-based ligand according to any of the previous claims, wherein the at least one radionuclide is positioned on R₄, R₁ or R₂ and further comprising an unlabled chelator.

12. PSMA targeting urea-based ligand according to any of the previous claims, comprising one J positioned at either X or L and wherein J is selected from J (i) or J (ii).

13. PSMA targeting urea-based ligand according to any of the previous claims, wherein R₇ comprises ¹⁸F.

14. PSMA targeting urea-based ligand according to claim 1 selected from the group consisting of: 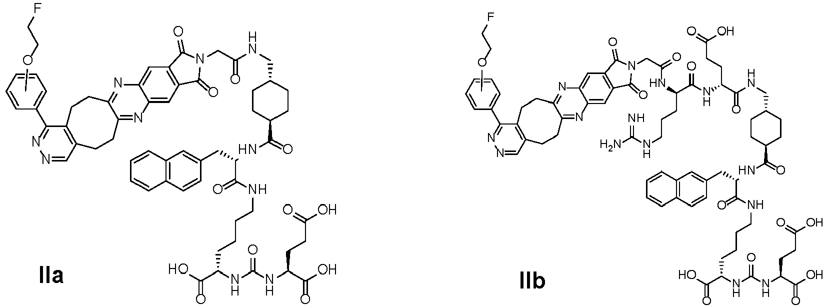 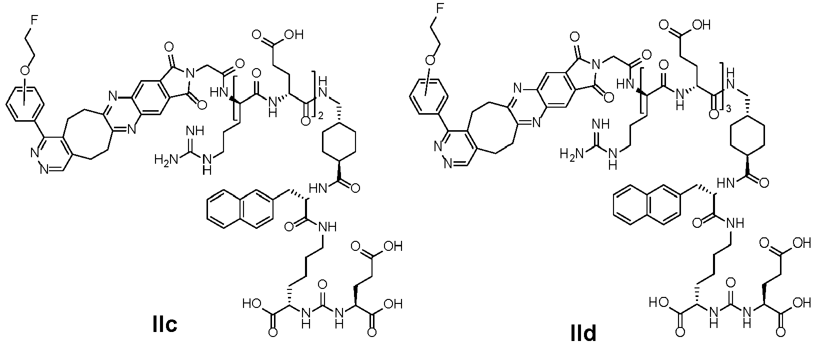 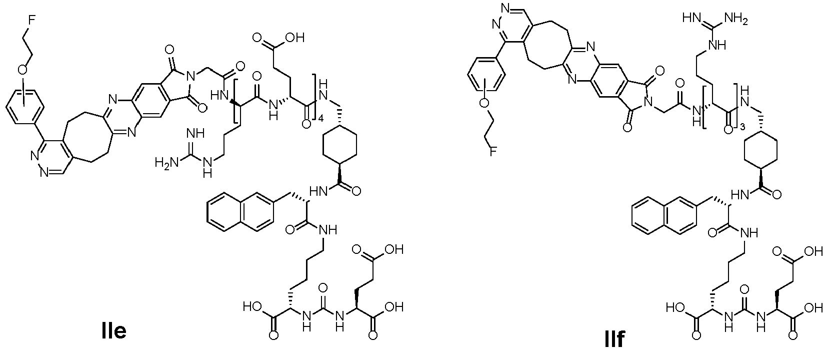 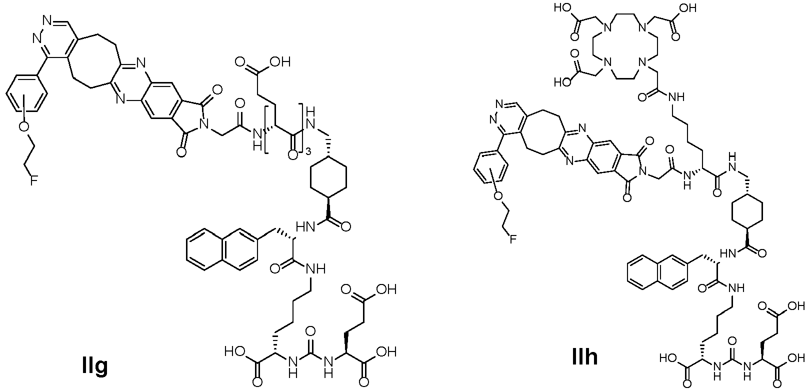 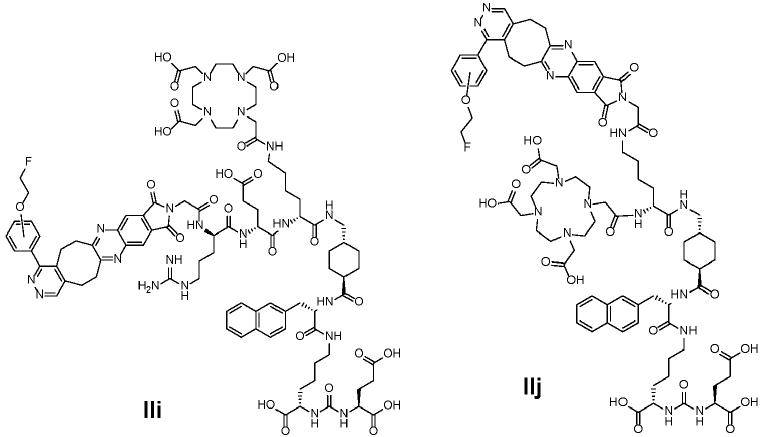 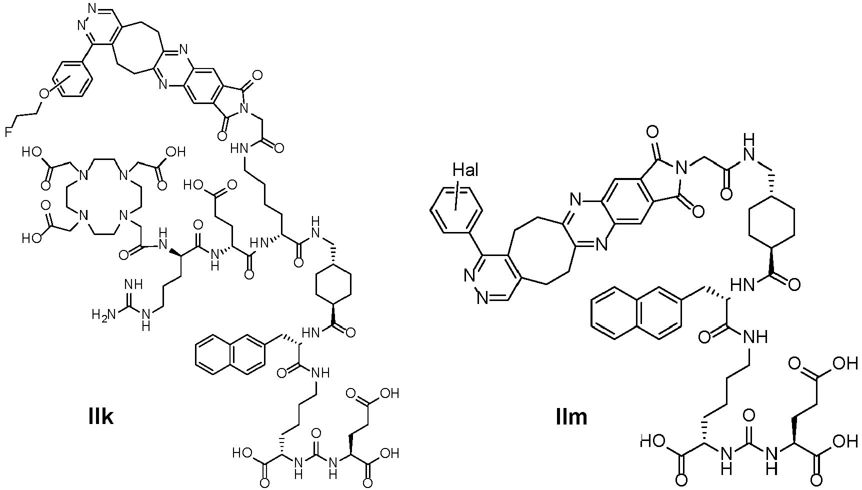 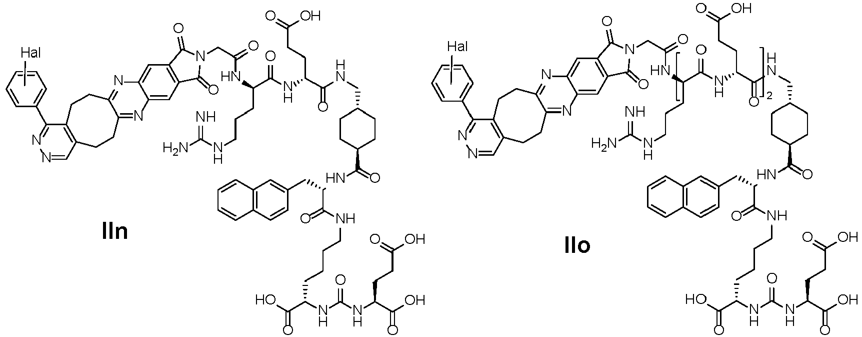 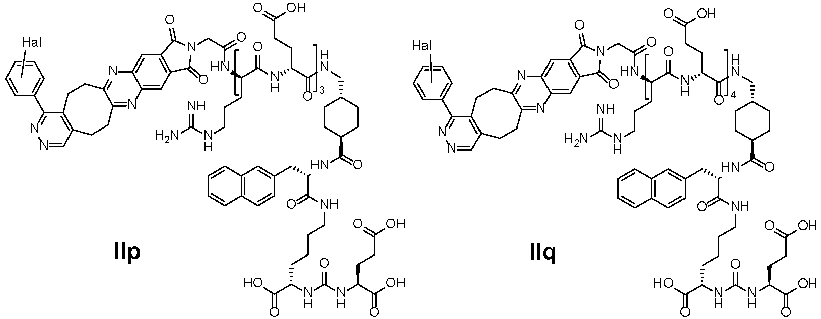 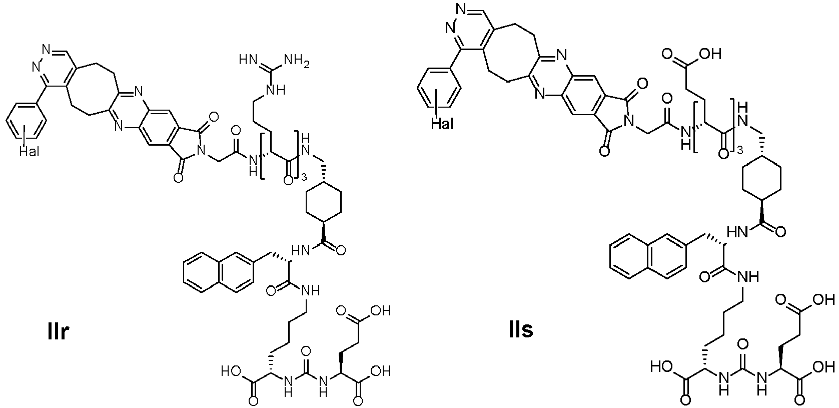 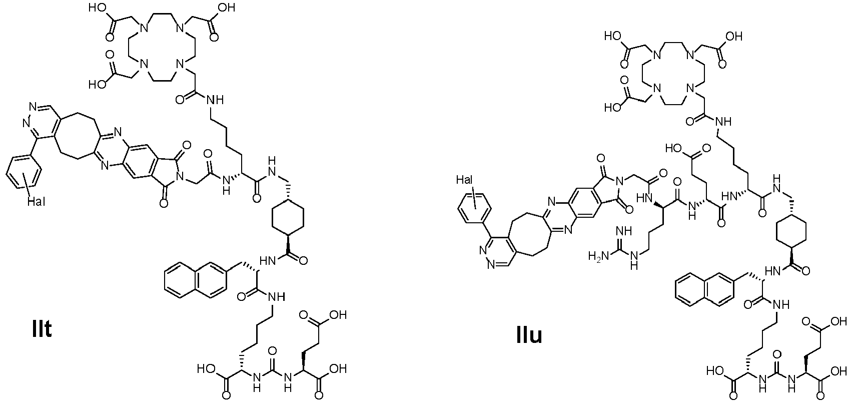 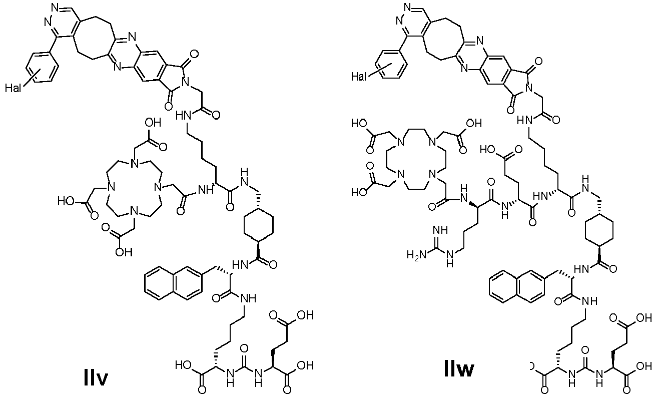 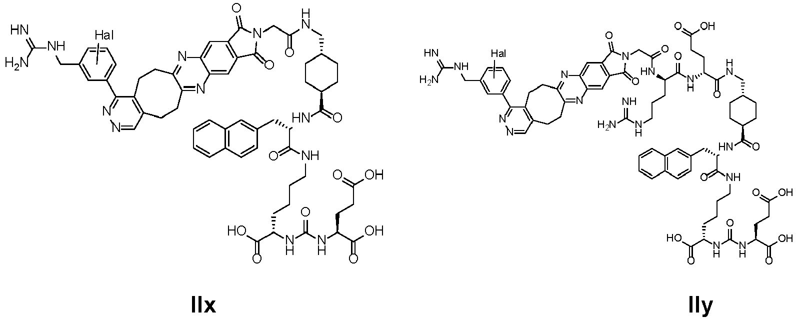 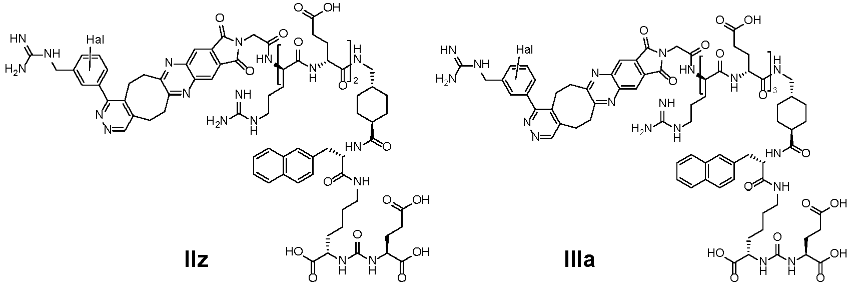 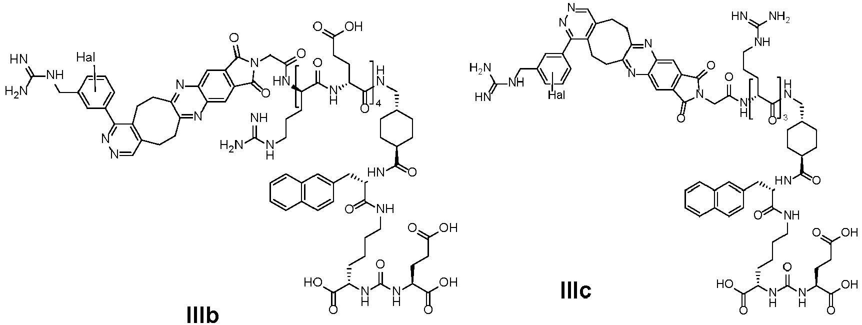 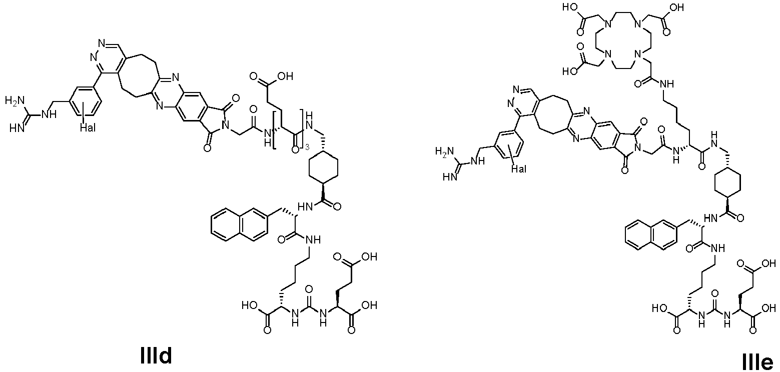 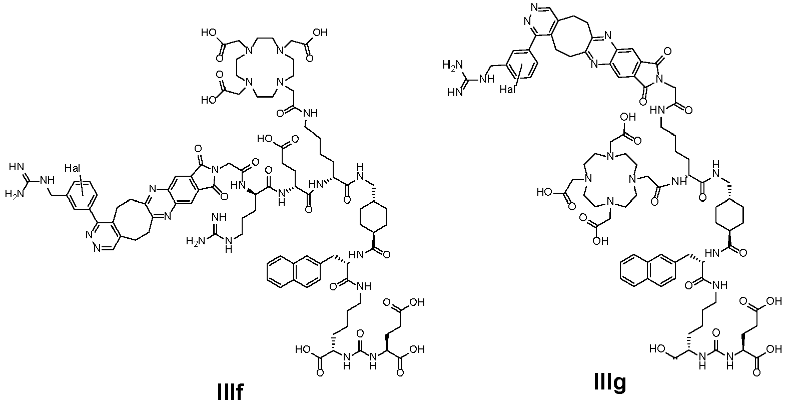 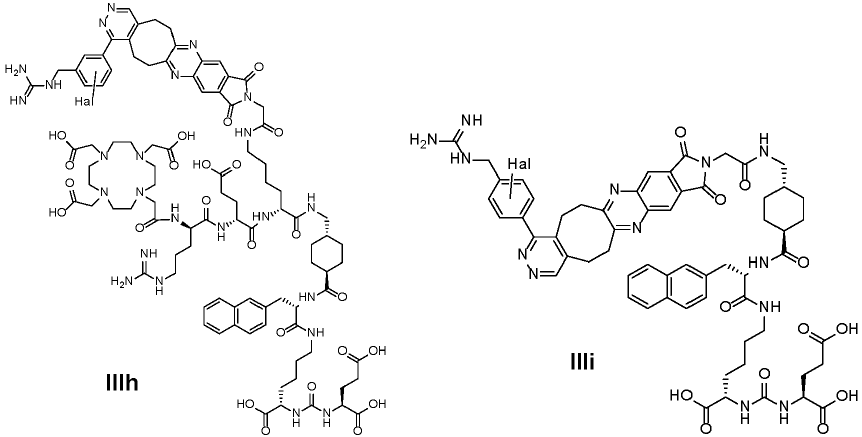 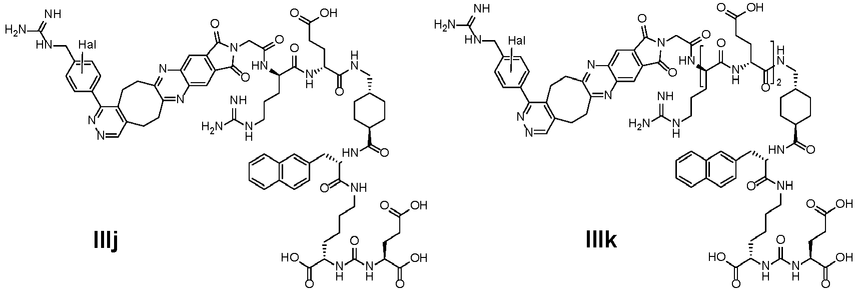 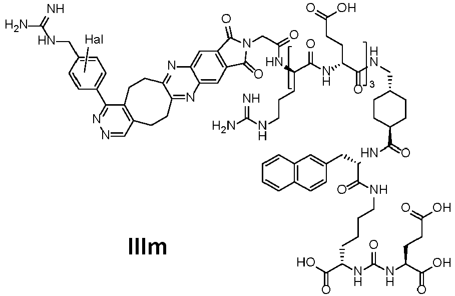 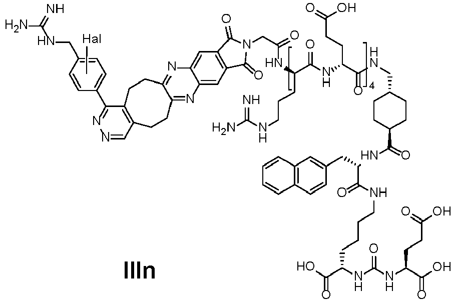 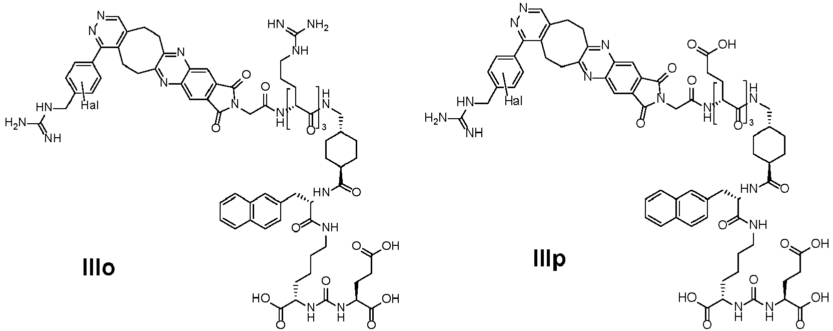 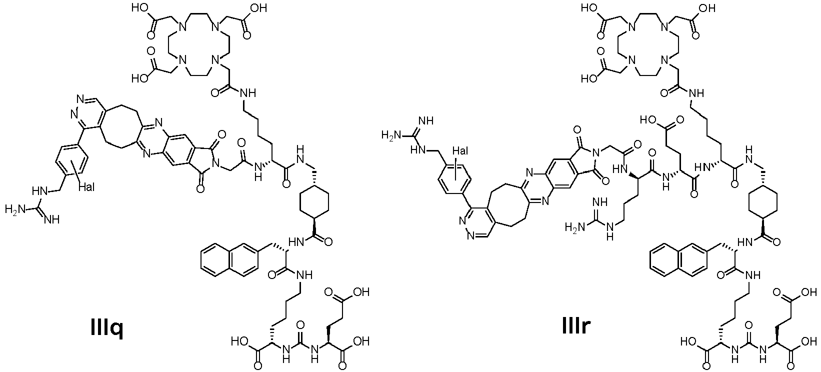 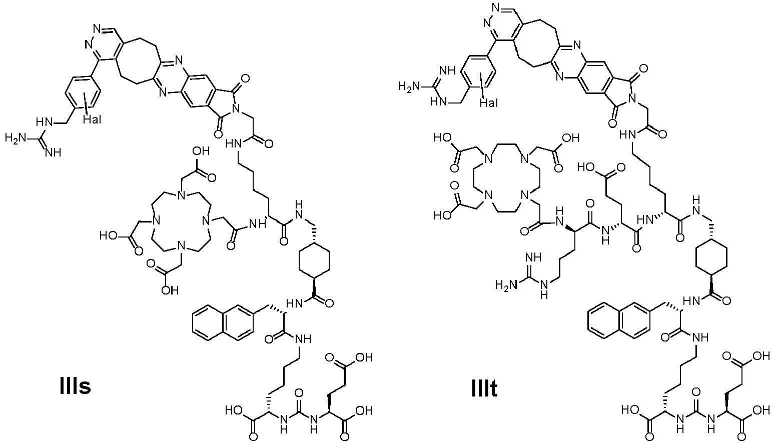 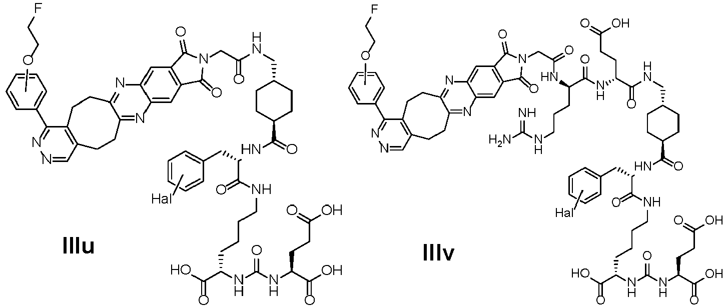 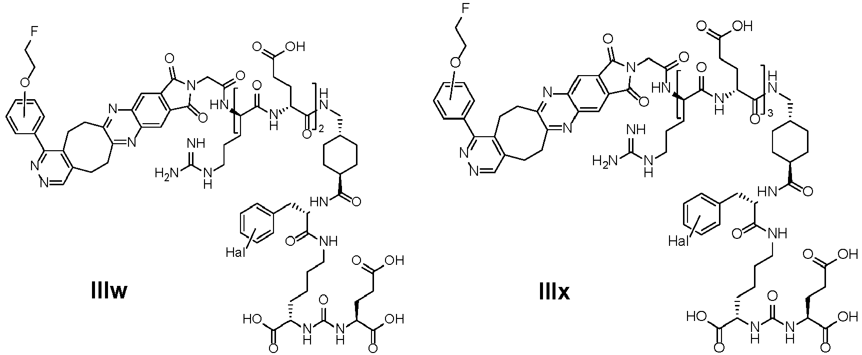 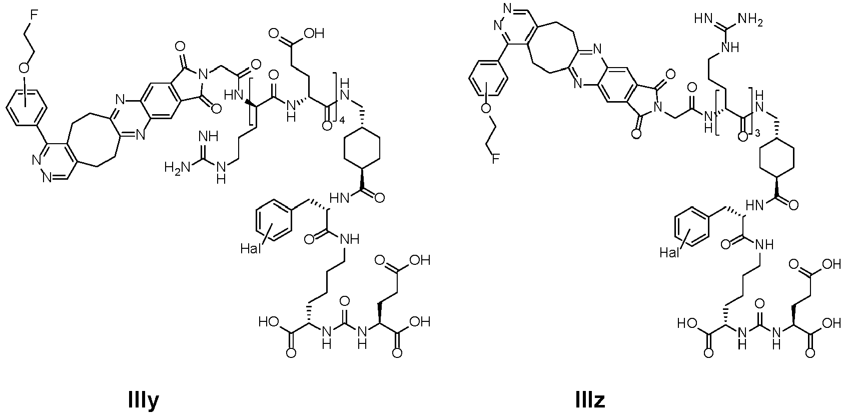 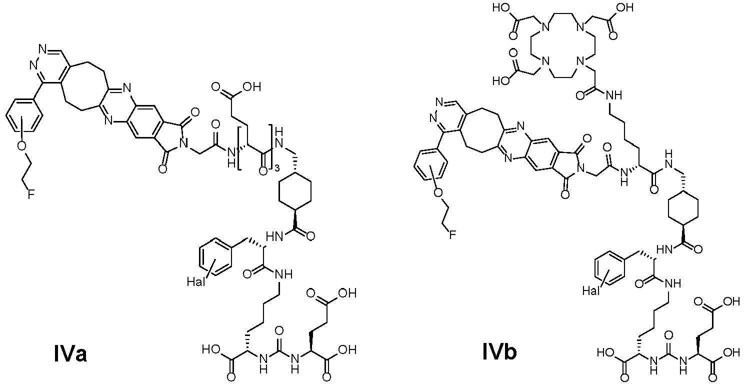 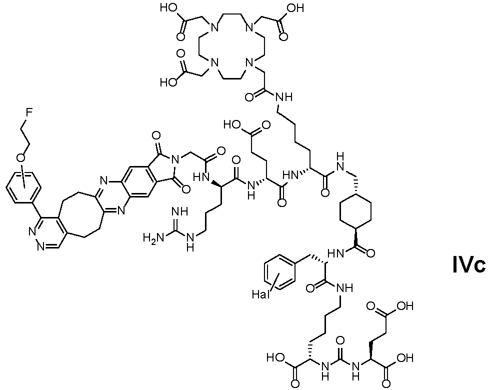 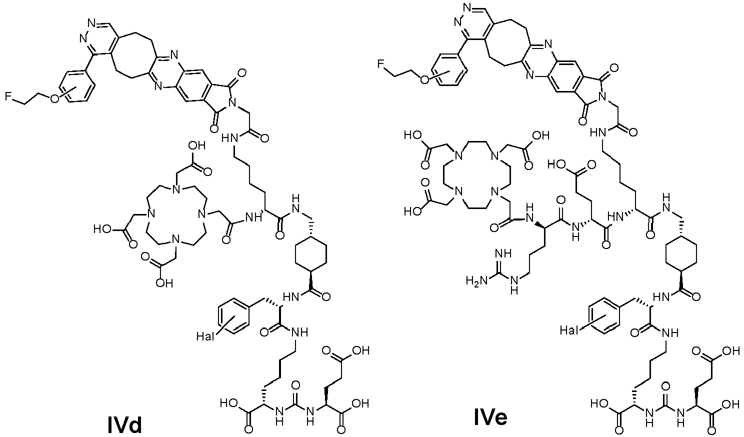
Wherein F is ¹⁸F or ¹⁹F
and Hal is ¹⁸F, ¹⁹F, ¹⁸F, ¹²⁵I, ¹²³I, ¹²⁷I, ¹³¹I, ¹²⁴I, ²¹¹At, ⁷⁷Br, ⁷⁹Br, or ^{80m}Br

15. Precursor for providing a PSMA targeting urea-based ligand according to claim 1, selected from 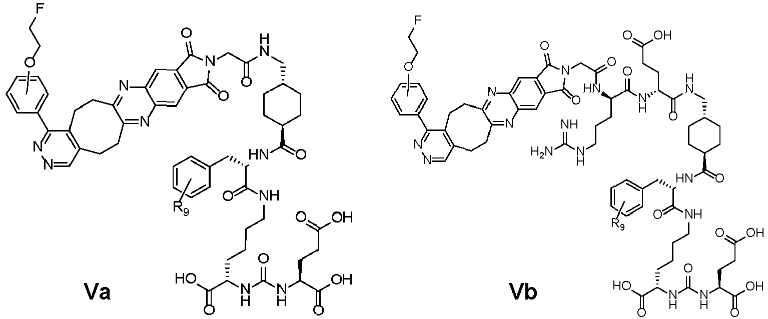 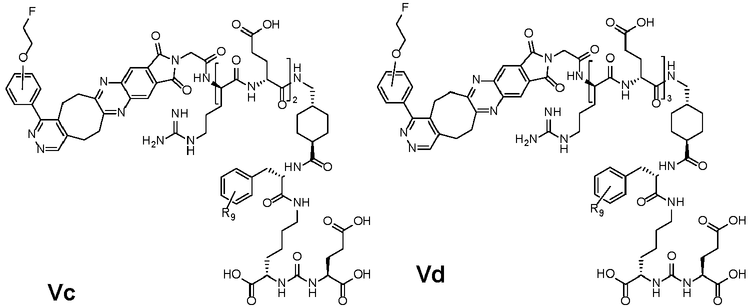 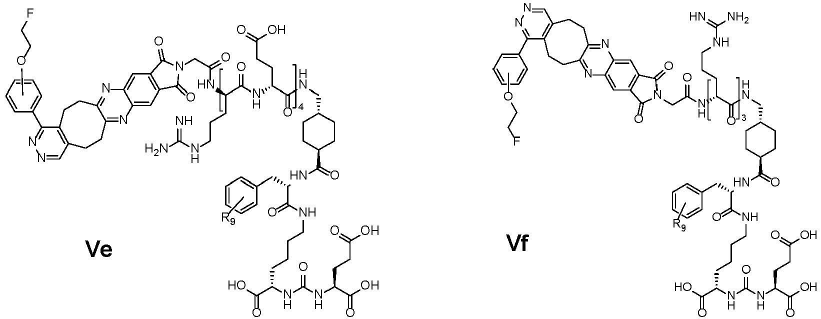 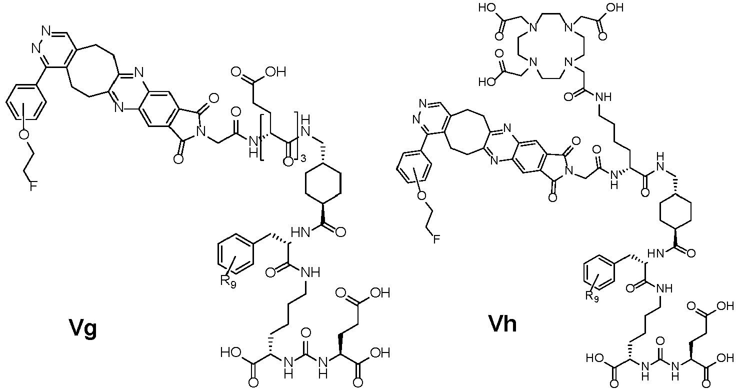 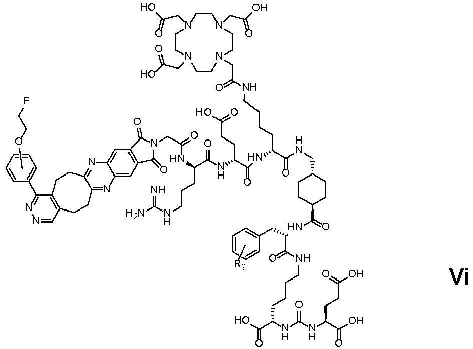 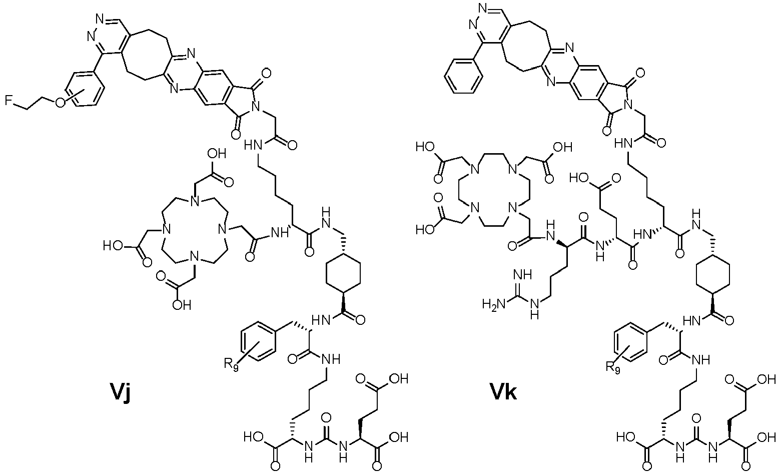 wherein R₉ is -Si(CH₃)₃, -Si(CH₂CH₂CH₂CH₃)₃, -Sn(CH₃)₃, -Sn(CH₂CH₂CH₂CH₃)₃, -B(OH)₂, or
